# EUROPEAN PATENT APPLICATION

(11) **EP 4 692 105 A1**
(43) Date of publication of application: **11.02.2026**
(21) Application number: 24777627.1
(22) Date of filing: 01.03.2024
(51) Int. Cl.: C07K 7/06, A61K 38/08, A61K 51/08

(54) **CYCLIC POLYPEPTIDE COMPOUND AND USE THEREOF**

(30) Priority: 27.03.2023 CN 202310307579; 27.07.2023 CN 202310934748
(71) Applicant: Bivision Pharmaceuticals, Inc, Pudong New Area, Shanghai 201315 (CN)
(72) Inventor: WANG, Eric Yanjun, Nanjing, Jiangsu 211500 (CN); HE, Jinqiu, Nanjing, Jiangsu 211500 (CN); YU, Haihua, Nanjing, Jiangsu 211500 (CN); WANG, Kevin Yu, Nanjing, Jiangsu 211500 (CN); SUN, Yueguang, Nanjing, Jiangsu 211500 (CN); ZHAN, Jian, Nanjing, Jiangsu 211500 (CN); YU, Yaoming, Nanjing, Jiangsu 211500 (CN)
(74) Representative: Michalski Hüttermann & Partner Patentanwälte mbB
(86) International application number: PCT/CN2024/079635
(87) International publication number: WO 2024/198837

(57) **Abstract**

Provided are a cyclic polypeptide compound and the use thereof, and specifically disclosed are a cyclic polypeptide compound A and a cyclic polypeptide compound B. The cyclic polypeptide compound A is a compound formed by ion chelation of a compound X and a therapeutic radionuclide. The cyclic polypeptide compound B is a compound formed by ion chelation of a compound X and a diagnostic radionuclide. The compound can be used for diagnosis and treatment of FAP-related or mediated tumors, has high tumor uptake and long retention time, and has a wide application prospect.

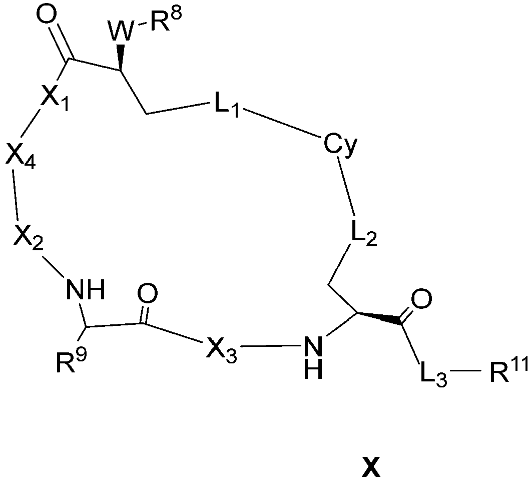

## Description

The present application claims the priorities of Chinese patent application 202310307579.5 filed on March 27, 2023. The contents of the above Chinese patent application are incorporated herein by reference in its entirety.

The present application claims the priorities of Chinese patent application 202310934748.8 filed on July 27, 2023. The contents of the above Chinese patent application are incorporated herein by reference in its entirety.

### TECHNICAL FIELD

The present disclosure relates to a cyclic polypeptide compound and a use thereof.

### BACKGROUND

Chemotherapy remains widely used in the treatment of cancer patients and other diseases. Traditional anticancer chemotherapy drugs act on fundamental mechanisms of cell survival and cannot effectively distinguish between healthy and malignant cells. Moreover, these drugs do not efficiently reach or accumulate at the disease site after systemic administration. Non-specific mechanisms of action and low efficiency in tumor site localization are the reasons for side effects and poor therapeutic effects.

To treat diseases more effectively, the development of targeted drugs has been one of the hot topics in new drug research and development in recent years. These drugs can selectively localize at the disease site and exert their effects after systemic administration. Such drugs are substances formed by combining representative chemicals with therapeutic effects (such as cytotoxic drugs or radionuclides) and ligands with specificity for targeting cells. Disease-specific monoclonal antibodies, peptides, and small-molecule ligands are the preferred ligands for developing targeted drug products. For targeted applications, the use of small molecule ligands offers more rapid and efficient tumor penetration, lower immunogenicity, and lower manufacturing costs than larger molecules such as peptides and antibodies.

Tumors are complexes composed of tumor cells and their surrounding stromal cells and non-cellular components. The occurrence and progression of tumors represent a dynamic process of mutual promotion and co-evolution between tumor cells and tumor microenvironment (TME). The tumor microenvironment is composed of many heterogeneous cell types, such as immune cells, including endothelial cells, cancer associated fibroblasts (CAFs), and their extracellular products. Among them, cancer associated fibroblasts (CAFs) are the most important stromal cells in the tumor microenvironment, accounting for about 50% of the total number of tumor tissue cells. CAFs play an important role in tumor growth, metastasis, drug resistance, treatment resistance, *etc.* It is one of the hot topics in tumor diagnosis and treatment research in recent years.

A prominent feature of CAFs is the high expression of seprase or fibroblast activation protein (FAP). Both are the same cell-surface transmembrane serine proteases, possessing dual activities as dipeptidyl peptidase (DPP) and collagenase, capable of degrading dipeptides and type I collagen. FAP and dipeptidyl peptidase IV (DPPIV) have similar structural domains and dipeptidyl peptidase activity and belong to the same serine protease family. However, FAP possesses unique endopeptidase activity, capable of cleaving gelatin, denatured type I collagen, and α2-antiplasmin, whereas DPPIV lacks this function, which forms the distinction between the two. FAP is selectively expressed on the surface of stromal fibroblasts in 90% or more of epithelial malignancies, including breast cancer, ovarian cancer, lung cancer, colorectal cancer, gastric cancer, pancreatic cancer, cutaneous melanoma, *etc.* FAP is typically not expressed in benign and precancerous epithelial tumors, such as colorectal adenomas, breast phyllodes tumors, and fibroadenomas. FAP is generally not expressed in normal human tissues, but only exists in the cervix and endometrium, and is expressed briefly during embryonic development. Numerous studies have shown that the high expression of FAP in CAFs of epithelial tumors is associated with poorer patient prognosis, indicating that its activity level is related to cancer progression as well as the metastasis and spread of cancer cells.

Radiopharmaceuticals are medical preparations composed of radioactive isotopes paired with molecular agents that target specific organs and tissues. They are radioactive drugs that can be used for imaging diagnosis and clinical treatment. Based on their uses, they can be categorized into diagnostic radiopharmaceuticals and therapeutic radiopharmaceuticals.

Diagnostic radiopharmaceuticals include two categories: drugs for organ imaging and drugs for functional determination. When combined with SPECT or PET, they enable the study of drug functions and metabolic processes in vivo at the molecular level, achieving rapid, non-destructive, and real-time imaging of physiological and pathological processes. This provides a means for truly early diagnosis and timely treatment.

Therapeutic radiopharmaceuticals refer to radioactive drugs that, when administered orally or intravenously to patients, highly selectively accumulate in diseased tissues. The rays radiated by radioisotopes produce local ionizing radiation biological effects, thereby inhibiting or destroying the diseased tissues to achieve therapeutic effects.

In recent years, the use of FAP as a target in tumor diagnosis and treatment has received widespread attention. In diagnosis, compared with FDG imaging, FAP-targeted imaging exhibits lower background activity in organs such as the brain and liver, while demonstrating higher detection rates for tumor lesions. However, to date, such probes have not shown particularly effective therapeutic effects, primarily due to their generally low bioactivity, poor uptake at lesion sites, and short retention in vivo. In addition, from the perspective of ligand selection, the binding of ligands and receptors showed a one-to-one correspondence, and the stability of binding to receptors was poor, and the low lesion uptake greatly limited the diagnosis and treatment effect. Therefore, Using FAP as a specific molecular target with nuclear medical imaging is a promising strategy for several applications, including the early diagnosis of malignant tumors, accurate tumor staging, and both companion diagnostics and efficacy evaluation of cancer treatments.

### SUMMARY

The objective of the present disclosure is to address the existing problems in the current technical field, such as low affinity of compounds for FAP, short retention time at the target site, or insufficient uptake in lesions. The invention provides a cyclic polypeptide compound and a use thereof. This cyclic polypeptide compound offers advantages including simple preparation, good stability, high tumor uptake, and prolonged retention, making it suitable for clinical promotion and application.

The present disclosure provides a compound X or a pharmaceutically acceptable salt thereof, 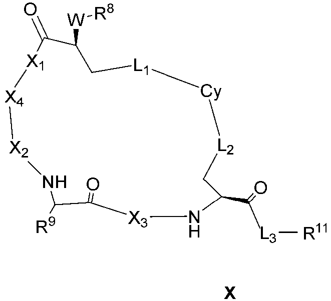
wherein -X₁- is 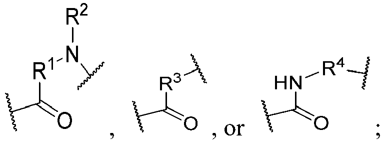
R¹ is C₁-C₄ alkylene, C₃-C₆ cycloalkylene, C₁-C₄ alkylene-C₃-C₆ cycloalkylene, or C₁-C₄ alkylene-C₃-C₆ heterocycloalkylene, wherein the heteroatom of the 3- to 6-membered heterocycloalkylene is selected from one or more of N, O, and S, the number of heteroatoms is 1, 2, or 3, and at least one N is included;
R² is hydrogen or C₁-C₄ alkyl;
R³ is C₁-C₄ alkylene, C₁-C₄ alkylene-3- to 6-membered heterocycloalkylene, 3- to 6-membered heterocycloalkylene, or 6- to 12-membered heteroarylene; the heteroatom of the 3-to 6-membered heterocycloalkylene is selected from one or more of N, O, and S, the number of heteroatoms is 1, 2, or 3, and at least one N is included; the heteroatom of the 6- to 12-membered heteroarylene is selected from one or more of N, O, and S, the number of heteroatoms is 1, 2, or 3, and at least one N is included;
R⁴ is 3- to 6-membered heterocycloalkylene; the heteroatom of the 3- to 6-membered heterocycloalkylene is selected from one or more of N, O, and S, the number of heteroatoms is 1, 2, or 3, and at least one N is included;
-X₂- is -X₂₋₁-Y₁-; -X₂₋₁- is a chemical bond, non-natural amino acid residue, or natural amino acid residue; Y₁ is 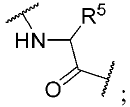
R⁵ is each independently C₁-C₄ alkylene or C₁-C₄ alkylene substituted by 1, 2, or 3 R⁵⁻¹;
R⁵⁻¹ is each independently halogen, hydroxyl, guanidino (-NHC(=NH)NH₂), carboxyl (-COOH), or amido (-CONH₂);
-X₃- is -X₃₋₁-Y₂-; -X₃₋₁- is a chemical bond, non-natural amino acid residue, or natural amino acid residue; Y₂ is 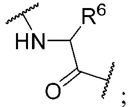
R⁶ is C₁-C₄ alkyl, C₁-C₄ alkyl substituted by 1 or 2 R⁶⁻¹, or C₃-C₆ cycloalkyl; each R⁶⁻¹ is independently halogen, C₃-C₆ cycloalkyl, C₆-C₁₀ aryl, or C₆-C₁₀ aryl substituted by one or more R⁶⁻¹⁻¹;
R⁶⁻¹⁻¹ is each independently halogen;
-X₄- is 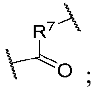 R⁷ is 3- to 6-membered heterocycloalkylene; the heteroatom of the 3- to 6-membered heterocycloalkylene is selected from one or more of N, O, and S, the number of heteroatoms is 1, 2, or 3, and at least one N is included;
W is -NH-CO- or -NH-CO-NH-;
R⁸ is linear C₁₋₁₀ alkyl;
R⁹ is H or C₁-C₄ alkyl substituted by 1 or 2 R⁹⁻¹; R⁹⁻¹ is carboxyl (-COOH) or amido (-CONH₂);
L₁ and L₂ are each independently a chemical bond, C₁-C₄ alkylene, or -S-(CH₂)ₖ-; k is 0, 1, 2, 3, or 4;
Cy is a chemical bond, 3- to 6-membered heterocycloalkylene, 5- to 6-membered heteroarylene, C₆-C₁₀ arylene, or C₆-C₁₀ arylene-L₄-R¹²; the heteroatom of the 3- to 6-membered heterocycloalkylene is selected from one or more of N, O, and S, the number of heteroatoms is 1, 2, or 3, and at least one N is included; the heteroatom of the 5- to 6-membered heteroarylene is selected from one or more of N, O, and S, the number of heteroatoms is 1, 2, or 3, and at least one N is included;
-L₃- is -L₃₋₁-L₃₋₂-L₃₋₃-;
-L₃₋₁ is a chemical bond, 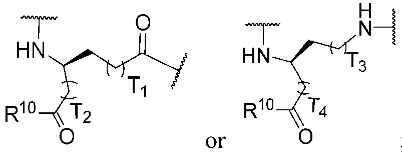 ; T₁ and T₂ are each independently 0, 1, 2, 3, or 4; T₃ and T₄ are each independently 0, 1, 2, 3, or 4;
-L₃₋₂- is -X₅-Y₃-; -X- is a chemical bond, a natural amino acid residue, or a polypeptide formed by 2-6 natural amino acids; -Y₃- is a chemical bond, 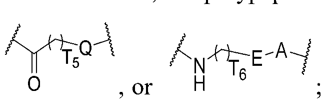 T₅ and T₆ are each independently 0, 1, 2, 3, or 4; E and Q are each independently C₁₋₆ alkylene or -(OCH₂CH₂)_{j'}-, and j' is 1, 2, 3, 4, 5, or 6; A is a chemical bond or -NH-;
-L₃₋₃- is a chemical bond, -NH-, -carbonyl-6- to 12-membered heteroarylene-, - carbonyl-C₁₋₆ alkylene-3- to 6-membered heterocycloalkylene-, -NH-C₁₋₆ alkylene-3- to 6-membered heterocycloalkylene-, or 3- to 6-membered heterocycloalkylene; the heteroatom of the 3- to 6-membered heterocycloalkylene is selected from one or more of N, O, and S, the number of heteroatoms is 1, 2, or 3, and at least one N is included; the heteroatom of the 6- to 12-membered heteroarylene is selected from one or more of N, O, and S, the number of heteroatoms is 1, 2, or 3, and at least one N is included;
R¹⁰ is hydroxyl or amino;
-L₄- is 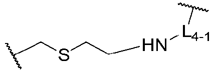 ; L₄₋₁ is a chemical bond, 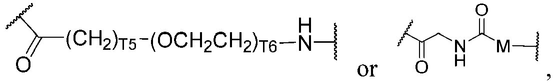 T₅ and T₆ are each independently a natural number from 0 to 10;
M is 6- to 12-membered heteroarylene; the heteroatom of the 6- to 12-membered heteroarylene is selected from one or more of N, O, and S, the number of heteroatoms is 1, 2, or 3, and at least one N is included;
R¹¹ and R¹² are each independently H or a chelating group; and when R¹¹ is H, R¹² is a chelating group, or when R¹² is H, R¹¹ is a chelating group;
the chelating group is 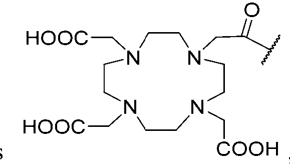 , NOTA, HBED-CC, NODAGA, NOTAGA, DOTAGA, TRAP, NOPO, PCTA, DFO, DTPA, CHX-DTPA, AAZTA, or DEDPA;
the compound X satisfies one or more of the following conditions:
   (1) -X₁- is 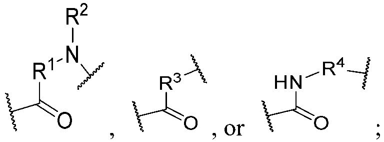
      R¹ is C₁-C₄ alkylene, C₃-C₆ cycloalkylene, C₁-C₄ alkylene-C₃-C₆ cycloalkylene, or C₁-C₄ alkylene-C₃-C₆ heterocycloalkylene;
      R² is hydrogen or C₁-C₄ alkyl;
      R³ is C₁-C₄ alkylene, C₁-C₄ alkylene-3- to 6-membered heterocycloalkylene, 4-membered or 6-membered heterocycloalkylene, or 6- to 12-membered heteroarylene; the heteroatom of the 3- to 6-membered heterocycloalkylene is selected from one or more of N, O, and S, the number of heteroatoms is 1, 2, or 3, and at least one N is included; the heteroatom of the 4-membered or 6-membered heterocycloalkylene is selected from one or more of N, O, and S, the number of heteroatoms is 1, 2, or 3, and at least one N is included; the heteroatom of the 6- to 12-membered heteroarylene is selected from one or more of N, O, and S, the number of heteroatoms is 1, 2, or 3, and at least one N is included;
      R⁴ is 3- to 6-membered heterocycloalkylene; the heteroatom of the 3- to 6-membered heterocycloalkylene is selected from one or more of N, O, and S, the number of heteroatoms is 1, 2, or 3, and at least one N is included;
   (2) -X₂₋₁- is a natural amino acid or a non-natural amino acid;
   (3) -X₃_₁- is a natural amino acid or a non-natural amino acid, or R⁶ is C₁-C₄ alkyl, C₁-C₄ alkyl substituted by 1 or 2 R⁶⁻¹, or C₃-C₆ cycloalkyl; each R⁶⁻¹ is independently halogen, C₃-C₆ cycloalkyl, or C₆-C₁₀ aryl substituted by one or more R⁶⁻¹⁻¹;
   (4) -X₄- is 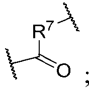 R⁷ is 4-membered heterocycloalkylene; the heteroatom of the 4-membered heterocycloalkylene is selected from one or more of N, O, and S, the number of heteroatoms is 1, 2, or 3, and at least one N is included;
   (5) L₄₋₁ is

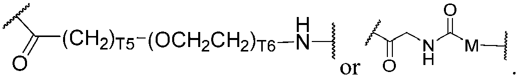

In one embodiment, the definitions of certain substituents in compound X may be as follows, and the definitions of substituents not mentioned are as described in any of the above embodiments:
In one embodiment, in R¹, the C₁-C₄ alkylene from the C₁-C₄ alkylene, the C₁-C₄ alkylene-C₃-C₆ cycloalkylene, and the C₁-C₄ alkylene-C₃-C₆ heterocycloalkylene is each independently methylene, ethylene, n-propylene, or isopropylene, for example, methylene.

In one embodiment, in R¹, the C₃-C₆ cycloalkylene from the C₁-C₄ alkylene-C₃-C₆ cycloalkylene is cyclobutylene (e.g., 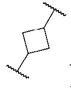 ) or cyclopropylene.

In one embodiment, in R¹, the C₃-C₆ heterocycloalkylene from the C₁-C₄ alkylene-C₃-C₆ heterocycloalkylene is C₄-C₆ heterocycloalkylene, where in the C₃-C₆ heterocycloalkylene, the heteroatom is preferably N, and the number of heteroatoms is preferably 1, for example, the C₃-C₆ heterocycloalkylene is piperidinylene (e.g., 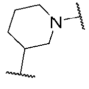 ).

In one embodiment, in R², the C₁-C₄ alkyl is methyl, ethyl, n-propyl, or isopropyl.

In one embodiment, in R³, the C₁-C₄ alkylene from the C₁-C₄ alkylene and the C₁-C₄ alkylene-3- to 6-membered heterocycloalkylene is each independently methylene, ethylene, n-propylene, or isopropylene.

In one embodiment, in R³, the 3- to 6-membered heterocycloalkylene from the C₁-C₄ alkylene-3- to 6-membered heterocycloalkylene and the 3- to 6-membered heterocycloalkylene is each independently 4- to 6-membered heterocycloalkylene, where in the 3- to 6-membered heterocycloalkylene, the heteroatom is preferably N, and the number of heteroatoms is preferably 1, for example, the 3- to 6-membered heterocycloalkylene is azetidinylene (e.g., 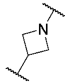 ).

In one embodiment, in R³, the 6- to 12-membered heteroarylene is 6- to 10-membered heteroarylene, where in the 6- to 12-membered heteroarylene, the heteroatom is preferably N, and the number of heteroatoms is preferably 1.

In one embodiment, in R⁴, the 3- to 6-membered heterocycloalkylene is 4- to 6-membered heterocycloalkylene, where in the 3- to 6-membered heterocycloalkylene, the heteroatom is preferably N, and the number of heteroatoms is preferably 1, for example, the 3-to 6-membered heterocycloalkylene is piperidinylene (e.g., 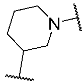 ).

In one embodiment, in -X₂₋₁-, the non-natural amino acid residue is D-alaninyl (e.g., 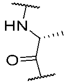 ) or

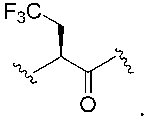

In one embodiment, in -X₂₋₁-, the natural amino acid residue is a glycinyl (e.g., 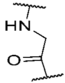 ). or an L-alanniyl (e.g., 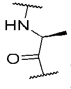 ).

In one embodiment, in R⁵, the C₁-C₄ alkylene from the C₁-C₄ alkylene and the C₁-C₄ alkylene substituted by 1, 2, or 3 R⁵⁻¹ is each independently methylene, ethylene, n-propylene, or isopropylene, for example ethylene or n-propylene.

In one embodiment, in -X₃₋₁-, the non-natural amino acid residue is

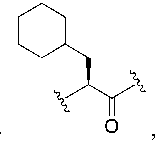

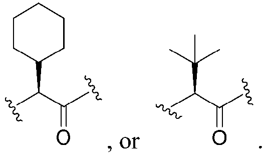

In one embodiment, in -X₃₋₁-, the natural amino acid residue is glycinyl (e.g., 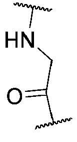 ), 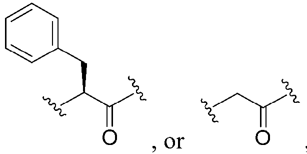 , for example, the natural amino acid residue is

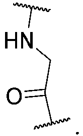

In one embodiment, in R⁶, the C₁-C₄ alkylene from the C₁-C₄ alkylene and the C₁-C₄ alkylene substituted by 1 or 2 R⁶⁻¹ is each independently methylene, ethylene, n-propylene, or isopropylene, for example methylene.

In one embodiment, in R⁶, the C₃-C₆ cycloalkyl is cyclobutyl, cyclopentyl, or cyclohexyl.

In one embodiment, in R⁶⁻¹, the C₃-C₆ cycloalkyl is cyclobutyl, cyclopentyl, or cyclohexyl, for example cyclohexyl (e.g., 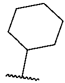 ).

In one embodiment, in R⁶⁻¹, the C₆-C₁₀ aryl from the C₆-C₁₀ aryl and the C₆-C₁₀ aryl substituted by one or more R⁶⁻¹⁻¹ is each independently phenyl or naphthyl, for example phenyl.

In one embodiment, in R⁶⁻¹⁻¹, the halogen is fluorine, chlorine, or bromine.

In one embodiment, in R⁷, the 3- to 6-membered heterocycloalkylene is 4- to 6-membered heterocycloalkylene, where in the 3- to 6-membered heterocycloalkylene, the heteroatom is preferably N, and the number of heteroatoms is preferably 1, for example, the 3-to 6-membered heterocycloalkylene is pyrrolidinylene (e.g., 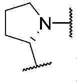 ).

In one embodiment, in R⁸, the C₁₋₁₀ alkyl is methyl, ethyl, n-propyl, n-butyl, n-pentyl, n-hexyl, n-heptyl, n-octyl, or n-nonyl, for example methyl, n-pentyl, n-nonyl, n-heptyl, or n-butyl.

In one embodiment, in R⁹, the C₁-C₄ alkyl is methyl, ethyl, n-propyl, or isopropyl, for example ethyl (e.g., 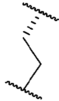 ).

In one embodiment, in L₁ and L₂, the C₁-C₄ alkylene is methylene, ethylene, n-propylene, or isopropylene, for example n-propylene.

In one embodiment, in Cy, the 3- to 6-membered heterocycloalkylene is 5- to 6-membered heterocycloalkylene, where in the 3- to 6-membered heterocycloalkylene, the heteroatom is preferably N, and the number of heteroatoms is preferably 3.

In one embodiment, in Cy, the heteroatom in the 5- to 6-membered heteroarylene is N, and the number of heteroatoms is preferably 1 or 3, for example, the 5- to 6-membered heteroarylene is pyridyl (e.g., 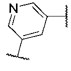 ) or 1H-1,2,3-triazole (e.g., 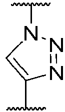 ).

In one embodiment, in Cy, the C₆-C₁₀ aryl from the C₆-C₁₀ arylene or C₆-C₁₀ arylene-L₄-R¹² is each independently phenyl or naphthyl, for example phenyl (e.g., 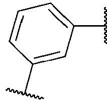 ).

In one embodiment, in L₃₋₂, the natural amino acid residue from the natural amino acid residue or the polypeptide formed by 2 to 6 natural amino acids is selected from one or more of glutamic acid, arginine, glycine, and aspartic acid; preferably, the polypeptide is formed by 3 natural amino acids; more preferably, the polypeptide is 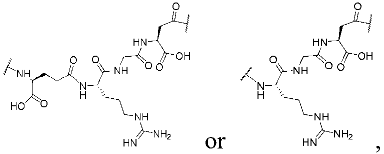 for example

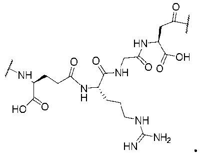

In one embodiment, in Y₃, the C₁₋₆ alkylene is linear C₁₋₆ alkylene; preferably, the C₁₋₆ alkylene is methyl, ethyl, n-propyl, n-butyl, n-pentyl, or n-hexyl, for example, methyl, ethyl, or n-propyl.

In one embodiment, in L₃₋₃, the 6- to 12-membered heteroarylene is 12-membered heteroarylene, where in the 6- to 12-membered heteroarylene, the heteroatom is preferably N, and the number of heteroatoms is preferably 1, for example, the 6- to 12-membered heteroarylene is

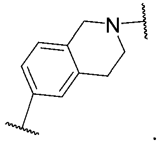

In one embodiment, in L₃₋₃, the 3- to 6-membered heterocycloalkylene from the carbonyl-C₁₋₆ alkylene-3- to 6-membered heterocycloalkylene, NH-C₁₋₆alkylene-3- to 6-membered heterocycloalkylene, and 3- to 6-membered heterocycloalkylene is each independently 4- to 6-membered heterocycloalkylene, the heteroatom is preferably N, and the number of heteroatoms is preferably 1 or 2, for example, the 3- to 6-membered heterocycloalkylene is piperidinylene (e.g., 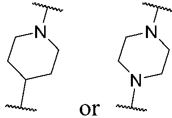 ).

In one embodiment, in L₃₋₃, the C₁₋₆ alkylene from the carbonyl-C₁₋₆ alkylene-3- to 6-membered heterocycloalkylene and NH-C₁₋₆ alkylene-3- to 6-membered heterocycloalkylene is each independently methyl, ethyl, n-propyl, n-butyl, n-pentyl, or n-hexyl, for example methyl, ethyl, or n-propyl.

In one embodiment, in -L₃₋₁-, 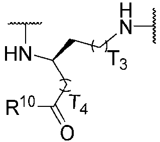 is

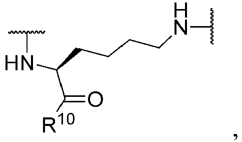

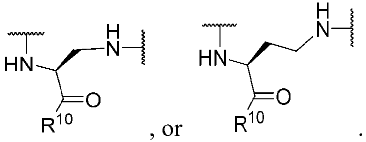

In one embodiment, in -L₃₋₁-, 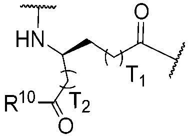 is 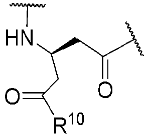 or

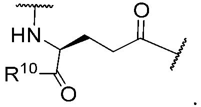

In one embodiment, -X₁- is 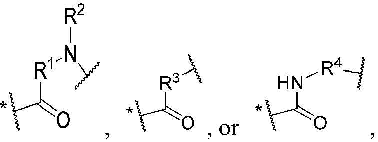 wherein 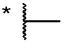 is connected to X₄.

In one embodiment, -X₂- is -X₂₋₁-Y₁-, wherein X₂₋₁ is connected to X₄.

In one embodiment, -X₃- is -X₃₋₁-Y₂-, wherein X₃₋₁ is connected to -NH- (the -NH- in 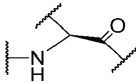 ) or carbonyl (the carbonyl in 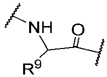 ).

In one embodiment, -X₄- is 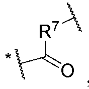 , wherein 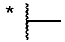 is connected to X₂.

In one embodiment, W is -NH-CO-* or -NH-CO-NH-*, wherein -* is connected to R⁸.

In one embodiment, L₁ and L₂ are each independently a chemical bond, C₁-C₄ alkylene, or -S-(CH₂)ₖ-*, wherein -* is connected to Cy.

In one embodiment, -L₃- is -L₃₋₁-L₃₋₂-L₃₋₃-*, wherein -* is connected to R¹¹.

In one embodiment, -L₃₋₁- is a chemical bond, 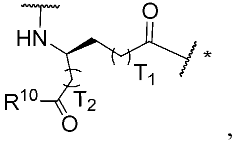 or 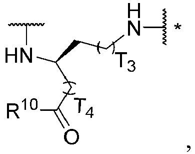 wherein 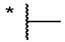 is connected to -L₃₋₂-.

In one embodiment, -L₃₋₂- is *-X₅-Y₃-, wherein *- is connected to -L₃₋₁-.

In one embodiment, -L₃₋₃- is a chemical bond, -NH-, -carbonyl-6- to 12-membered heteroarylene-*, -carbonyl-C₁₋₆ alkylene-3- to 6-membered heterocycloalkylene-*, -NH-C₁₋₆ alkylene-3- to 6-membered heterocycloalkylene-*, or 3- to 6-membered heterocycloalkylene, wherein -* is connected to R¹¹.

In one embodiment, -L₄- is 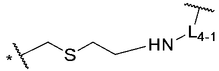 , wherein 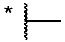 is connected to Cy.

In one embodiment, -X₁- is 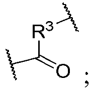 R³ is 3- to 6-membered heterocycloalkylene; the heteroatom of the 3- to 6-membered heterocycloalkylene is selected from one or more of N, O, and S, the number of heteroatoms is 1, 2, or 3, and at least one N is included.

In one embodiment, R¹ is C₃-C₆ cycloalkylene or C₁-C₄ alkylene-C₃-C₆ heterocycloalkylene, wherein the heteroatom of the 3- to 6-membered heterocycloalkylene is selected from one or more of N, O, and S, the number of heteroatoms is 1, 2, or 3, and at least one N is included.

In one embodiment, R² is hydrogen.

In one embodiment, R³ is 3- to 6-membered heterocycloalkylene; the heteroatom of the 3- to 6-membered heterocycloalkylene is selected from one or more of N, O, and S, the number of heteroatoms is 1, 2, or 3, and at least one N is included;

In one embodiment, -X₂₋₁- is a chemical bond, a non-natural amino acid residue, or a natural amino acid residue, wherein the unnatural amino acid residue is 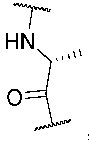 , and the natural amino acid residue is 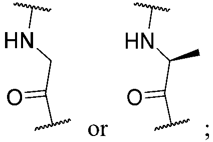 for example, -X₂₋₁- is a chemical bond.

In one embodiment, -X₂₋₁- is -X₂₋₁-Y₁-; -X₂₋₁- is a chemical bond, and Y₁ is

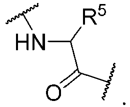

In one embodiment, R⁵ is each independently C₁-C₄ alkylene substituted by 1, 2, or 3 R⁵⁻¹.

In one embodiment, R⁵⁻¹ is each independently halogen, hydroxyl, guanidino (-NHC(=NH)NH₂), or amido, for example hydroxyl, guanidino (-NHC(=NH)NH₂), or amido, such as hydroxyl.

In one embodiment, -X₃₋₁- is a chemical bond or a natural amino acid residue, wherein the natural amino acid residue is, for example, 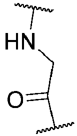 , and for example, -X₃₋₁- is a chemical bond.

In one embodiment, -X₃- is -X₃₋₁-Y₂-; -X₃₋₁- is a chemical bond, and Y₂ is

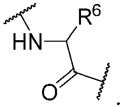

In one embodiment, R⁶ is C₁-C₄ alkyl substituted by 1 or 2 R⁶⁻¹.

In one embodiment, R⁶⁻¹ is each independently halogen, C₃-C₆ cycloalkyl, or C₆-C₁₀ aryl, for example C₃-C₆ cycloalkyl or C₆-C₁₀ aryl.

In one embodiment, R⁹ is C₁-C₄ alkyl substituted by 1 or 2 R⁹⁻¹, wherein R⁹⁻¹ is amido (-CONH₂); for example, R⁹ is C₁-C₄ alkyl substituted by 1 R⁹⁻¹, wherein R⁹⁻¹ is amido (-CONH₂).

In one embodiment, L₁ and L₂ are -S-(CH₂)ₖ-; k is 1.

In one embodiment, k is 0 or 1, for example 1.

In one embodiment, Cy is a chemical bond, 5- to 6-membered heteroarylene, C₆-C₁₀ arylene, or C₆-C₁₀ arylene-L₄-R¹², where in the 5- to 6-membered heteroarylene, the heteroatom is selected from one or more of N, O, and S, the number of heteroatoms is 1, 2, or 3, and at least one N is included; for example, Cy is C₆-C₁₀ arylene (e.g., phenylene).

In one embodiment, Cy is a chemical bond, 3- to 6-membered heterocycloalkylene, 5-to 6-membered heteroarylene, or C₆-C₁₀ arylene; the heteroatom of the 3- to 6-membered heterocycloalkylene is selected from one or more of N, O, and S, the number of heteroatoms is 1, 2, or 3, and at least one N is included; in the 5- to 6-membered heteroarylene, the heteroatom is selected from one or more of N, O, and S, the number of heteroatoms is 1, 2, or 3, and at least one N is included;
R¹¹ is a chelating group;
preferably, Cy is C₆-C₁₀ arylene (e.g., 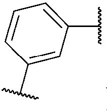 ); R¹¹ is a chelating group.

In one embodiment, T₁ and T₂ are each independently 0 or 1.

In one embodiment, T₃ and T₄ are each independently 0, 1, or 3, for example 0 or 1.

In one embodiment, -L₃₋₂- is -X₅-Y₃-; -X₅- is a chemical bond or a polypeptide formed by 2-6 natural amino acids, and -Y₃- is a chemical bond, 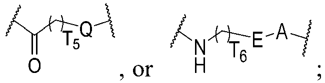 for example, -X₅- is a chemical bond and -Y₃- is 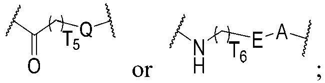 alternatively, - X₅- is a polypeptide formed by 2-6 natural amino acids and -Y₃- is a chemical bond or 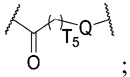 as another example, -X₃- is a chemical bond and -Y₃- is a chemical bond, 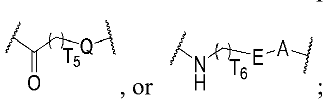 A is a chemical bond or -NH-.

In one embodiment, -L₃₋₁ is

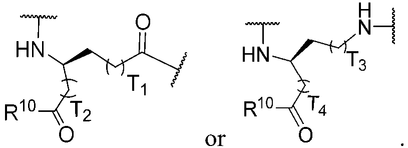

In one embodiment, -L₃₋₂- is -X₅-Y₃-; -X₅- is a chemical bond and -Y₃- is a chemical bond, 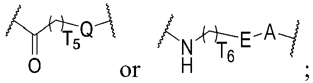 T₅ and T₆ are each independently 0, 1, 2, or 4, and E and Q are each independently C₁₋₆ alkylene; A is a chemical bond or -NH-.

In one embodiment, -L₃₋₃- is a chemical bond, -NH-, -carbonyl-C₁₋₆ alkylene-3- to 6-membered heterocycloalkylene-, -NH-C₁₋₆ alkylene-3- to 6-membered heterocycloalkylene-; for example -L₃₋₃- is -NH-, -carbonyl-C₁₋₆ alkylene-3- to 6-membered heterocycloalkylene-, - NH-C₁₋₆ alkylene-3- to 6-membered heterocycloalkylene-; the heteroatom of the 3- to 6-membered heterocycloalkylene is selected from one or more of N, O, and S, the number of heteroatoms is 1, 2, or 3, and at least one N is included.

In one embodiment, -L₃₋₁ is 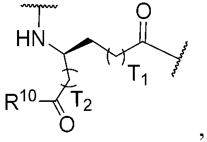 wherein T₁ and T₂ are each independently 0, 1, 2, 3, or 4;
-L₃₋₂- is -X₅-Y₃-; -X₅- is a chemical bond and -Y₃- is 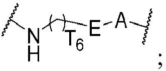 T₆ is 0, 1, 2, 3, or 4, and E is C₁₋₆ alkylene or -(OCH₂CH₂)_{j'}-, wherein j' is 1, 2, 3, 4, 5, or 6 (e.g., 1); A is a chemical bond or -NH-;
-L₃₋₃- is a chemical bond.

In one embodiment, -L₃₋₁ is 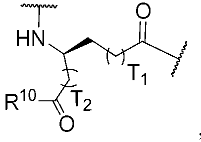 , wherein T₁ and T₂ are each independently 0, 1, 2, 3, or 4;
-L₃₋₂- is -X₅-Y₃-; -X₅- is a chemical bond and -Y₃- is a chemical bond;
-L₃₋₃- is -NH-C₁₋₆ alkylene-3- to 6-membered heterocycloalkylene- or 3- to 6-membered heterocycloalkylene, wherein the heteroatom of the 3- to 6-membered heterocycloalkylene is selected from one or more of N, O, and S, the number of heteroatoms is 1, 2, or 3, and at least one N is included.

In one embodiment, -L₃- is -L₃₋₁-L₃₋₂-L₃₋₃-;
-L₃₋₁ is 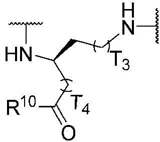 , wherein T₃ and T₄ are each independently 0, 1, 2, 3, or 4;
-L₃₋₂- is -X₅-Y₃-; -X₅- is a chemical bond and -Y₃- is 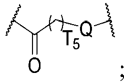 T₅ is 0, 1, 2, 3, or 4, and Q is C₁₋₆ alkylene or -(OCH₂CH₂)_{j'}-, wherein j' is 1, 2, 3, 4, 5, or 6;
-L₃₋₃- is -NH-.

In one embodiment, -L₃₋₁ is 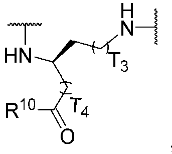 , wherein T₃ and T₄ are each independently 0, 1, 2, 3, or 4;
-L₃₋₂- is -X₅-Y₃-; -X₅- is a chemical bond and -Y₃- is a chemical bond;
-L₃₋₃- is -carbonyl-6- to 12-membered heteroarylene- or -carbonyl-C₁₋₆ alkylene-3- to 6-membered heterocycloalkylene- (e.g., -carbonyl-C₁₋₆ alkylene-3- to 6-membered heterocycloalkylene-), wherein the heteroatom of the 3- to 6-membered heterocycloalkylene is selected from one or more of N, O, and S, the number of heteroatoms is 1, 2, or 3, and at least one N is included; the heteroatom of the 6- to 12-membered heteroarylene is selected from one or more of N, O, and S, the number of heteroatoms is 1, 2, or 3, and at least one N is included.

In one embodiment, -L₃₋₁ is 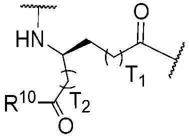 , wherein T₁ and T₂ are each independently 0, 1, 2, 3, or 4;
-L₃₋₂- is -X₅-Y₃-; -X₅- is a natural amino acid residue or a polypeptide formed by 2-6 natural amino acids (e.g., 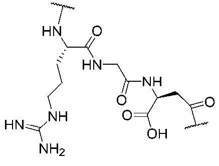 ) and -Y₃- is a chemical bond;
-L₃₋₃- is a chemical bond.

In one embodiment, E and Q are each independently C₁₋₆ alkylene.

In one embodiment, T₅ and T₆ are each independently 0, 1, 2, or 4, for example 0, 1, or 2.

In one embodiment, j' is 1, 2, or 3.

In one embodiment, R¹⁰ is hydroxyl.

In one embodiment, -L₄- is 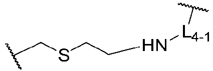 ; L₄₋₁ is a chemical bond.

In one embodiment, the chelating group is

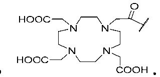

In one embodiment, R¹¹ is a chelating group; the chelating group is

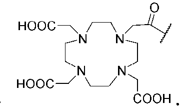

In one embodiment, the compound X satisfies one or more of the following conditions:
(1) -X₁- is 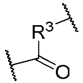 ; R³ is 4-membered or 6-membered heterocycloalkylene; the heteroatom of the 4- to 6-membered heterocycloalkylene is selected from one or more of N, O, and S, the number of heteroatoms is 1, 2, or 3, and at least one N is included;
(2) R⁶ is C₁-C₄ alkyl substituted by 1 or 2 R⁶⁻¹; each R⁶⁻¹ is independently C₃-C₆ cycloalkyl.

In one technical solution, the compound X is compound X-1, compound X-2, compound X-3, or compound X-4, 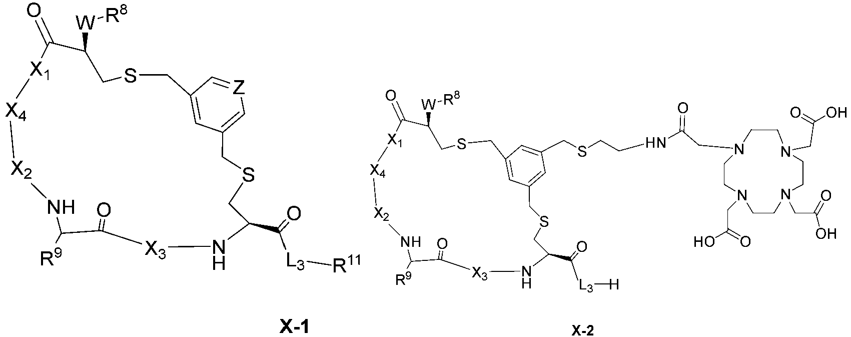 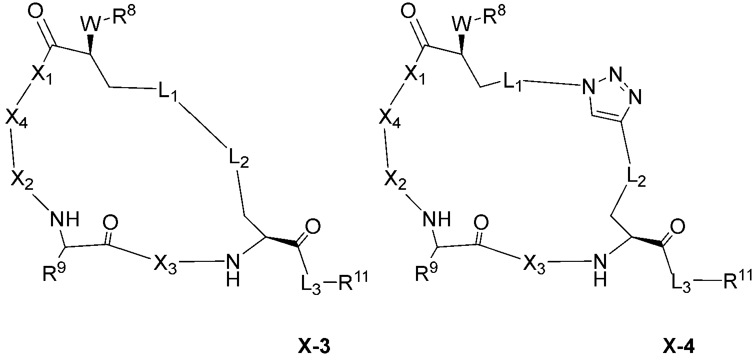
Z is N or CH;
X₁, X₂, X₃, X₄, R⁹, R⁸, L₁, W, L₂, L₃, and R¹¹ are as defined in any one of the embodiments of the present disclosure.

In one technical solution, the compound X is compound X-5, 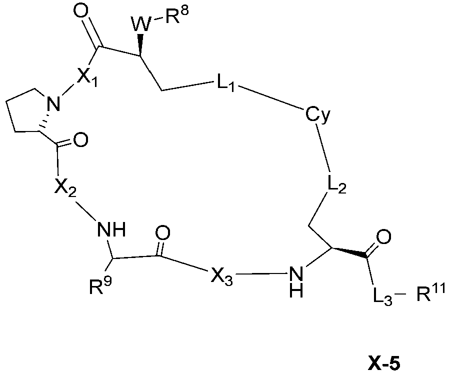 X₁, X₂, X₃, R⁹, R⁸, W, L₁, L₂, Cy, L₃, and R¹¹ are as defined in any one of the embodiments of the present disclosure.

In one technical solution, the compound X is compound X-6, compound X-7, compound X-8, or compound X-9, 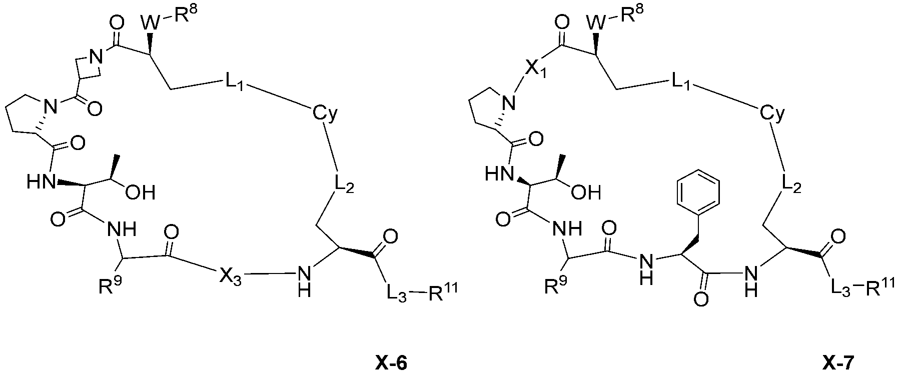 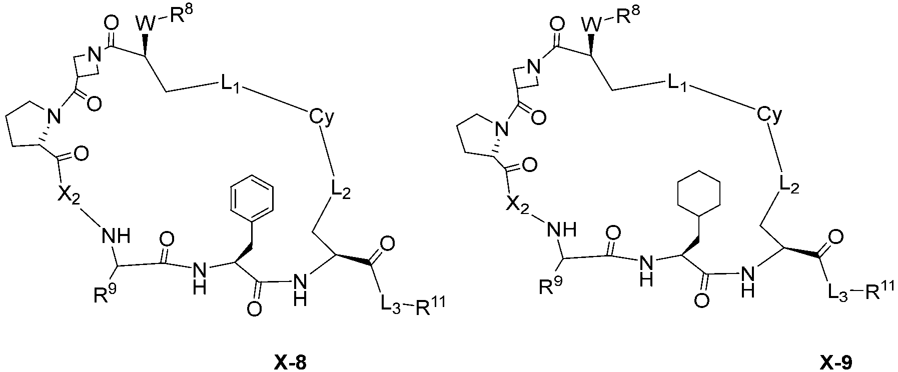 wherein X₁, X₂, R⁹, R⁸, L₁, L₂, Cy, L₃, and R¹¹ are as defined in any one of the embodiments of the present disclosure.

In one embodiment, in the compound X,
-X₁- is 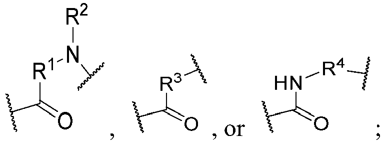
R¹ is C₃-C₆ cycloalkylene or C₁-C₄ alkylene-C₃-C₆ heterocycloalkylene, wherein the heteroatom of the 3- to 6-membered heterocycloalkylene is selected from one or more of N, O, and S, the number of heteroatoms is 1, 2, or 3, and at least one N is included;
R² is hydrogen;
R³ is 3- to 6-membered heterocycloalkylene; the heteroatom of the 3- to 6-membered heterocycloalkylene is selected from one or more of N, O, and S, the number of heteroatoms is 1, 2, or 3, and at least one N is included;
R⁴ is 3- to 6-membered heterocycloalkylene; the heteroatom of the 3- to 6-membered heterocycloalkylene is selected from one or more of N, O, and S, the number of heteroatoms is 1, 2, or 3, and at least one N is included;
-X₂- is -X₂₋₁-Y₁-; -X₂₋₁- is a chemical bond, non-natural amino acid residue, or natural amino acid residue,
the non-natural amino acid residue is 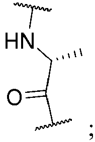 the natural amino acid residue is 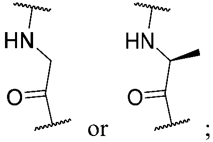 Y₁ is 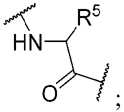
R⁵ *is* each independently C₁-C₄ alkylene substituted by 1, 2, or 3 R⁵⁻¹.
R⁵⁻¹ is each independently hydroxyl, guanidino, or amido;
-X₃- is -X₃₋₁-Y₂-; -X₃₋₁- is a chemical bond or a natural amino acid residue,
the natural amino acid residue is 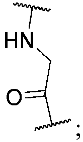 Y₂ is 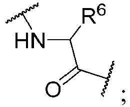
R⁶ is C₁-C₄ alkyl substituted by 1 or 2 R⁶⁻¹; each R⁶⁻¹ is independently halogen, C₃-C₆ cycloalkyl, or C₆-C₁₀ aryl;
-X₄- is 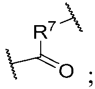 R⁷ is 3- to 6-membered heterocycloalkylene; the heteroatom of the 3- to 6-membered heterocycloalkylene is selected from one or more of N, O, and S, the number of heteroatoms is 1, 2, or 3, and at least one N is included;
W is -NH-CO- or -NH-CO-NH-;
R⁸ is linear C₁₋₁₀ alkyl;
R⁹ is H or C₁-C₄ alkyl substituted by 1 or 2 R⁹⁻¹; R⁹⁻¹ is carboxyl (-COOH) or amido (-CONH₂);
L₁ and L₂ are each independently a chemical bond, C₁-C₄ alkylene, or -S-(CH₂)ₖ-; k is 0 or 1;
Cy is a chemical bond, 5- to 6-membered heteroarylene, C₆-C₁₀ arylene, or C₆-C₁₀ arylene-L₄-R¹²; in the 5- to 6-membered heteroarylene, the heteroatom is selected from one or more of N, O, and S, the number of heteroatoms is 1, 2, or 3, and at least one N is included;
-L₃- is -L₃₋₁-L₃₋₂-L₃₋₃-;
-L₃₋₁ is a chemical bond, 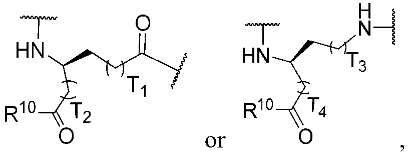 T₁ and T₂ are each independently 0 or 1, and T₃ and T₄ are each independently 0, 1, or 3;
-L₃₋₂- is -X₅-Y₃-; -X₅- is a chemical bond or a polypeptide formed by 2-6 natural amino acids; -Y₃- is a chemical bond, 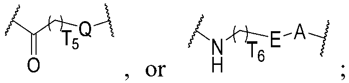 T₅ and T₆ are each independently 0, 1, 2, or 4; E and Q are each independently C₁₋₆ alkylene or -(OCH₂CH₂)_{j'}-, and j' is 1, 2, or 3; A is a chemical bond or -NH-;
-L₃₋₃- is a chemical bond, -NH-, -carbonyl-6- to 12-membered heteroarylene-, - carbonyl-C₁₋₆ alkylene-3- to 6-membered heterocycloalkylene-, -NH-C₁₋₆ alkylene-3- to 6-membered heterocycloalkylene-, or 3- to 6-membered heterocycloalkylene; the heteroatom of the 3- to 6-membered heterocycloalkylene is selected from one or more of N, O, and S, the number of heteroatoms is 1, 2, or 3, and at least one N is included; the heteroatom of the 6- to 12-membered heteroarylene is selected from one or more of N, O, and S, the number of heteroatoms is 1, 2, or 3, and at least one N is included;
R¹⁰ is hydroxyl or amino;
L₄ is 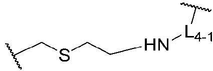 ; L₄₋₁ is a chemical bond;
R¹² and R¹¹ are each independently H or a chelating group; and when R¹¹ is H, R¹² is a chelating group, or when R¹² is H, R¹¹ is a chelating group;
the chelating group is 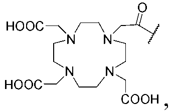 NOTA, HBED-CC, NODAGA, NOTAGA, DOTAGA, TRAP, NOPO, PCTA, DFO, DTPA, CHX-DTPA, AAZTA, or DEDPA;
the compound X satisfies one or more of the following conditions:
   (1) -X₁- is 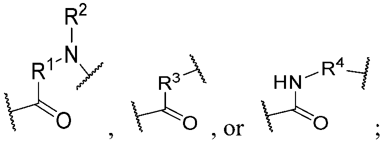
      R¹ is C₃-C₆ cycloalkylene or C₁-C₄ alkylene-C₃-C₆ heterocycloalkylene;
      R² is hydrogen;
      R³ is 4-membered or 6-membered heterocycloalkylene; the heteroatom of the 4-membered or 6-membered heterocycloalkylene is selected from one or more of N, O, and S, the number of heteroatoms is 1, 2, or 3, and at least one N is included;
      R⁴ is 3- to 6-membered heterocycloalkylene; the heteroatom of the 3- to 6-membered heterocycloalkylene is selected from one or more of N, O, and S, the number of heteroatoms is 1, 2, or 3, and at least one N is included;
   (2) -X₂₋₁- is a natural amino acid or a non-natural amino acid;
   (3) -X₃₋₁- is a natural amino acid; or R⁶ is C₁-C₄ alkyl substituted by 1 or 2 R⁶⁻¹; each R⁶⁻¹ is independently C₃-C₆ cycloalkyl or halogen;
   (4) -X₄- is 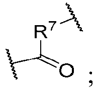 R⁷ is 4-membered heterocycloalkylene; the heteroatom of the 4-membered heterocycloalkylene is selected from one or more of N, O, and S, the number of heteroatoms is 1, 2, or 3, and at least one N is included.

In one embodiment, in the compound X,
-X₁- is 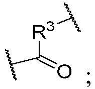
R³ is 3- to 6-membered heterocycloalkylene; the heteroatom of the 3- to 6-membered heterocycloalkylene is selected from one or more of N, O, and S, the number of heteroatoms is 1, 2, or 3, and at least one N is included;
-X₂- is -X₂₋₁-Y₁-; -X₂₋₁- is a chemical bond and Y₁ is 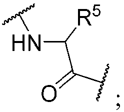
R⁵ *is* each independently C₁-C₄ alkylene substituted by 1, 2, or 3 R⁵⁻¹.
R⁵⁻¹ is each independently hydroxyl;
-X₃- is -X₃₋₁-Y₂-; -X₃₋₁- is a chemical bond and Y₂ is 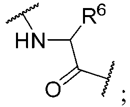
R⁶ is C₁-C₄ alkyl substituted by 1 or 2 R⁶⁻¹; each R⁶⁻¹ is independently C₃-C₆ cycloalkyl or C₆-C₁₀ aryl;
-X₄- is 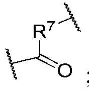 ; R⁷ is 3- to 6-membered heterocycloalkylene; the heteroatom of the 3- to 6-membered heterocycloalkylene is selected from one or more of N, O, and S, the number of heteroatoms is 1, 2, or 3, and at least one N is included;
W is -NH-CO- or -NH-CO-NH-;
R⁸ is linear C₁₋₁₀ alkyl;
R⁹ is H or C₁-C₄ alkyl substituted by 1 or 2 R⁹⁻¹; R⁹⁻¹ is amido (-CONH₂);
L₁ and L₂ are each independently -S-(CH₂)ₖ-; k is 1;
Cy is C₆-C₁₀ arylene;
-L₃- is -L₃₋₁-L₃₋₂-L₃-₃-;
-L₃₋₁ is a chemical bond, 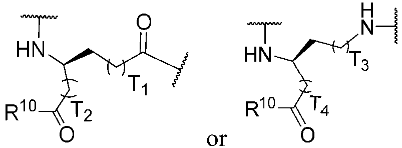 , T₁ and T₂ are each independently 0 or 1, and T₃ and T₄ are each independently 0 or 1;
-L₃₋₂- is -X₅-Y₃-; -X₅- is a chemical bond and -Y₃- is a chemical bond, 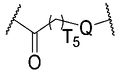 or 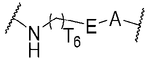 ; T₅ and T₆ are each independently 0, 1, or 2, and E and Q are each independently C₁₋₆ alkylene; A is a chemical bond or -NH-;
-L₃₋₃- is a chemical bond, -NH-, -carbonyl-C₁₋₆ alkylene-3- to 6-membered heterocycloalkylene-, -NH-C₁₋₆ alkylene-3- to 6-membered heterocycloalkylene-, or 3- to 6-membered heterocycloalkylene, wherein the heteroatom of the 3- to 6-membered heterocycloalkylene is selected from one or more of N, O, and S, the number of heteroatoms is 1, 2, or 3, and at least one N is included;
R¹⁰ is hydroxyl;
R¹¹ is a chelating group; the chelating group is 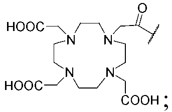
the compound X satisfies one or more of the following conditions:
   (1) -X₁- is 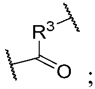 R³ is 4-membered or 6-membered heterocycloalkylene; the heteroatom of the 4- to 6-membered heterocycloalkylene is selected from one or more of N, O, and S, the number of heteroatoms is 1, 2, or 3, and at least one N is included;
   (2) R⁶ is C₁-C₄ alkyl substituted by 1 or 2 R⁶⁻¹; each R⁶⁻¹ is independently C₃-C₆ cycloalkyl.

In one embodiment, in the compound X,
-X₁- is 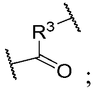
R³ is 3- to 6-membered heterocycloalkylene; the heteroatom of the 3- to 6-membered heterocycloalkylene is selected from one or more of N, O, and S, the number of heteroatoms is 1, 2, or 3, and at least one N is included;
-X₂- is -X₂₋₁-Y₁-; -X₂₋₁- is a chemical bond and Y₁ is 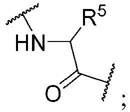
R⁵ *is* each independently C₁-C₄ alkylene substituted by 1, 2, or 3 R⁵⁻¹.
R⁵⁻¹ is each independently hydroxyl;
-X₃- is -X₃₋₁-Y₂-; -X₃₋₁- is a chemical bond and Y₂ is 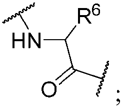
R⁶ is C₁-C₄ alkyl substituted by 1 or 2 R⁶⁻¹; each R⁶⁻¹ is independently C₃-C₆ cycloalkyl or C₆-C₁₀ aryl;
-X₄- is 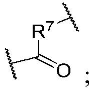 R⁷ is 3- to 6-membered heterocycloalkylene; the heteroatom of the 3- to 6-membered heterocycloalkylene is selected from one or more of N, O, and S, the number of heteroatoms is 1, 2, or 3, and at least one N is included;
W is -NH-CO- or -NH-CO-NH-;
R⁸ is linear C₁₋₁₀ alkyl;
R⁹ is C₁-C₄ alkyl substituted by 1 R⁹⁻¹; R⁹⁻¹ is amido (-CONH₂);
L₁ and L₂ are each independently -S-(CH₂)ₖ-; k is 1;
Cy is C₆-C₁₀ arylene;
-L₃- is -L₃₋₁-L₃₋₂-L₃₋₃-;
-L₃₋₁ is 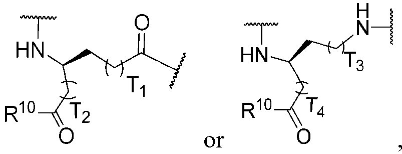 T₁ and T₂ are each independently 0 or 1, and T₃ and T₄ are each independently 0 or 1;
-L₃₋₂- is -X₃-Y₃-; -X₃- is a chemical bond, and -Y₃- is a chemical bond, 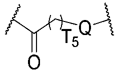 or 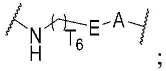 T₅ and T₆ are each independently 0, 1, or 2, and E and Q are each independently C₁₋₆ alkylene or -(OCH₂CH₂)_{j'}-, wherein j' is 1; A is a chemical bond or -NH-;
-L₃₋₃- is a chemical bond, -NH-, -carbonyl-C₁₋₆ alkylene-3- to 6-membered heterocycloalkylene- or -NH-C₁₋₆ alkylene-3- to 6-membered heterocycloalkylene-, wherein the heteroatom of the 3- to 6-membered heterocycloalkylene is selected from one or more of N, O, and S, the number of heteroatoms is 1, 2, or 3, and at least one N is included;
R¹⁰ is hydroxyl or amino;
R¹¹ is a chelating group;
the chelating group is 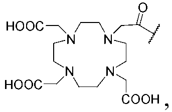 NOTA, HBED-CC, NODAGA, NOTAGA, DOTAGA, TRAP, NOPO, PCTA, DFO, DTPA, CHX-DTPA, AAZTA, or DEDPA.

In one embodiment, in the compound X,
-X₁- is 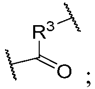
R³ is 3- to 6-membered heterocycloalkylene; the heteroatom of the 3- to 6-membered heterocycloalkylene is selected from one or more of N, O, and S, the number of heteroatoms is 1, 2, or 3, and at least one N is included;
-X₂- is -X₂₋₁-Y₁-; -X₂₋₁- is a chemical bond and Y₁ is 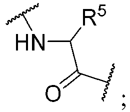
R⁵ is each independently C₁-C₄ alkylene substituted by 1, 2, or 3 R⁵⁻¹.
R⁵⁻¹ is each independently hydroxyl;
-X₃- is -X₃₋₁-Y₂-; -X₃₋₁- is a chemical bond and Y₂ is 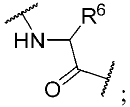
R⁶ is C₁-C₄ alkyl substituted by 1 or 2 R⁶⁻¹; each R⁶⁻¹ is independently C₃-C₆ cycloalkyl or C₆-C₁₀ aryl;
-X₄- is 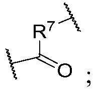 R⁷ is 3- to 6-membered heterocycloalkylene; the heteroatom of the 3- to 6-membered heterocycloalkylene is selected from one or more of N, O, and S, the number of heteroatoms is 1, 2, or 3, and at least one N is included;
W is -NH-CO- or -NH-CO-NH-;
R⁸ is linear C₁₋₁₀ alkyl;
R⁹ is C₁-C₄ alkyl substituted by 1 or 2 R⁹⁻¹; R⁹⁻¹ is amido (-CONH₂);
L₁ and L₂ are each independently -S-(CH₂)ₖ-; k is 1;
Cy is C₆-C₁₀ arylene;
-L₃- is -L₃₋₁-L₃₋₂-L₃₋₃-;
-L₃₋₁ is 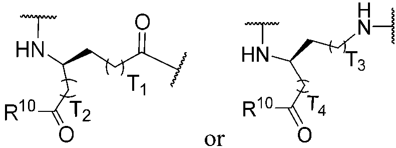 , T₁ and T₂ are each independently 0 or 1, and T₃ and T₄ are each independently 0 or 1;
-L₃₋₂- is -X₅-Y₃-; -X₅- is a chemical bond and -Y₃- is a chemical bond, 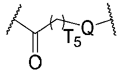 or 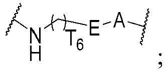 T₅ and T₆ are each independently 0, 1, 2, or 4, and E and Q are each independently C₁₋₆ alkylene; A is a chemical bond or -NH-;
-L₃₋₃- is -NH-, -carbonyl-C₁₋₆ alkylene-3- to 6-membered heterocycloalkylene- or - NH-C₁₋₆ alkylene-3- to 6-membered heterocycloalkylene-, wherein the heteroatom of the 3- to 6-membered heterocycloalkylene is selected from one or more of N, O, and S, the number of heteroatoms is 1, 2, or 3, and at least one N is included;
R¹⁰ is hydroxyl;
R¹¹ is a chelating group;
the chelating group is 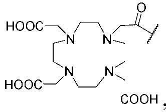 , NOTA, HBED-CC, NODAGA, NOTAGA, DOTAGA, TRAP, NOPO, PCTA, DFO, DTPA, CHX-DTPA, AAZTA, or DEDPA.

In one embodiment, -X₁- is 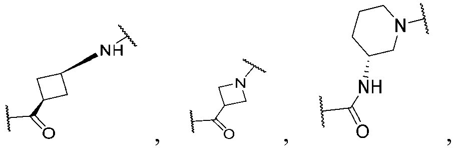 or 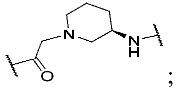 preferably, -X₁- is 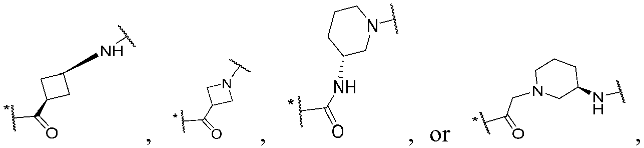 wherein 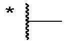 is connected to X₄.

In one embodiment, -X₄- is 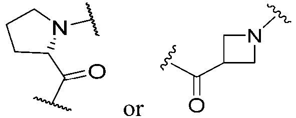 , for example 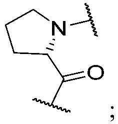 preferably, -X₄- is 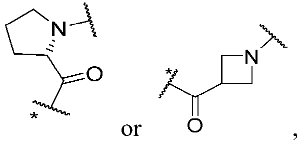 wherein 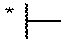 is connected to X₂.

In one embodiment, -X₂- is 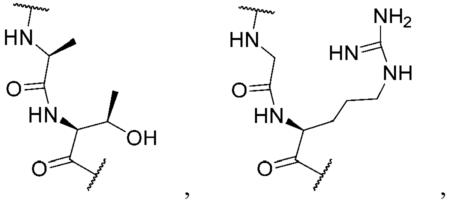 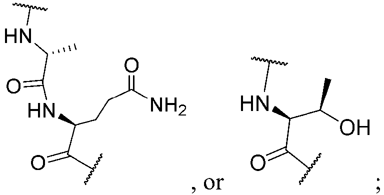 preferably, -X₂- is 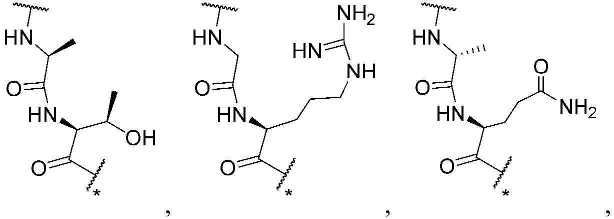 or 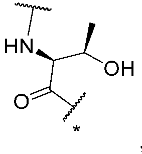 , wherein 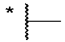 is connected to -NH- (the -NH- in 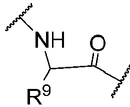 ).

In one embodiment, -X₃- is 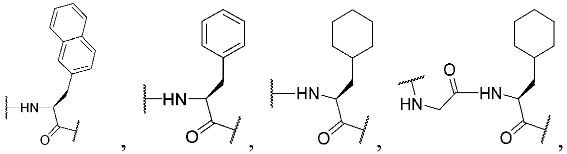 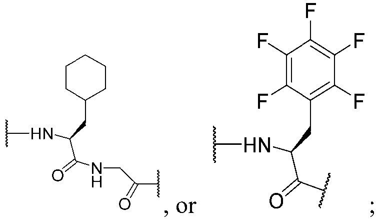 preferably, -X₃-is 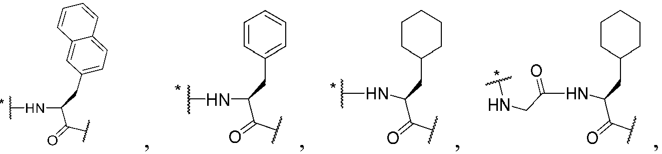 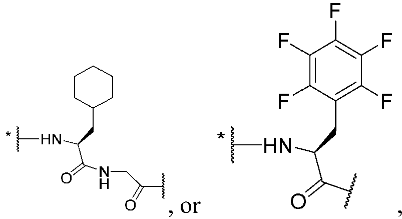 wherein 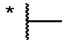 is connected to carbonyl (the -CO- in 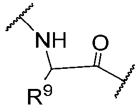 ).

In one embodiment, -L₄- is 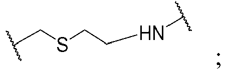 preferably, -L₄- is 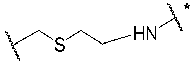 , wherein 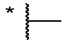 is connected to R¹¹.

In one embodiment, -L₃₋₁- is a chemical bond, 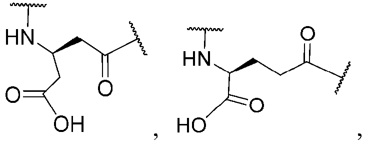 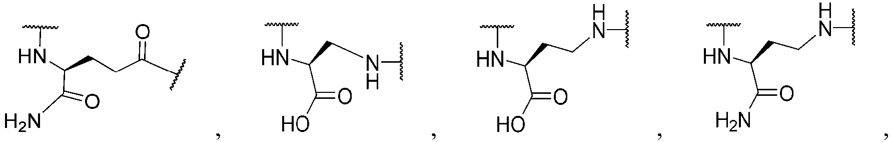 or 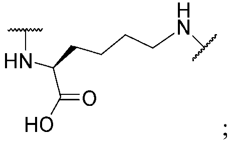 preferably, -L₃₋₁- is a chemical bond, 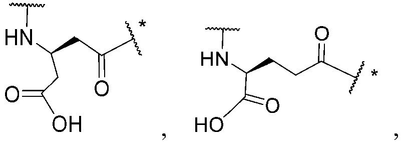 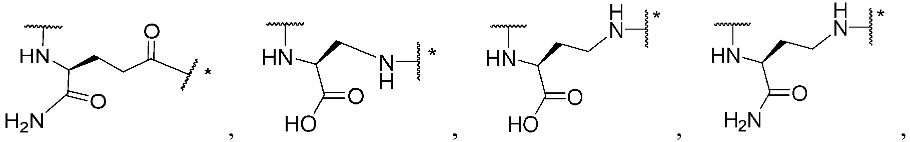 or 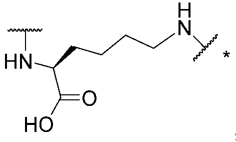 , wherein 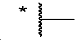 is connected to -L₃₋₂-.

In one embodiment, -L₃₋₂- is a chemical bond,

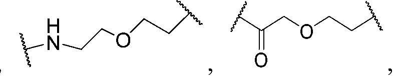

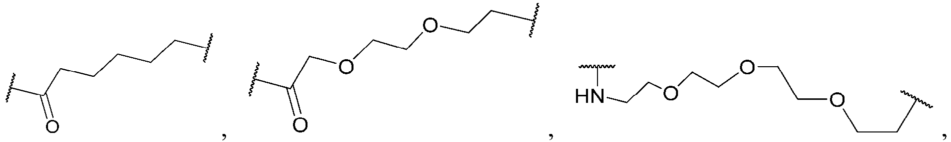

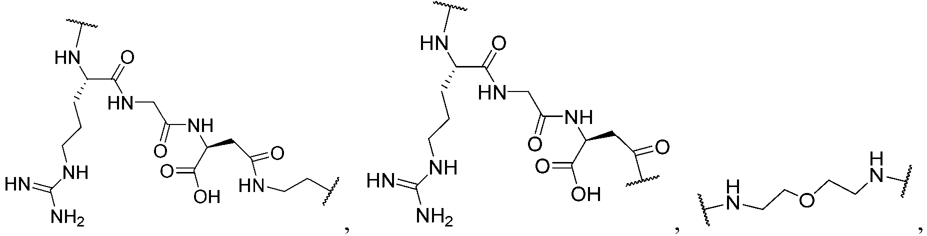

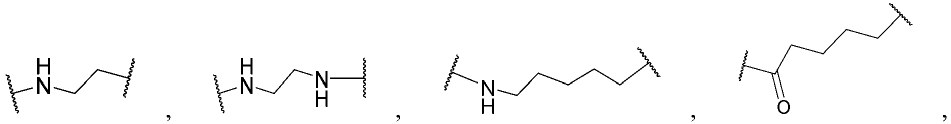

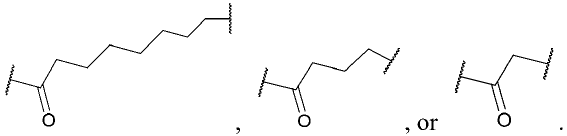

Preferably, -L₃₋₂- is a chemical bond, 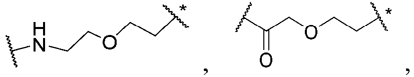 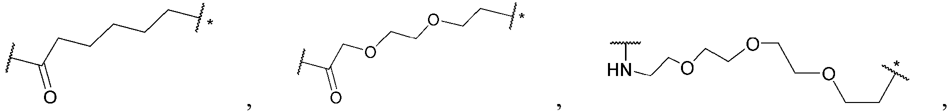 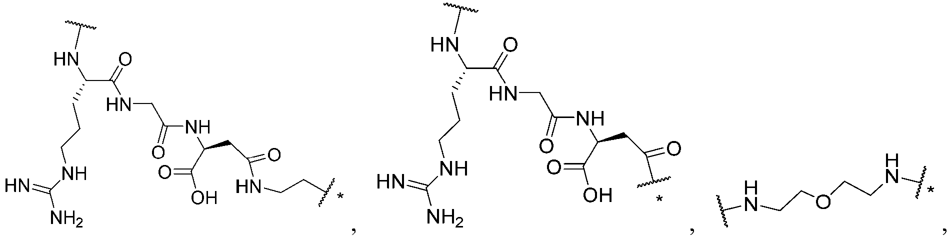 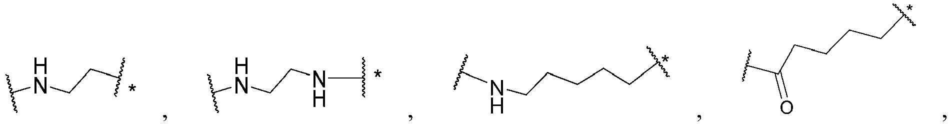 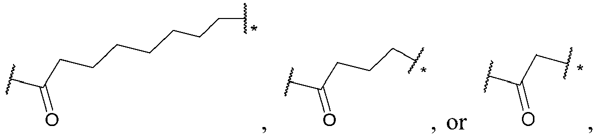 L₃₋₃-. wherein 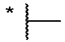 is connected to -

In one embodiment, -L₃₋₃- is a chemical bond, -NH-, 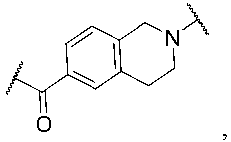 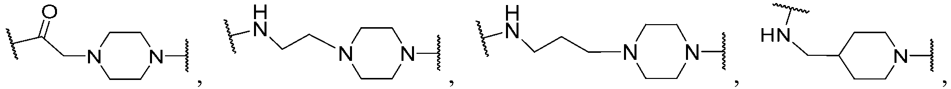 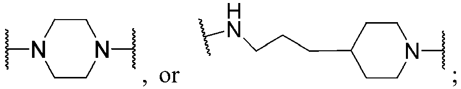 preferably, -L₃₋₃- is a chemical bond, 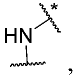 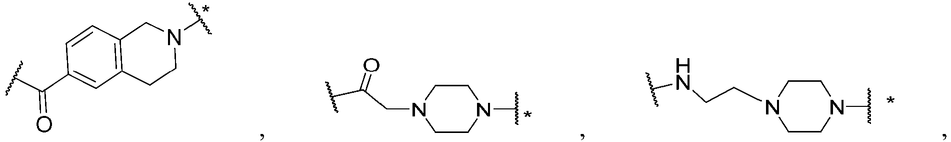 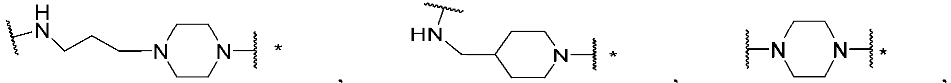 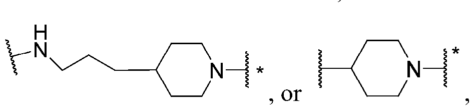 wherein 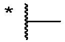 is connected to R¹¹.

In one embodiment, R⁸ is linear C₁₋₁₀ alkyl, for example methyl, n-pentyl, n-nonyl, n-heptyl, or n-butyl.

In one embodiment, R⁹ is H,

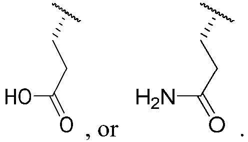

In one embodiment, R¹⁰ is -OH or -NH₂.

In one embodiment, L₁ and L₂ are each independently -S-methylene-, n-propylene, a chemical bond, or -S-.

In one embodiment, Cy is 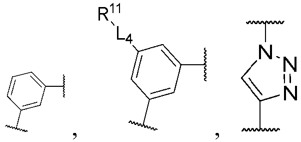 (e.g., 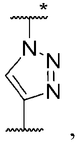 wherein 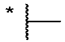 is connected to L₁), a chemical bond, or

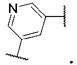

In one embodiment, the structure of the compound X is any one of the following:

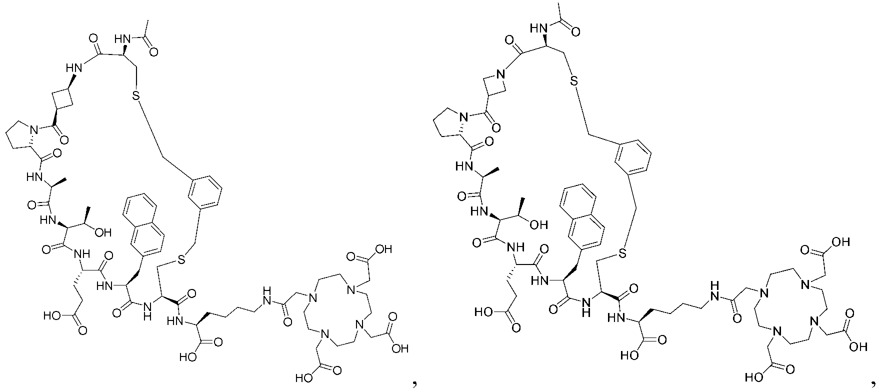

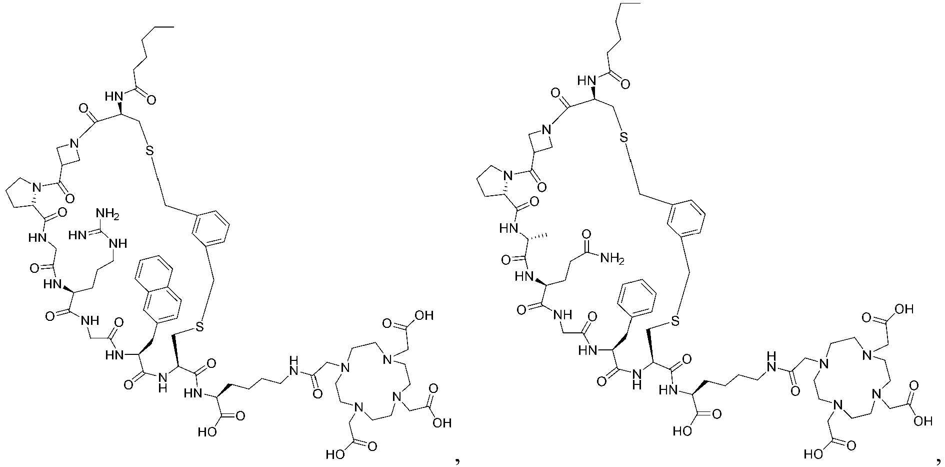

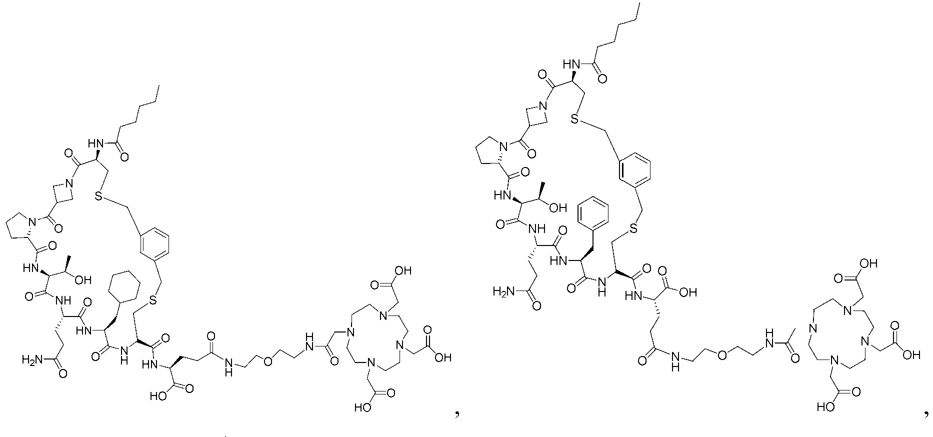

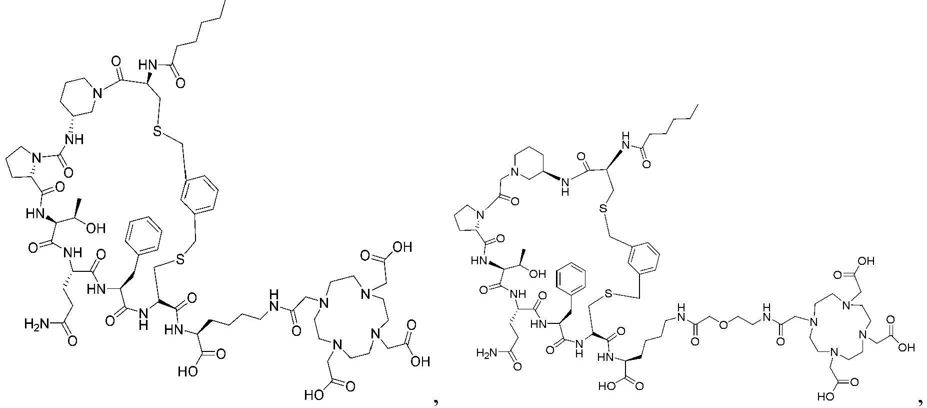

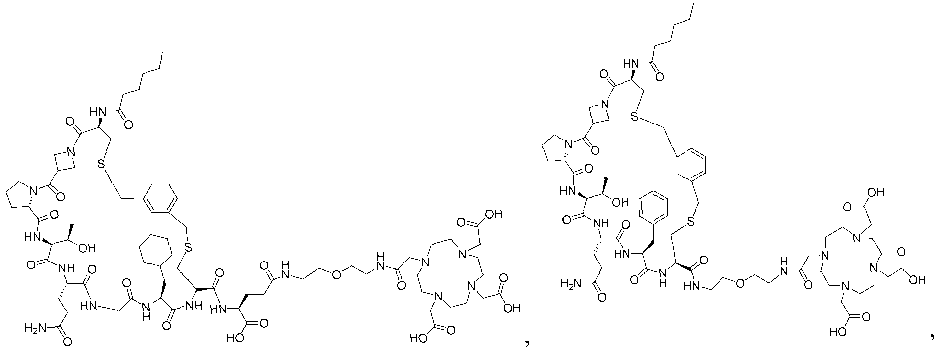

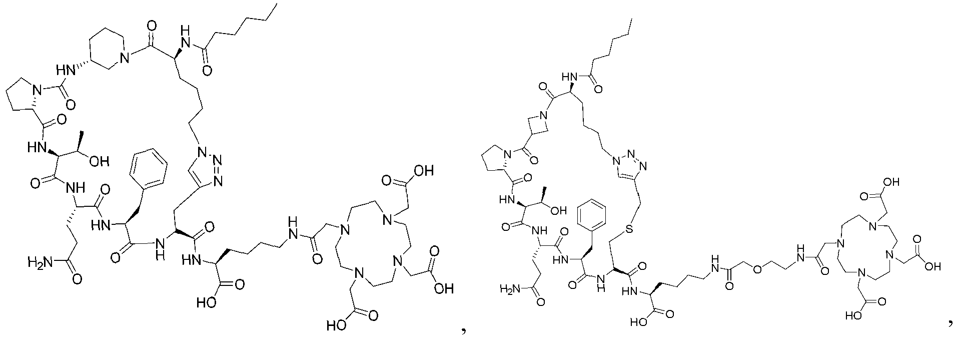

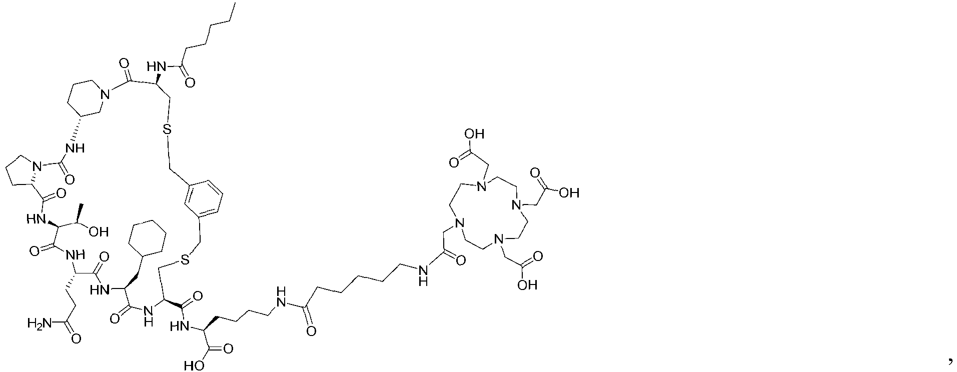

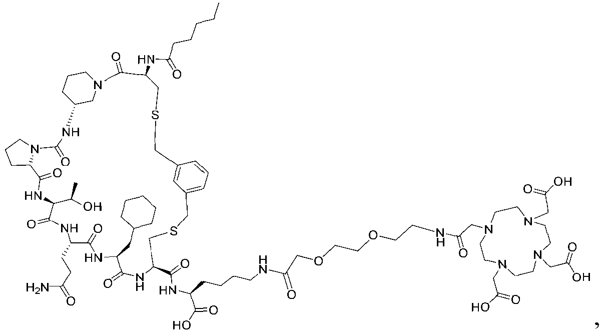

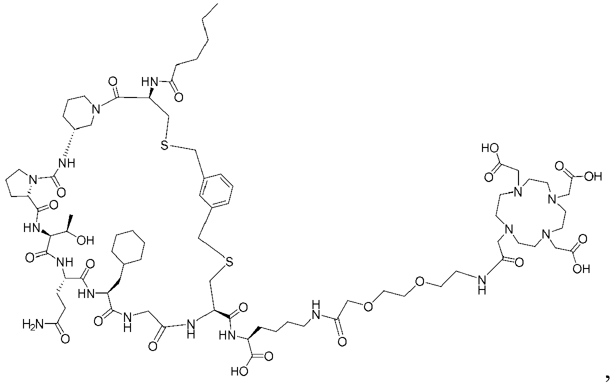

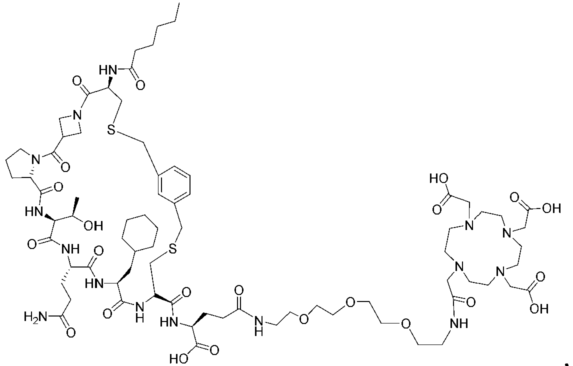

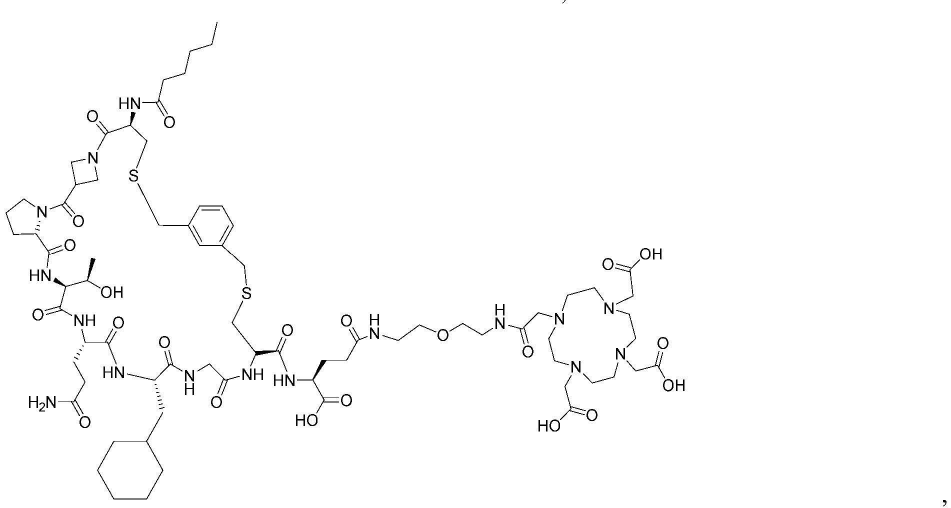

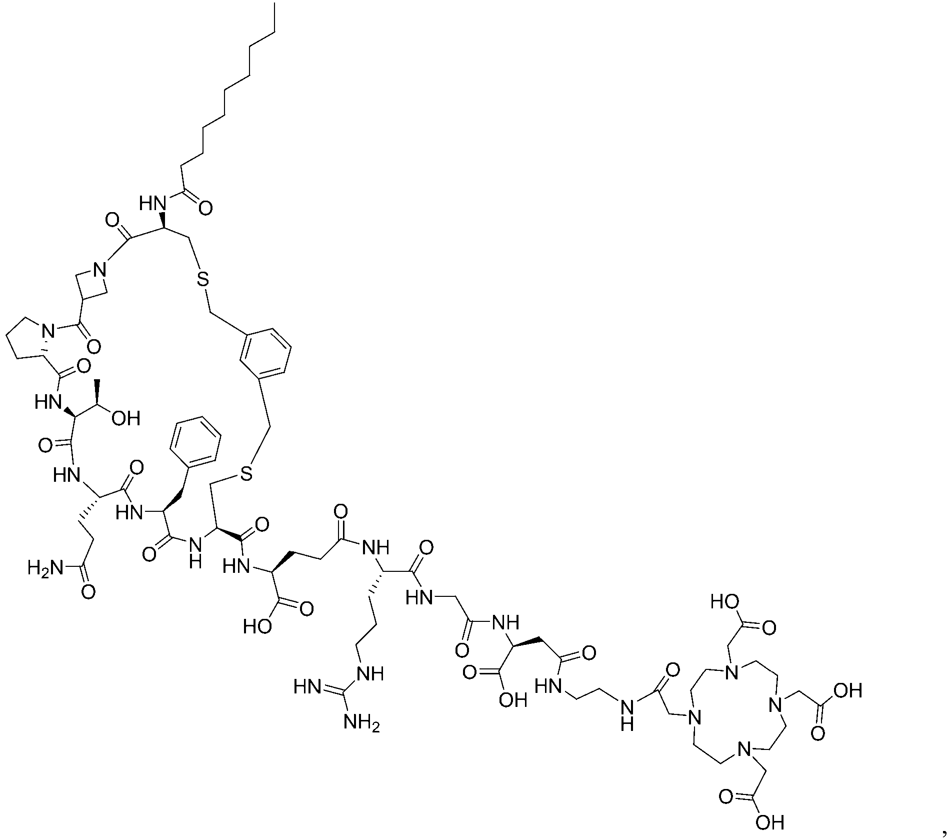

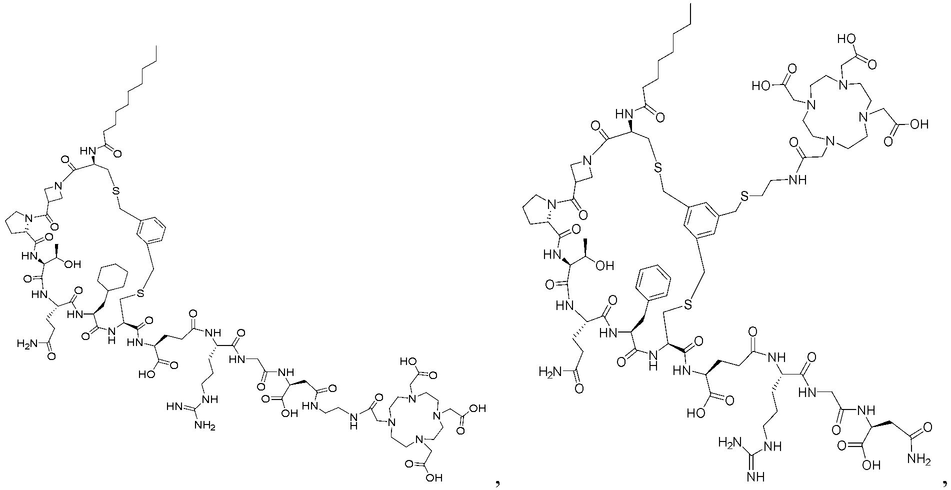

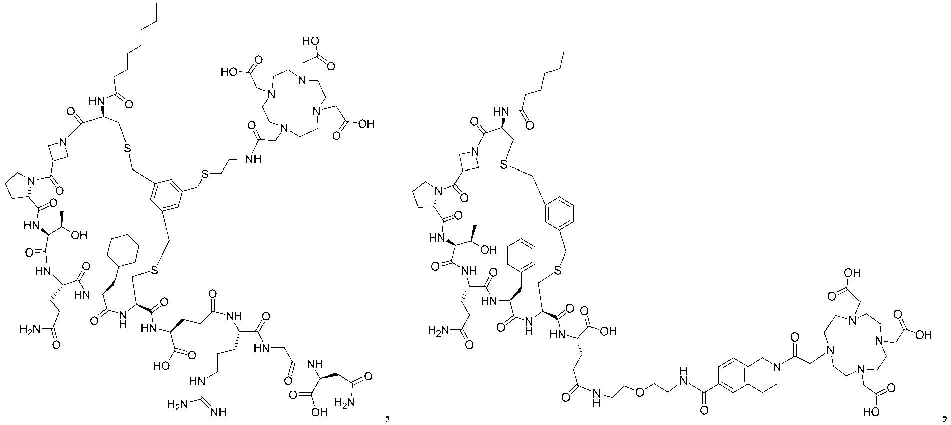

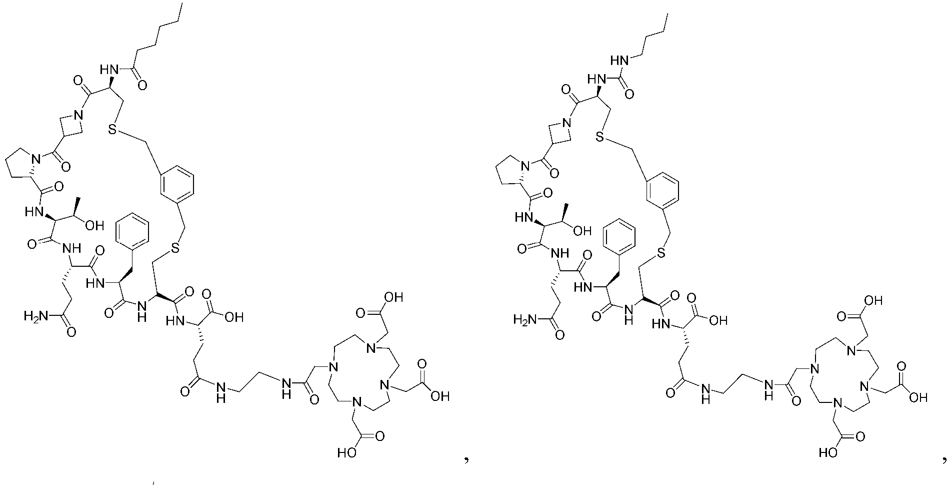

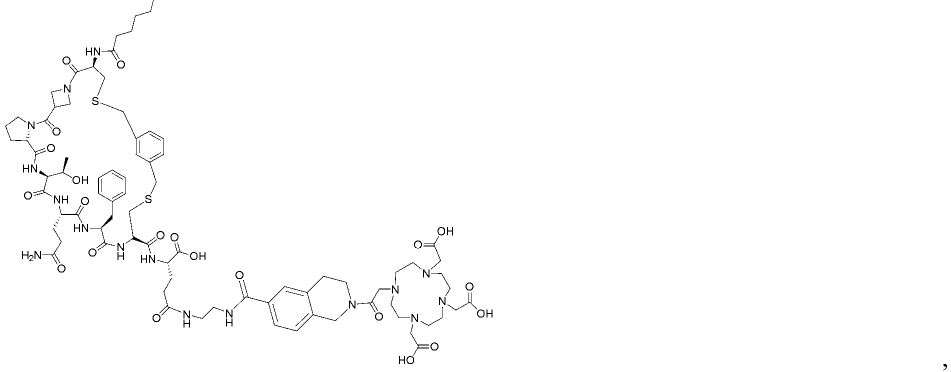

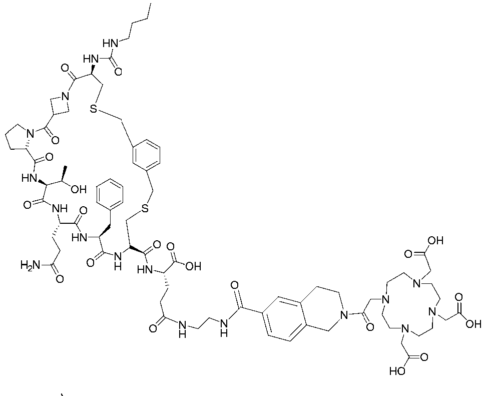

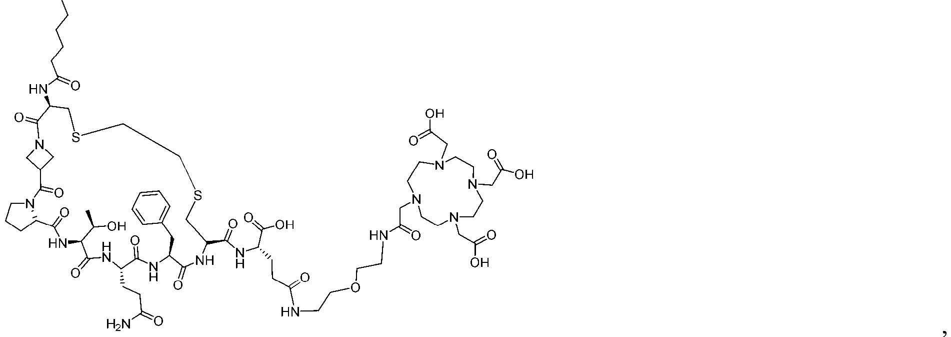

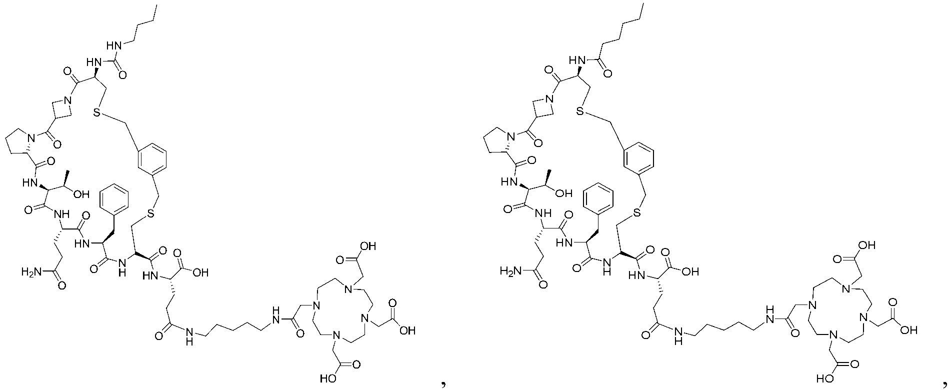

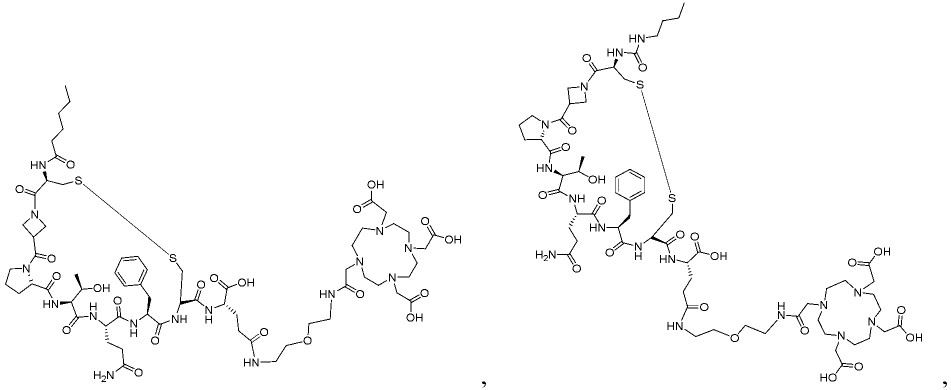

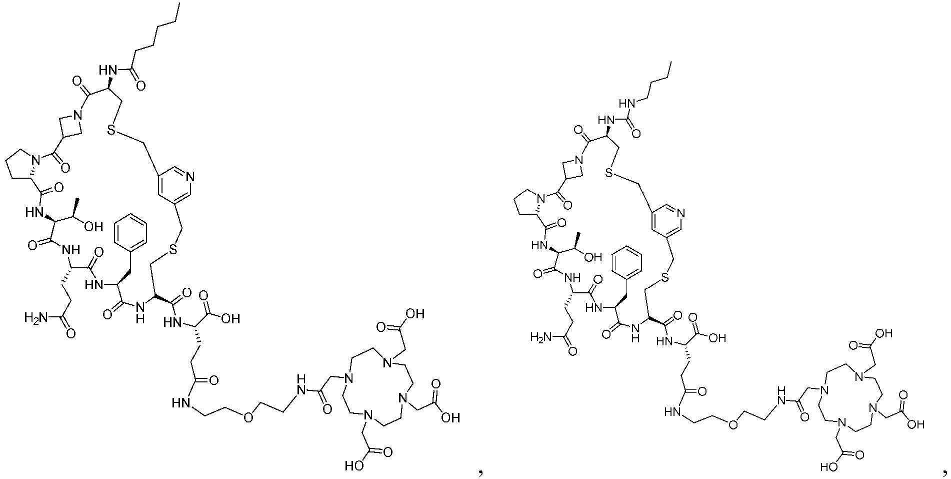

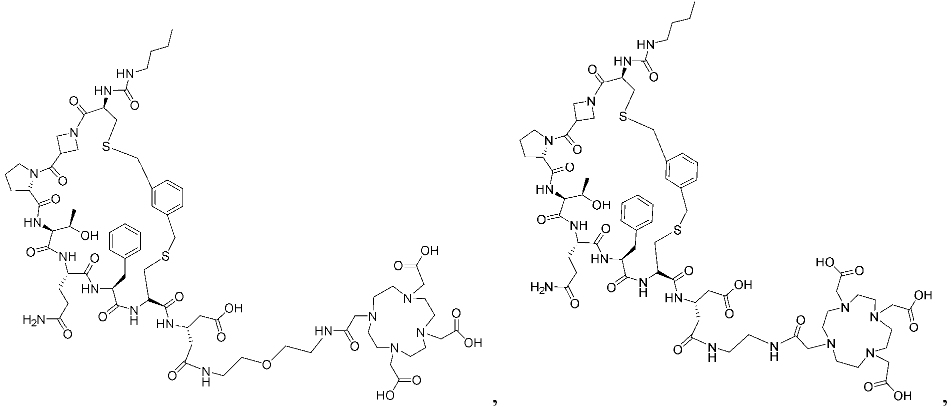

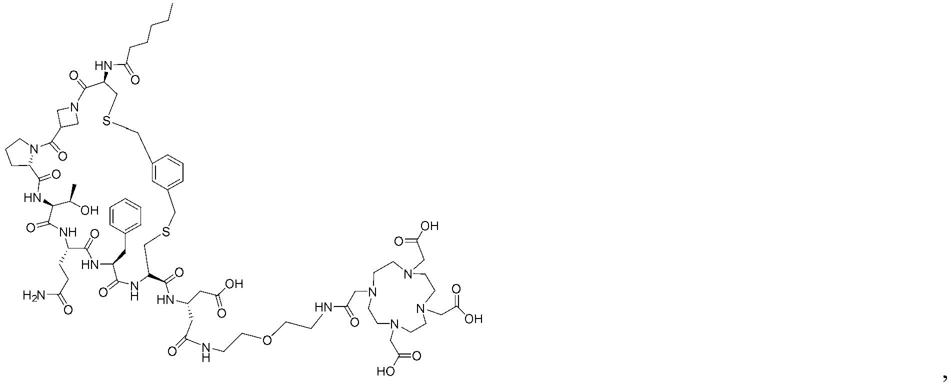

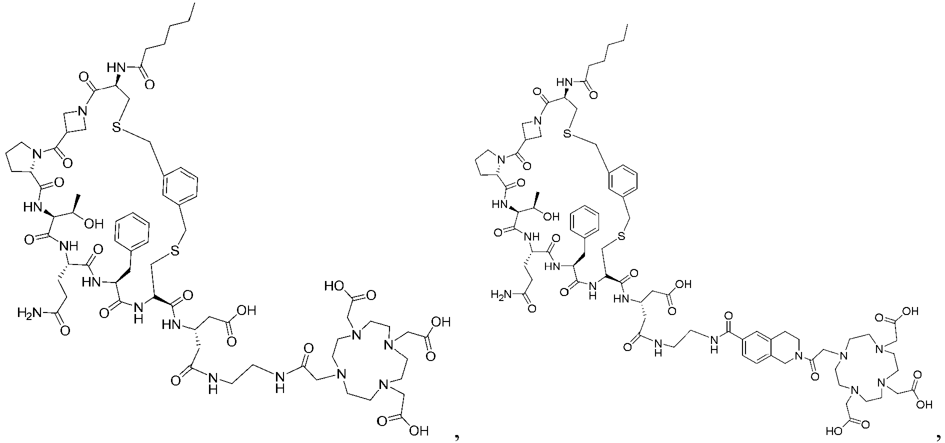

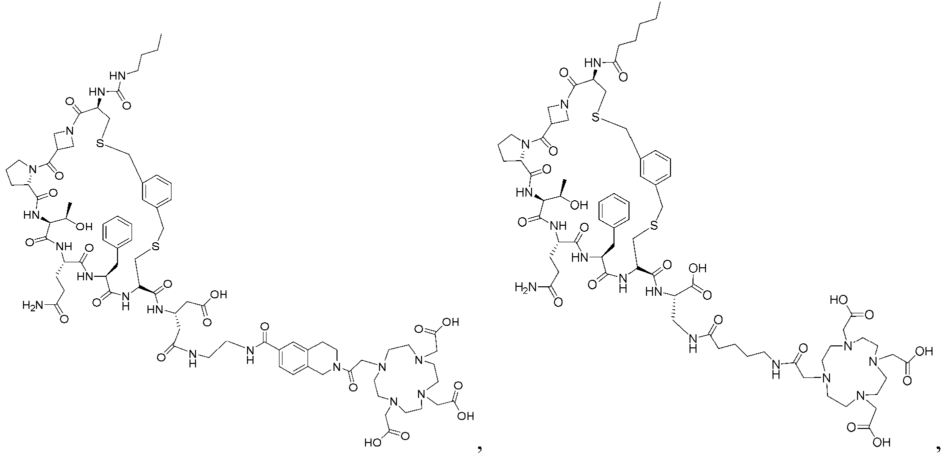

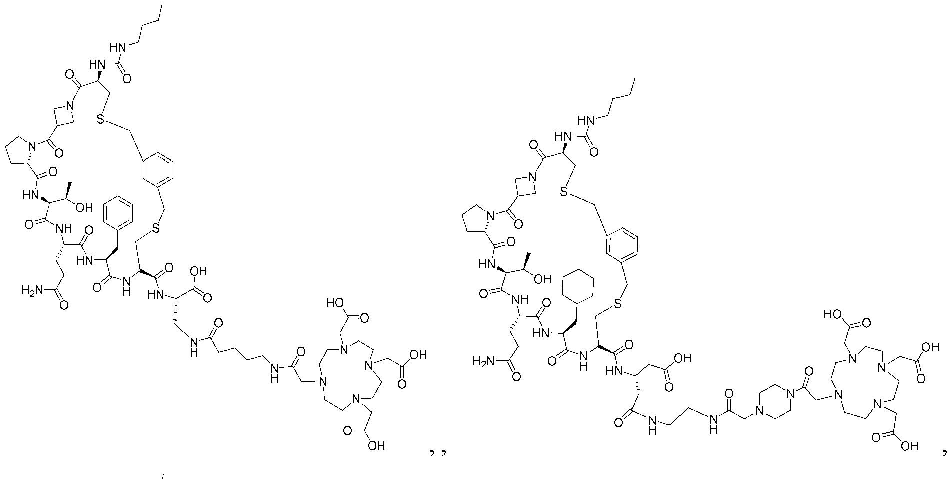

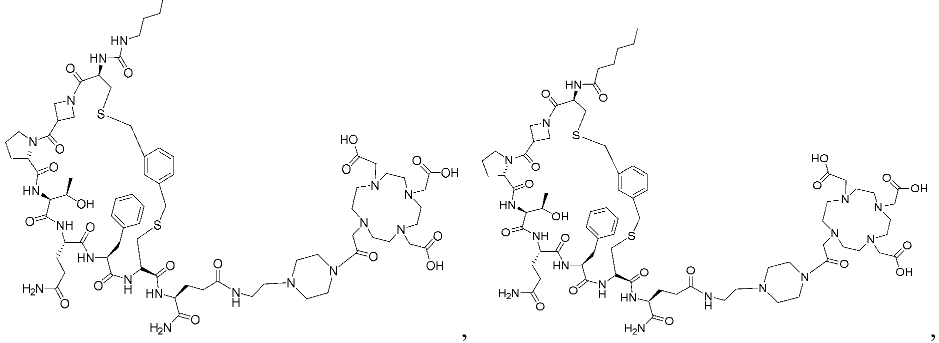

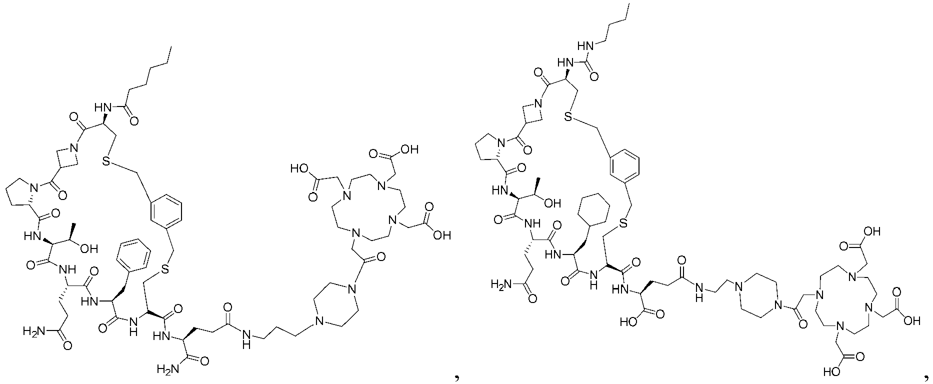

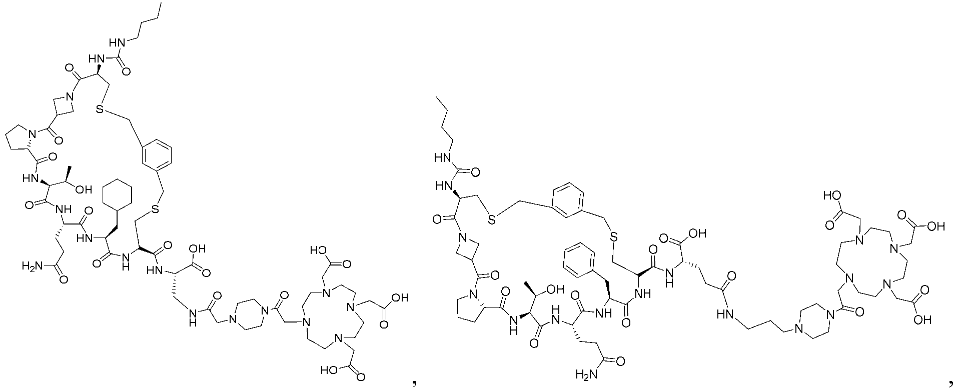

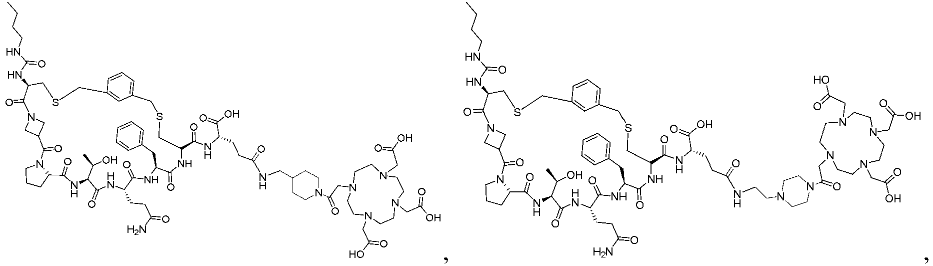

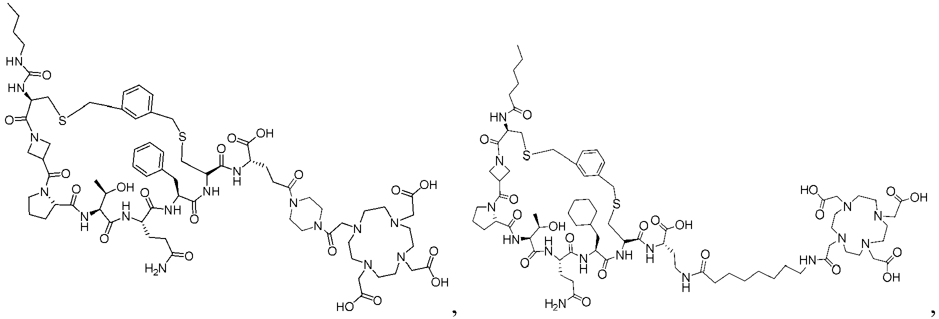

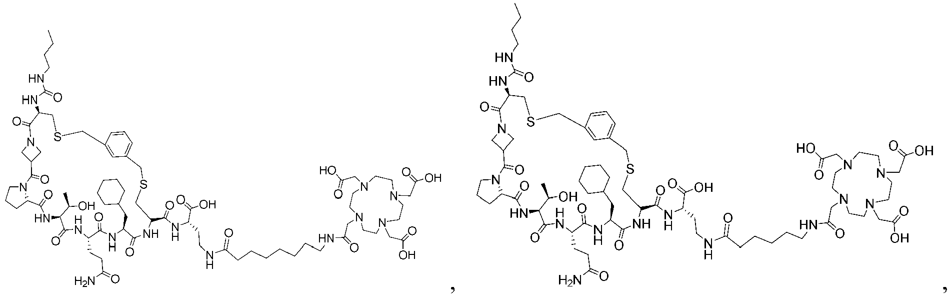

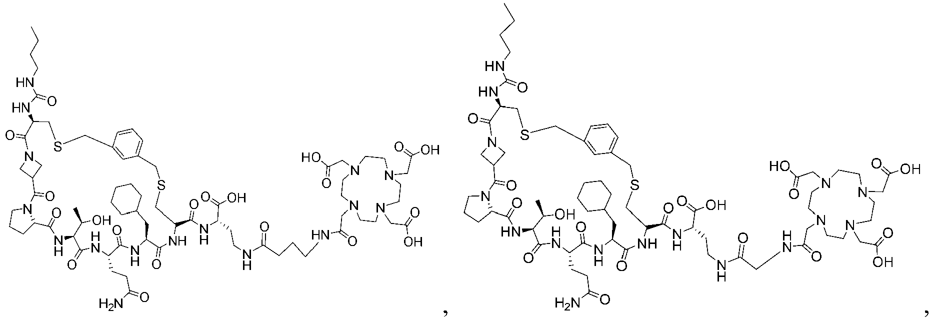

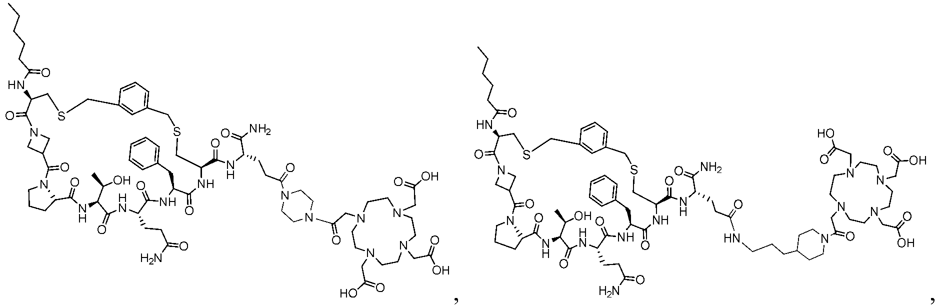

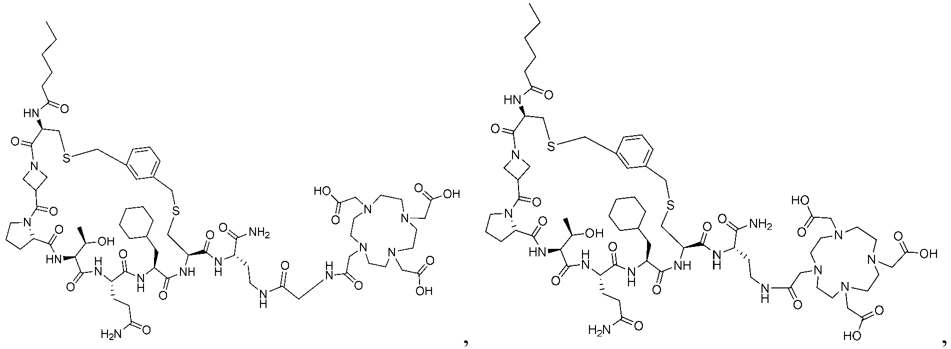

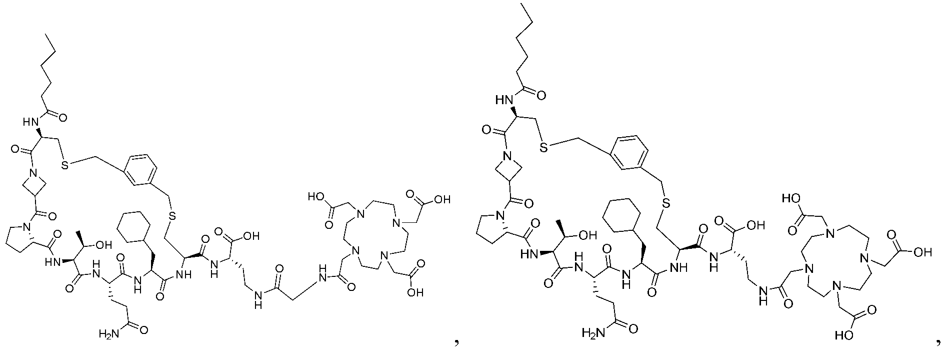

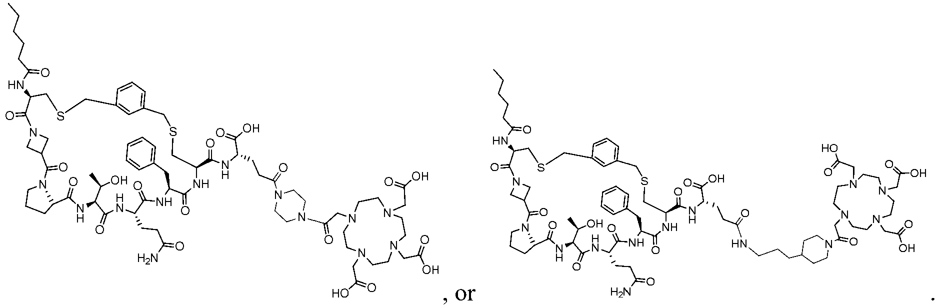

The present disclosure also provides a cyclic polypeptide compound A, wherein the cyclic polypeptide compound A is a compound formed by chelating an ion of a therapeutic radionuclide with the compound X as described above.

In some embodiments, the therapeutic radionuclide is ¹⁷⁷Lu, ⁹⁰Y, ⁸⁹Sr, ¹⁸⁸Re, ²²⁵Ac, ²¹³Bi, or ²¹²Pb.

In some embodiments, the valence state of the ion of the therapeutic radionuclide is monovalent, divalent, trivalent, or tetravalent, for example trivalent.

In some embodiments, the ion of the therapeutic radionuclide is ¹⁷⁷Lu³⁺, ²²⁵Ac³⁺, ⁹⁰Y³⁺, ²¹²Pb²⁺, or ²¹³Bi³⁺, for example ¹⁷⁷Lu³⁺.

In some embodiments, the cyclic polypeptide compound A is a compound formed by chelating ¹⁷⁷Lu³⁺ with a compound X, wherein the structure of the compound X is as described above.

The present disclosure also provides a cyclic polypeptide compound B, which is a compound formed by chelating an ion of a diagnostic radionuclide with a compound X, wherein the structure of the compound X is as described above.

In some embodiments, the diagnostic radionuclide is ¹⁸F, ⁶⁸Ga, ¹¹¹In, or ⁶⁴Cu.

In some embodiments, the valence state of the ion of the diagnostic radionuclide is monovalent, divalent, trivalent, or tetravalent, for example trivalent.

In some embodiments, the ion of a diagnostic radioactive metal is ⁶⁸Ga³⁺ or ⁶⁴Cu²⁺.

In some embodiments, the cyclic polypeptide compound B is a compound formed by chelating ⁶⁸Ga³⁺ with a compound X, wherein the structure of the compound X is as described above.

The present disclosure also provides a cyclic polypeptide compound C, wherein the cyclic polypeptide compound C is selected from any one of the aforementioned compounds X.

The present disclosure also provides a pharmaceutical composition, comprising a substance Y and a pharmaceutically acceptable excipient, wherein the substance Y is the aforementioned cyclic peptide compound A or the aforementioned cyclic peptide compound B.

The present disclosure also provides a use of the aforementioned cyclic polypeptide compound A in the preparation of a medicament for treating tumors. The tumor may be a FAP-associated tumor, for example, breast cancer, ovarian cancer, lung cancer, colorectal cancer, gastric cancer, pancreatic cancer, prostate cancer, liver cancer, or cutaneous melanoma. Preferably, the tumor may be a solid tumor with positive FAP expression, for example, breast cancer, ovarian cancer, lung cancer, colorectal cancer, gastric cancer, pancreatic cancer, prostate cancer, liver cancer, or cutaneous melanoma.

The present disclosure also provides a use of the aforementioned cyclic polypeptide compound B in the preparation of a medicament for diagnosing tumors. The tumor may be a FAP-associated tumor, for example, breast cancer, ovarian cancer, lung cancer, colorectal cancer, gastric cancer, pancreatic cancer, prostate cancer, liver cancer, or cutaneous melanoma. Preferably, the tumor may be a solid tumor with positive FAP expression, for example, breast cancer, ovarian cancer, lung cancer, colorectal cancer, gastric cancer, pancreatic cancer, prostate cancer, liver cancer, or cutaneous melanoma.

Unless otherwise specified, the terms used in the present disclosure have the following meanings:

The term "pharmaceutically acceptable salt" refers to a salt formed by the reaction of the compound with a pharmaceutically acceptable (relatively non-toxic, safe, and suitable for patient use) acid or base. When the compound contains a relatively acidic functional group, a base addition salt can be obtained by contacting the free form of the compound with a sufficient amount of a pharmaceutically acceptable base in a suitable inert solvent. Pharmaceutically acceptable base addition salts include, but are not limited to, sodium salts, potassium salts, calcium salts, aluminum salts, magnesium salts, bismuth salts, ammonium salts, *etc.* When the compound contains a relatively basic functional group, an acid addition salt may be obtained by contacting the free form of the compound with a sufficient amount of a pharmaceutically acceptable acid in a suitable inert solvent. Pharmaceutically acceptable acid addition salts include, but are not limited to, hydrochloride, sulfate, mesylate, acetate, trifluoroacetate, *etc.* For details, please refer to the Handbook of Pharmaceutical Salts: Properties, Selection, and Use (P. Heinrich Stahl, 2002).

The terms ' 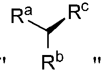 and ' 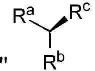 " both denote

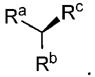

The term "alkyl" refers to a linear or branched alkyl group having a specified number of carbon atoms (e.g., C₁-C₃₀, C₁-C₂₀, or C₁-C₆). Alkyl includes, but is not limited to, methyl, ethyl, n-propyl, isopropyl, n-butyl, tert-butyl, isobutyl, sec-butyl, *etc.*

The term "alkylene" refers to a divalent group connected to the rest of the molecule via two single bonds, with all other definitions being the same as the term "alkyl."

The term "cycloalkyl" refers to a saturated cyclic group consisting solely of carbon atoms with a specified number of carbon atoms (e.g., C₃-C₈ or C₃-C₆), which is monocyclic, bridged, or spirocyclic. Cycloalkyl includes, but is not limited to, cyclopropyl, cyclobutyl, cyclopentyl, cyclohexyl, *etc.*

The term "cycloalkylene" refers to a divalent group connected to the rest of the molecule via two single bonds, with all other definitions being the same as the term "cycloalkyl."

The term "heterocycloalkyl" refers to a cyclic group having a specified number of ring atoms (e.g., 3- to 10-membered or 3- to 6-membered), a specified number of heteroatoms (e.g., 1, 2, or 3), and a specified type of heteroatom (one or more of N, O, and S), which is monocyclic, bridged, or spirocyclic, and each ring is saturated. Heterocycloalkyl includes, but is not limited to, azetidinyl, tetrahydropyrrolyl, tetrahydrofuranyl, morpholinyl, piperidinyl, pyrrolidinyl, piperidinyl, *etc.*

The term "heterocycloalkylene" refers to a divalent group connected to the rest of the molecule via two single bonds, with all other definitions being the same as the term "heterocycloalkyl."

The term "aryl" refers to a cyclic group consisting solely of carbon atoms with a specified number of carbon atoms (e.g., C₆-C₁₀), which is monocyclic or fused-ring, and at least one ring is aromatic (conforming to Hückel's rule). The aryl group is connected to other fragments in the molecule via an aromatic ring or a non-aromatic ring. The aryl group includes, but is not limited to, phenyl, naphthyl, *etc.*

The term "arylene" refers to a divalent group connected to the rest of the molecule via two single bonds, with all other definitions being the same as the term "aryl."

The term "heteroaryl" refers to a cyclic group having a specified number of ring atoms (e.g., 6- to 12-membered), a specified number of heteroatoms (e.g., 1, 2, or 3), and a specified type of heteroatom (one or more of N, O, and S), which is monocyclic or fused-ring, and at least one ring is aromatic (conforming to Hückel's rule). The heteroaryl group is connected to other fragments in the molecule via an aromatic ring or a non-aromatic ring of a fused-ring system. Heteroaryl includes, but is not limited to, pyridyl, pyrimidinyl, indolyl, 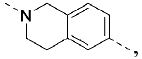 *etc.*

The term "heteroarylene" refers to a divalent group connected to the rest of the molecule via two single bonds, with all other definitions being the same as the term "heteroaryl."

The" 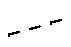 " in the structural fragment indicates that the structural fragment is connected to other fragments in the molecule via this site. For example, 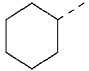 refers to cyclohexyl.

The term "pharmaceutical excipient" refers to the excipients and additives used in the production of pharmaceuticals and the compounding of prescriptions, encompassing all substances included in a drug formulation other than the active ingredients. For details, please refer to the Pharmacopoeia of the People's Republic of China (2020 Edition) or the Handbook of Pharmaceutical Excipients (Raymond C Rowe, 2009).

The term "therapeutically effective amount" refers to an amount of a compound administered to a patient that is sufficient to effectively treat the disease, as well as a radiation dose. The therapeutically effective amount will vary depending on the compound, type of disease, severity of the disease, age of the patient, and other factors, but can be adjusted by a person skilled in the art as appropriate.

The term "patient" refers to any animal that has received or is about to receive treatment, preferably a mammal, and most preferably a human. Mammals include, but are not limited to, cattle, horses, sheep, pigs, cats, dogs, mice, rats, rabbits, guinea pigs, monkeys, humans, *etc.*

The term "treatment" refers to any of the following circumstances: (1) alleviating one or more biological manifestations of a disease; (2) interfering with one or more points in the biological cascade that triggers the disease; (3) slowing the progression of one or more biological manifestations of the disease.

Without departing from common knowledge in the art, the above preferred conditions may be combined in any manner to obtain various preferred embodiments of the present disclosure.

All reagents and raw materials used in the present disclosure are commercially available.

The positive and progressive effects of the present disclosure lie in that: the present disclosure provides a class of cyclic polypeptide compounds with high affinity for FAP. These compounds can be used for the diagnosis and treatment of FAP-related tumors, exhibit high tumor uptake, long retention time, and possess broad application prospects.

### BRIEF DESCRIPTION OF THE DRAWINGS

FIG. 1 shows the biodistribution of ¹⁷⁷Lu-labeled compounds in tumor-bearing mice.
FIG. 2 shows PET-CT images of ⁶⁸Ga-FAP-2286 in tumor-bearing mice.
FIG. 3 shows PET-CT images of ⁶⁸Ga-JHDC16 in tumor-bearing mice.
FIG. 4 shows PET-CT images of ⁶⁸Ga-JHDC06 in tumor-bearing mice.
FIG. 5 shows the inhibition of tumor growth by ¹⁷⁷Lu-labeled compounds in a tumor-bearing mouse model.
FIG. 6 shows the effect of the ¹⁷⁷Lu-labeled compound on body weight in a tumor-bearing mouse model.

### DETAILED DESCRIPTION OF THE PREFERRED EMBODIMENT

The present disclosure is further illustrated below by way of examples, but is not thereby limited to the scope of the examples described. For experimental methods without specified conditions in the following examples, selection shall be made in accordance with conventional methods and conditions or according to the product instructions.

### Explanation of Structural Abbreviations:

Abbreviations for natural amino acids:

| Abbreviation | Natural amino acid |
|---|---|
| Ala | Alanine |
| Arg | Arginine |
| Asn | Asparagine |
| Asp | Aspartic acid |
| Cys | Cysteine |
| Gln | Glutamine |
| Glu | Glutamic acid |
| Gly | Glycine |
| His | Histidine |
| Ile | Isoleucine |
| Leu | Leucine |
| Lys | Lysine |
| Met | Methionine |
| Phe | Phenylalanine |
| Pro | Proline |
| Ser | Serine |
| Thr | Threonine |
| Trp | Tryptophan |
| Tyr | Tyrosine |
| Val | Valine |

Abbreviations for non-natural amino acids:

| **Abbreviation** | **Structure** |
|---|---|
| hhy02 | 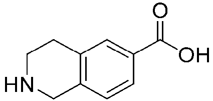 |
| hhy06 | 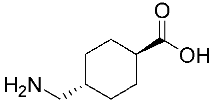 |
| hhy24 | 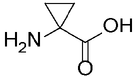 |
| hhy25 | 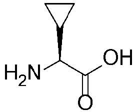 |
| hhy26 | 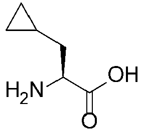 |
| hhy33 | 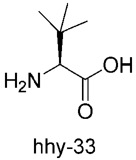 |
| hhy34 | 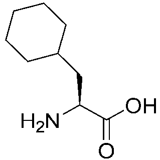 |
| hhy35 | 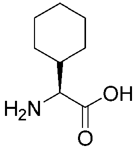 |
| hhy36 | 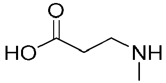 |
| hhy37 | 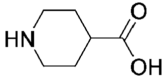 |
| hhy38 | 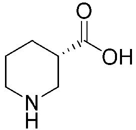 |
| hhy39 | 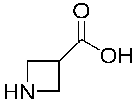 |
| hhy40 | 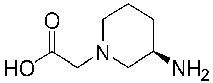 |
| hhy41 | 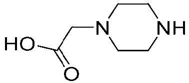 |
| hhy42 | 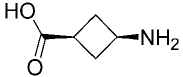 |
| hhy50 | 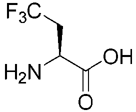 |
| PFPhe | 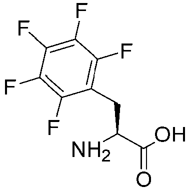 |
| hhy52 | 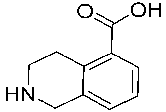 |
| Nal | 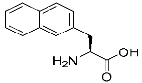 |
| 6AHA | 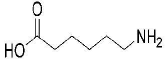 |
| APTA | 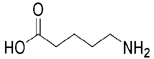 |
| ABA | 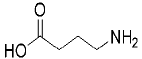 |
| DAPA | 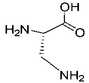 |
| AOA | 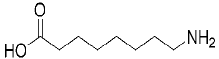 |
| DABA | 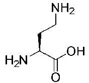 |

Abbreviations for intermediates

| **Abbreviation** | **Structure** |
|---|---|
| TAT | 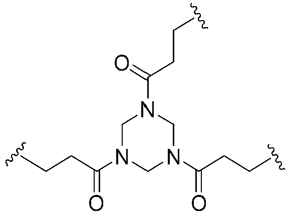 |
| hhy48 | 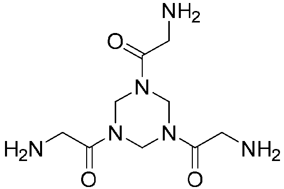 |
| hhy49 | 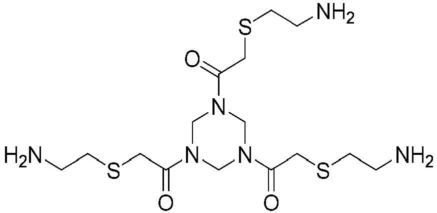 |
| 3MeBn | 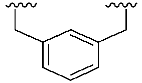 |
| tMeBn | 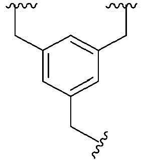 |
| 3MePy | 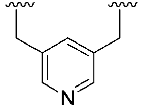 |
| AET | 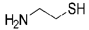 |
| Hex | 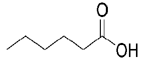 |
| Hep | 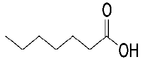 |
| Oct | 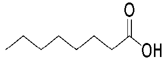 |
| Non | 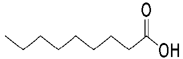 |
| Dec | 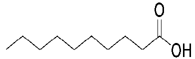 |
| DAPEG2 | 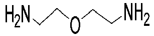 |
| AEAA | 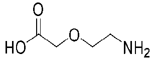 |
| 2AHA | 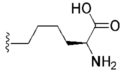 |
| CSMC | 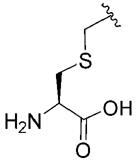 |
| triazole | 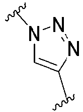 |
| CALA | 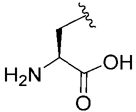 |
| DFCP | 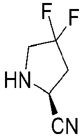 |
| AEEAA | 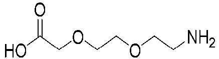 |
| DAPEG4 | 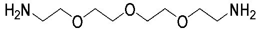 |
| EDA | 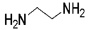 |
| ATTDA | 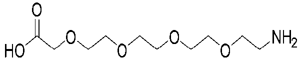 |
| PDA | 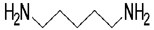 |
| APA | 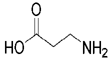 |
| AHLA | 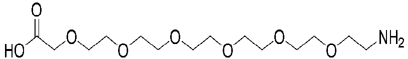 |
| SA | 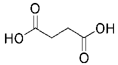 |
| hhy43 | 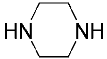 |
| hhy44 | 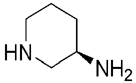 |
| hhy45 | 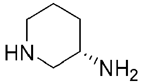 |
| hhy46 | 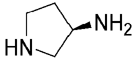 |
| hhy47 | 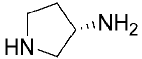 |

Abbreviations for chelating groups:

| **Abbreviation** | **Structure** |
|---|---|
| NOTA | 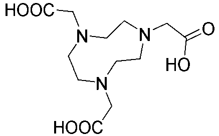 |
| HBED-CC | 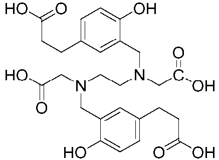 |
| NODAGA | 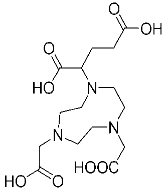 |
| NOTAGA | 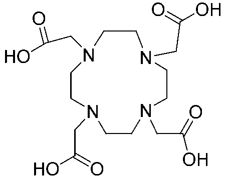 |
| DOTAGA | 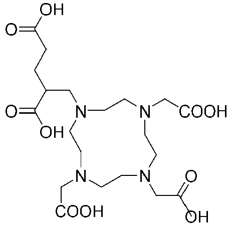 |
| TRAP | 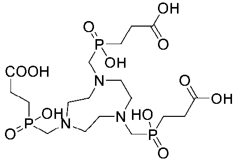 |
| NOPO | 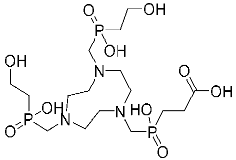 |
| PCTA | 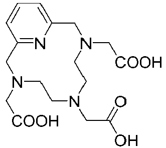 |
| DFO | 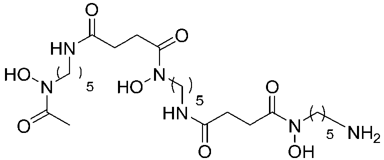 |
| DTPA | 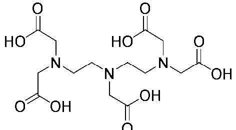 |

The above chelating group is connected to L via a carbonyl group (CO) through amidation reaction, for example, via an amide linkage.

The amino acid backbone sequences of the compounds in the examples are as follows:

| Compounds involved | Sequence | SEQ ID NO: | Note |
|---|---|---|---|
| JHDC01/JHDC02 | CX₁PATEX₂CK | 1 | X₁ is hhy42 or hhy39, X₂ is Nal |
| JHDC03 | CX₁PGRGX₂CK | 2 | X₁ is hhy39, X₂ is Nal |
| JHDC04 | CX₁PDAQGFCK | 3 | X₁ is hhy39 |
| JHDC05/JHDC06/JHDC16/JHDC22-39 (34-39 are β-Glu)/JHDC42-46 (42 is β-Glu)/JHDC48-51/JHDC57-58/JHDC63-64 | CX₁PTQX₂CE | 4 | X₁ is hhy39, X₂ is hhy34 or F |
| JHDC07 | PTQFCK | 5 | |
| JHDC08 | CX₁PTQFCK | 6 | X₁ is hhy40 |
| JHDC09 | CX₁PTQGX₂CE | 7 | X₁ is hhy39, X₂ is hhy34 |
| JHDC10 | CX₁PTQFC | 8 | X₁ is hhy39 |
| JHDC11/JHDC12 | PTQF | 9 | |
| JHDC13/JHDC14 | PTQX₁CK | 10 | X₁ is hhy34 |
| JHDC15 | PTQX₁GCK | 11 | X₁ is hhy34 |
| JHDC17 | CX₁PTQX₂GCE | 12 | X₁ is hhy39, X₂ is hhy34 |
| JHDC18/JHDC19/JHDC20/JHDC21 | CX₁PTQX₂CERGD | 13 | X₁ is hhy39, X₂ is hhy34 or F |
| JHDC40/JHDC41/JHDC47/JHDC52-56/JHDC59-62 | CX₁PTQX₂C | 14 | X₁ is hhy39, X₂ is hhy34 or F |

### Example 1 Synthesis of Compounds

### General synthesis method 1

Taking compound JHDC23 as an example: it is prepared by solid-phase synthesis through the following steps:

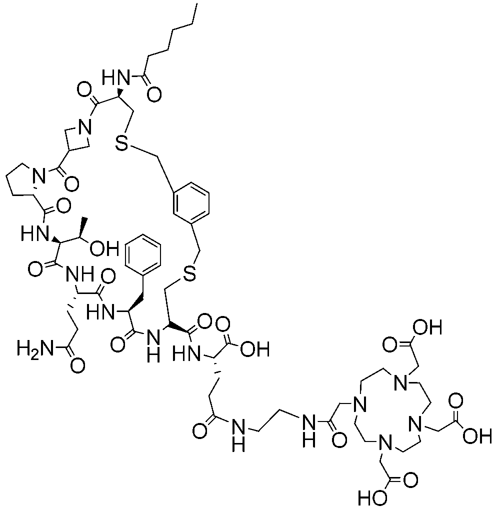

Hex[Cys(3MeBn)-hhy39-Pro-Thr-Gln-Phe-Cys]-Glu-(EDA-DOTA)-OH was synthesized using the following steps.
1. CTC resin (SUNRESIN, 1 g; 0.65 mmol) was weighed and added to the synthesis tube. After swelling and capping, Fmoc-Glu(OAll)-OH, Fmoc-Cys(Trt)-OH, Fmoc-Phe-OH, Fmoc-Gln(Trt)-OH, Fmoc-Thr(tBu)-OH, Fmoc-Pro-OH, hhy39, Fmoc-Cys(Trt)-OH, and Hex were sequentially coupled. Then the Alloc protecting group was removed, and subsequently Fmoc-ethylenediamine and DOTA-(OtBu)₃ were coupled. Condensation was performed using the PyBOP/DIEA system, and Fmoc deprotection was carried out with 20% piperidine/DMF. The Fmoc strategy was used to complete solid-phase synthesis. Cleavage was performed using a solution of TFA:DTT:Tis:H₂O (90:5:2.5:2.5) for 3-6 hours. The cleavage solution was added dropwise to 10 volumes of isopropyl ether, precipitating a solid. The solid was washed three times with isopropyl ether, dried under reduced pressure, yielding 550 mg of crude peptide (JHD00130-A1) [M/2+H]⁺=719.20, which was directly used in the next reaction.
2. JHD00130-A1 (550 mg; 382 mmol) and 1,3-bis(bromomethyl)benzene (131.4 mg; 497.7 mmol) were dissolved in H₂O (250 mL) and acetonitrile (250 mL). Its pH was adjusted to 8 by addition of an NH₄HCO₃(aq.) solution. The mixture was stirred, and the reaction was monitored by LCMS until completion. After lyophilization and pre-HPLC purification, 148 mg of crude product and 16.56 mg of pure product [M+H]⁺=1540.45 (JHDC23) were obtained.

### General preparative liquid chromatography method:

Chromatography column: Bonnasil-BS C18 (21.2 x 250 mm, 5 µm)
Mobile phase: H₂O (0.01% TFA) (A) / acetonitrile (0.01% TFA) (B)
Flow rate: 10 mL/min
Detection wavelength: 220 nm
Elution program: 15-45% B, 0-60 min.
Analytical method 1:
Chromatographic column: Shim-pack VP-ODS (4.6 × 150 mm, 5 µm)
Mobile phase: H₂O (0.01% TFA) (A) / acetonitrile (0.01% TFA) (B)
Flow rate: 1.0 mL/min
Elution program: A gradient from 10% to 40% of B over 20 minutes at 1.0 mL/min, followed by a gradient from 40% to 70% of B from 20 to 24 min, a gradient from 70% to 10% of B from 24 to 24.1 min, and 10% of B maintained from 24.1 to 30 min.
Column temperature: 30°C
Detection: UV (214, 4 nm).
Analytical method 2:
Chromatographic column: XBridge Peptide BEH C18 (4.6 × 150 mm, 3.5 µm)
Mobile phase: A: 0.05% TFA in water, B: 0.05% TFA in ACN
Elution program: 5% B for 1 min, 5-65% B within 20 min
Flow rate: 1.0 mL/min
Column temperature: 40°C
Detection wavelength: 220 nm.
Analytical method 3:
Chromatographic column: Phenomenex Luna 3u C18(2), (4.6*150mm*3µm)
Mobile phase: A: 0.1% TFA in 100% water, B: 0.1% TFA in 100% acetonitrile
Flow rate: 0.8 mL/min
Detection wavelength: 220 nm
Elution program: 15% B, 0.01 min; 15-60% B, 0.01-25.0 min; 60-90% B, 25-30 min.
Analytical method 4:
Chromatographic column: SHIMADZU Inertsil ODS-SP (4.6*250mm*5µm)
Mobile phase: A: 0.1% TFA in 100% water, B: 0.1% TFA in 100% acetonrtrile
Flow rate: 1 mL/min
Detection wavelength: 220 nm
Elution program: B: 20%, 0 min; B: 20-80%, 0-25 min; B: 100%, 25.01-30 min.
Analytical method 5:
Chromatographic column: SepaxGP-C18 (4.6*150mm*5µm)
Mobile phase A: 0.1% TFA in 100% water, B: 0.09% TFA in (80% acetonitrile/20% water)
Flow rate: 1 mL/min
Detection wavelength: 220 nm
Elution program: B: 39-49%, 0-20 min.
Analytical method 6:
Chromatographic column: SHIMADZU Inertsil ODS-SP (4.6*250mm*5µm)
Mobile phase: A: 0.1% TFA in 100% water, B: 0.1% TFA in 100% acetonrtrile
Flow rate: 1 mL/min
Detection wavelength: 220 nm
Elution program: B: 25% at 0 min; B: 25-65% from 0 to 20 min; B: 100% from 20.01 to 23 min; B: 100% from 23 to 38 min; B: 100-25% from 38 to 40 min.

The following compounds were prepared using the above general synthetic method 1. 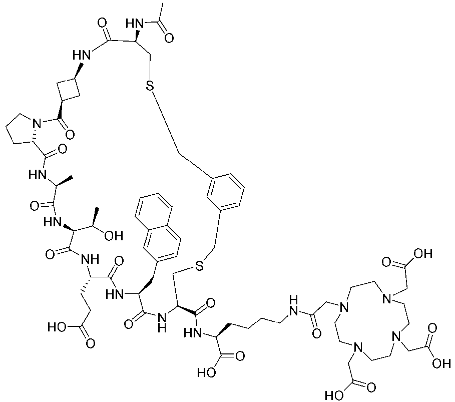
JHDC01 Ac[Cys(3MeBn)-hhy42-Pro-Ala-Thr-Glu-Nal-Cys]-Lys-(DOTA)-OH, synthesis method 1, analytical method 4 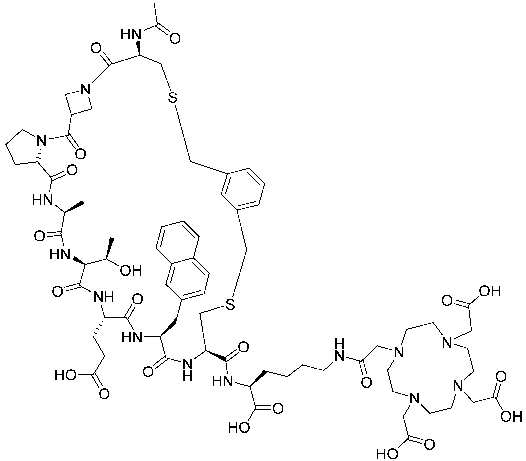
JHDC02 Ac[Cys(3MeBn)-hhy39-Pro-Ala-Thr-Glu-Nal-Cys]-Lys-(DOTA)-OH, synthesis method 1, analytical method 4 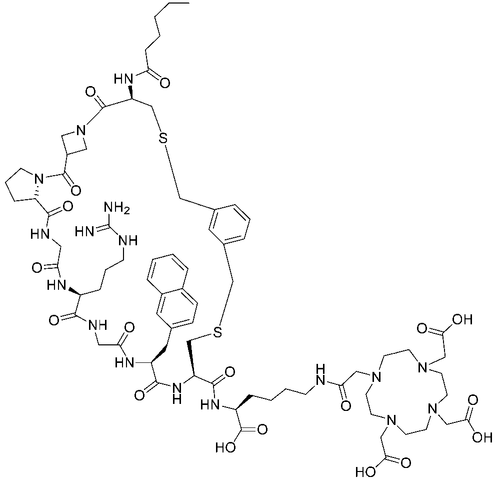
JHDC03 Hex[Cys(3MeBn)-hhy39-Pro-Gly-Arg-Gly-Nal-Cys]-Lys-(DOTA)-OH, synthesis method 1, analytical method 5 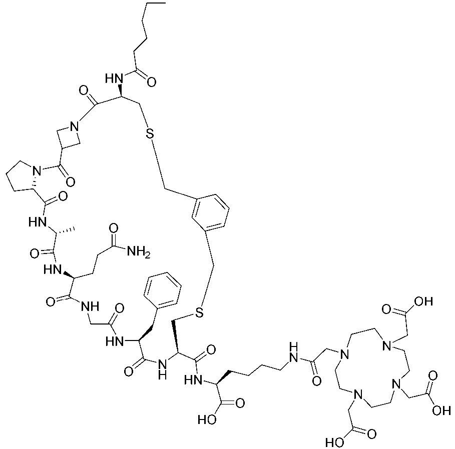
JHDC04 Hex[Cys(3MeBn)-hhy39-Pro-D-Ala-Gln-Gly-Phe-Cys]-Lys-(DOTA)-OH, synthesis method 1, analytical method 5 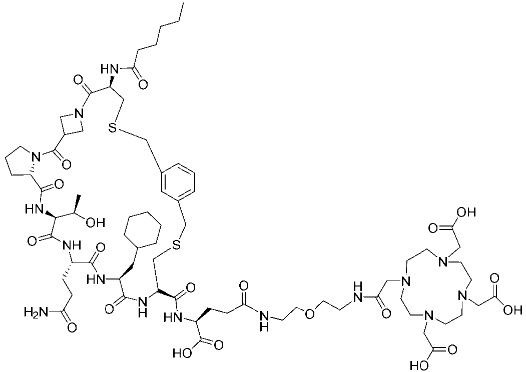
JHDC05 Hex[Cys(3MeBn)-hhy39-Pro-Thr-Gln-hhy34-Cys]-Glu-(DAPEG2-DOTA)-OH, synthesis method 1, analytical method 2 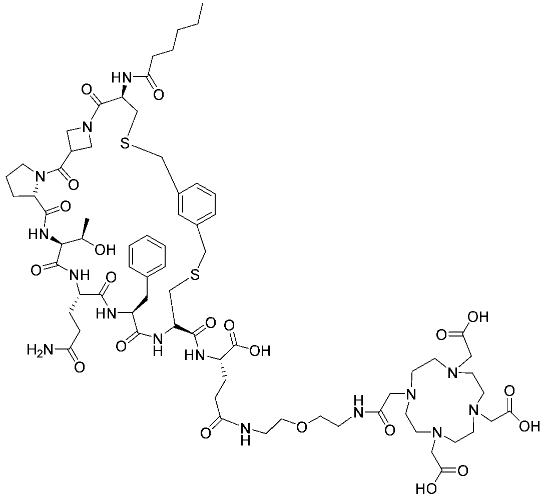
JHDC06 Hex[Cys(3MeBn)-hhy39-Pro-Thr-Gln-Phe-Cys]-Glu-(DAPEG2-DOTA)-OH, synthesis method 1, analytical method 2 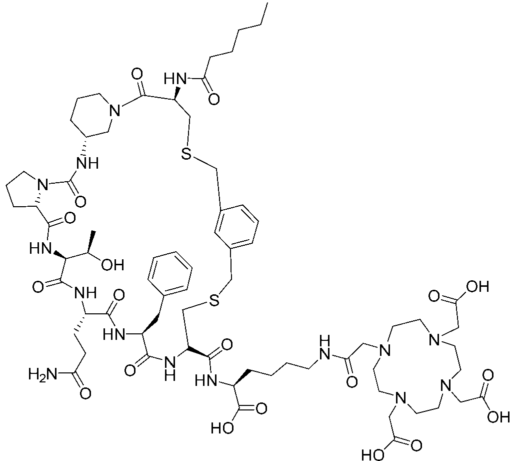
JHDC07 Hex[Cys(3MeBn)-hhy44(NH)-CO-Pro-Thr-Gln-Phe-Cys]-Lys-(DOTA)-OH, synthesis method 1, analytical method 2 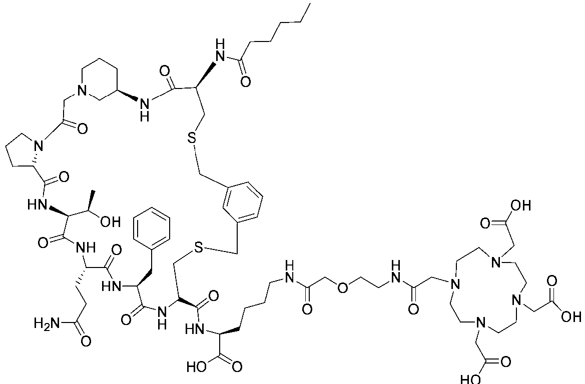
JHDC08 Hex[Cys(3MeBn)-hhy40-Pro-Thr-Gln-Phe-Cys]-Lys-(AEAA-DOTA)-OH, synthesis method 1, analytical method 2 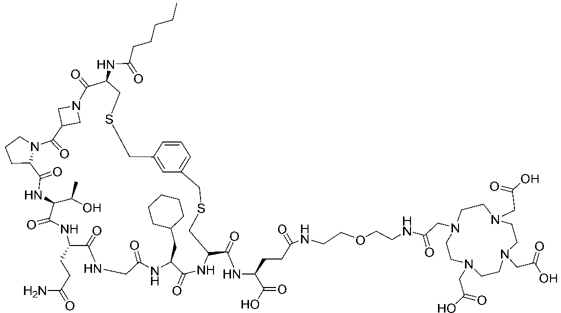
JHDC09 Hex[Cys(3MeBn)-hhy39-Pro-Thr-Gln-Gly-hhy34-Cys]-Glu-(DAPEG2-DOTA)-OH, synthesis method 1, analytical method 2 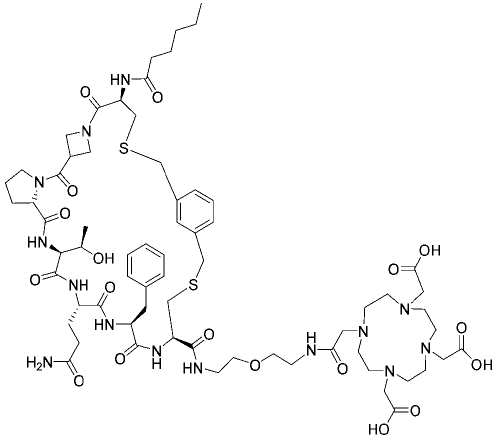
JHDC10 Hex[Cys(3MeBn)-hhy39-Pro-Thr-Gln-Phe-Cys]-DAPEG2-DOTA, synthesis method 1, analytical method 2 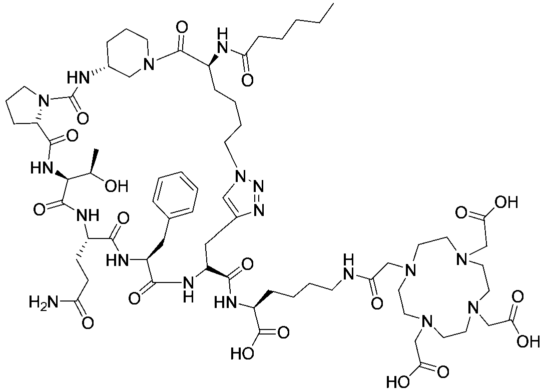
JHDC11 Hex-[2AHA(Triazole)-hhy44(NH)-CO-Pro-Thr-Gln-Phe-CALA]-Lys-(DOTA)-OH, synthesis method 1, analytical method 2 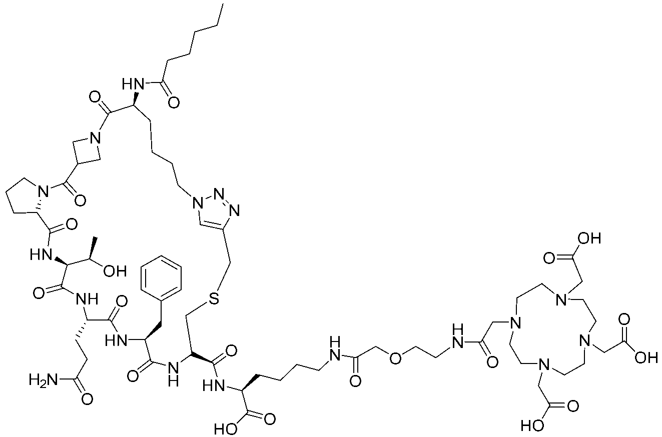
JHDC12 Hex[2AHA(Triazole)-hhy39-Pro-Thr-Gln-Phe-CSMC]-Lys-(AEAA-DOTA)-OH synthesis method 1, analytical method 2 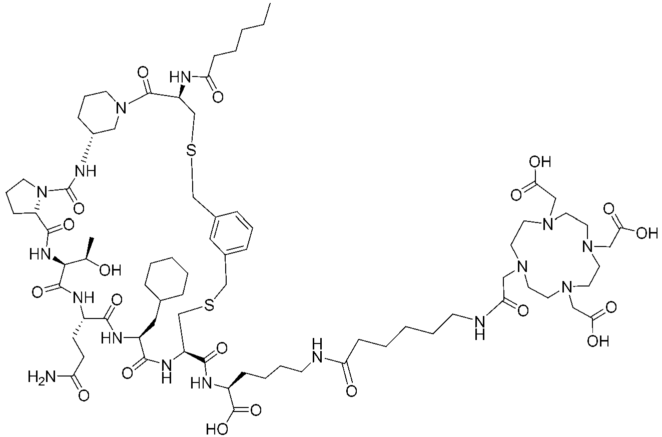
JHDC13 Hex-[Cys(3MeBn)-hhy44-NH-CO-Pro-Thr-Gln-hhy34-Cys]-Lys-(6AHA-DOTA)-OH, synthesis method 1, analytical method 2 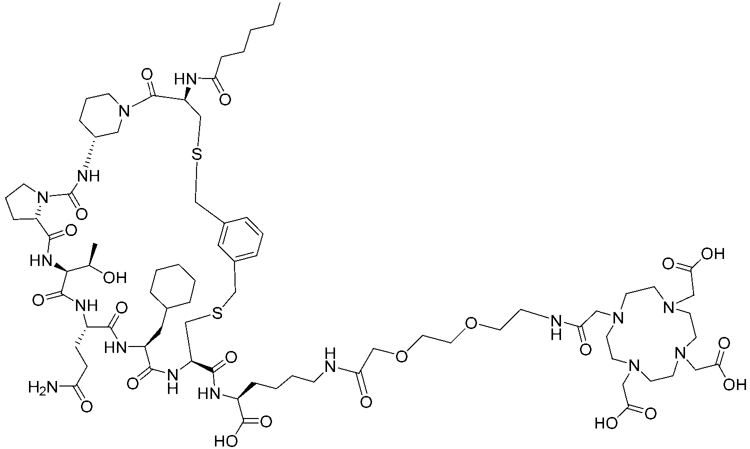
JHDC14 Hex-[Cys(3MeBn)-hhy44-NH-CO-Pro-Thr-Gln-hhy34-Cys]-Lys-(AEEAA-DOTA)-OH, synthesis method 1, analytical method 2 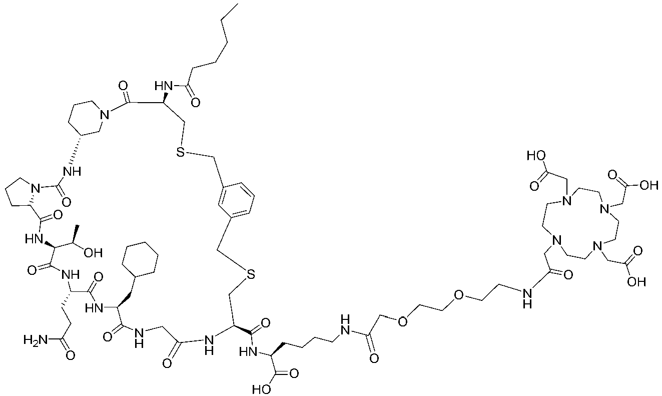
JHDC15 Hex-[Cys(3MeBn)-hhy44-NH-CO-Pro-Thr-Gln-hhy34-Gly-Cys]-Lys-(AEEAA-DOTA)-OH, synthesis method 1, analytical method 2 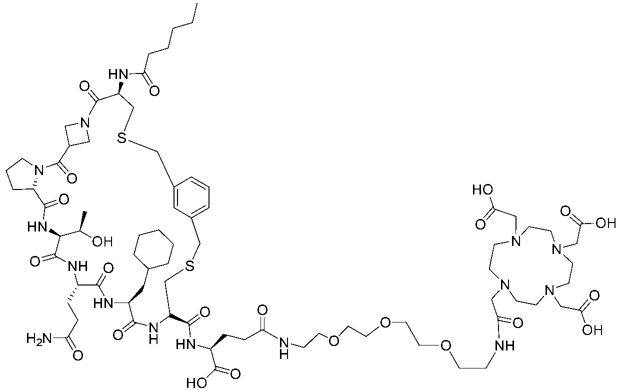
JHDC16 Hex[Cys(3MeBn)-hhy39-Pro-Thr-Gln-hhy34-Cys]-Glu-(DAPEG4-DOTA)-OH, synthesis method 1, analytical method 2 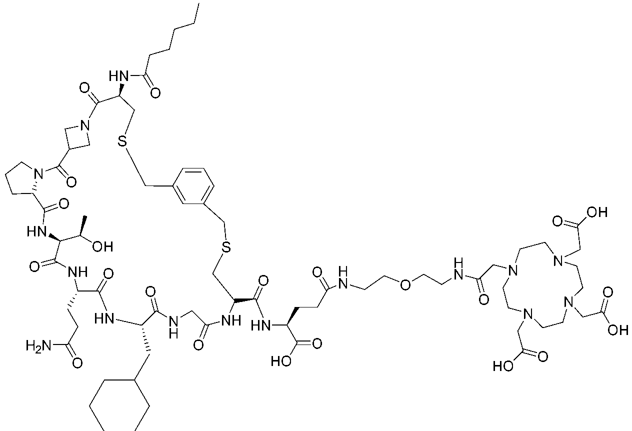
JHDC17 Hex[Cys(3MeBn)-hhy39-Pro-Thr-Gln-hhy34-Gly-Cys]-Glu-(DAPEG2-DOTA)-OH, synthesis method 1, analytical method 2 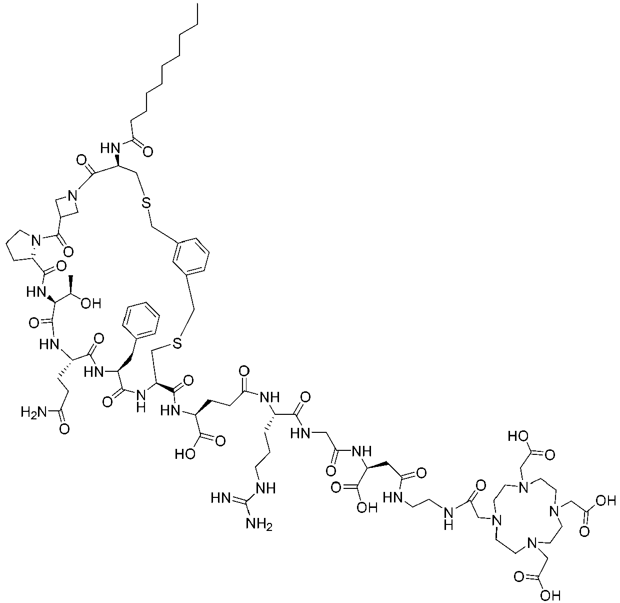
JHDC18 Dec[Cys(3MeBn)-hhy39-Pro-Thr-Gln-Phe-Cys]-Glu-(Arg-Gly-Asp(EDA-DOTA))-OH, synthesis method 1, analytical method 2 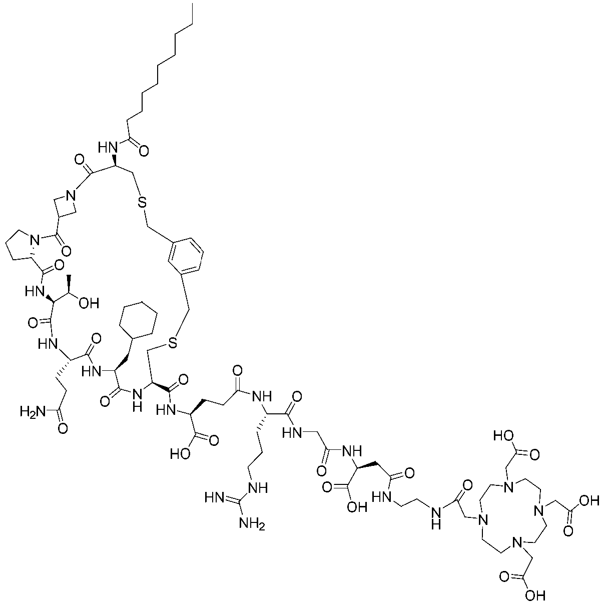
JHDC19 Dec[Cys(3MeBn)-hhy39-Pro-Thr-Gln-hhy34-Cys]-Glu-(Arg-Gly-Asp(EDA-DOTA ))-OH, synthesis method 1, analytical method 2 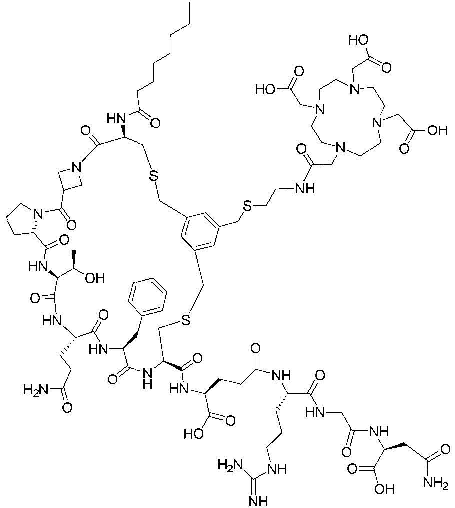
JHDC20 Oct[Cys(tMeBn-DOTA-AET)-hhy39-Pro-Thr-Gln-Phe-Cys]-Glu-(Arg-Gly-Asp(NH₂))-OH, synthesis method 1, analytical method 2 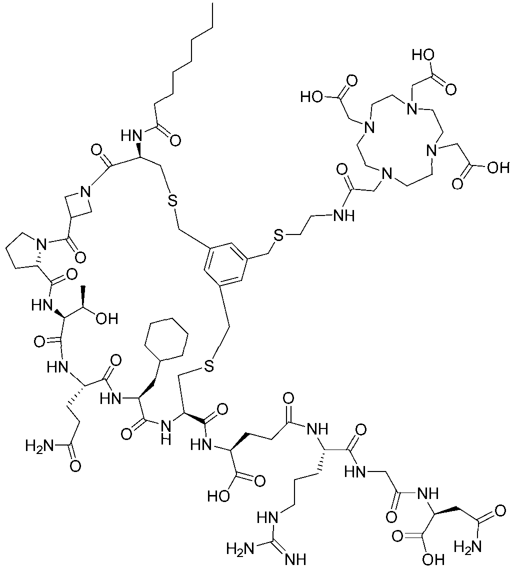
JHDC21 Oct[Cys(tMeBn-DOTA-AET)-hhy39-Pro-Thr-Gln-hhy34-Cys]-Glu-(Arg-Gly-Asp(NH₂))-OH, synthesis method 1, analytical method 2 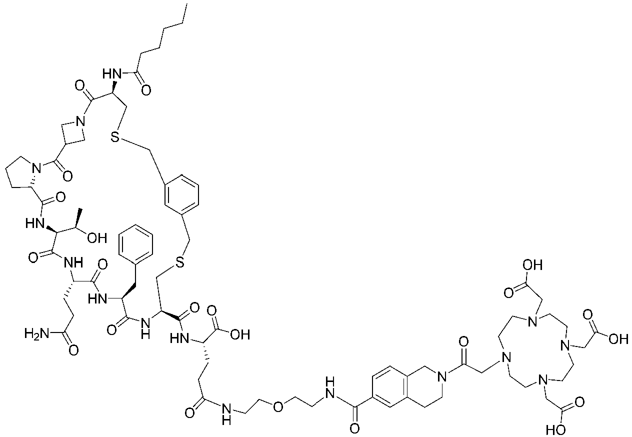
JHDC22 Hex[Cys(3MeBn)-hhy39-Pro-Thr-Gln-Phe-Cys]-Glu-(DAPEG2-hhy02-DOTA)-OH, synthesis method 1, analytical method 1 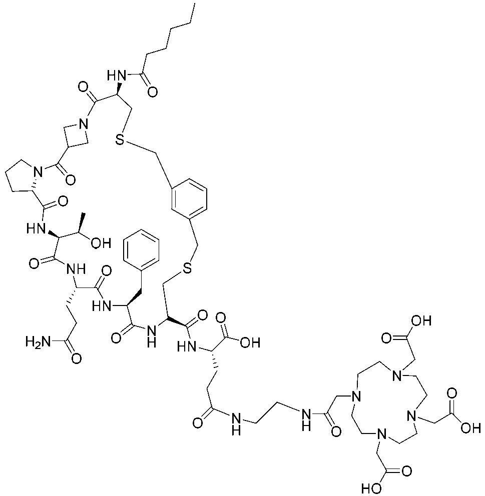
JHDC23 Hex[Cys(3MeBn)-hhy39-Pro-Thr-Gln-Phe-Cys]-Glu-(EDA-DOTA)-OH, synthesis method 1, analytical method 1 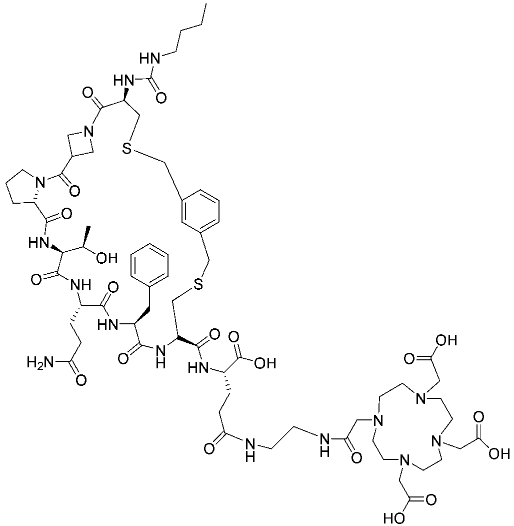
JHDC24 nBu-urea-[Cys(3MeBn)-hhy39-Pro-Thr-Gln-Phe-Cys]-Glu-(EDA-DOTA)-OH, synthesis method 1, analytical method 1 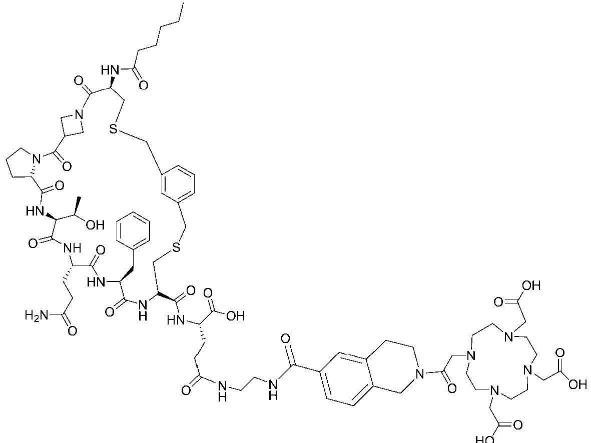
JHDC25 Hex[Cys(3MeBn)-hhy39-Pro-Thr-Gln-Phe-Cys]-Glu-(EDA-hhy02-DOTA)-OH, synthesis method 1, analytical method 1 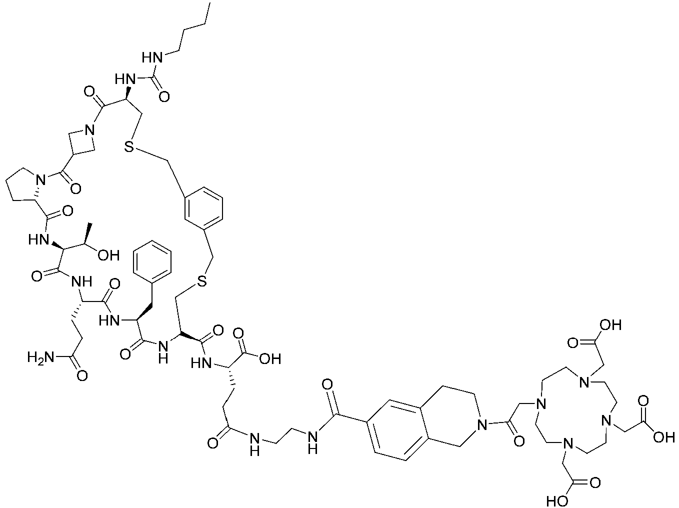
JHDC26 nBu-urea-[Cys(3MeBn)-hhy39-Pro-Thr-Gln-Phe-Cys]-Glu-(EDA-hhy02-DOTA)-OH, synthesis method 1, analytical method 1 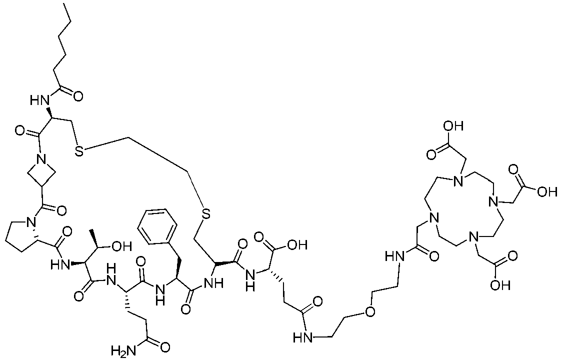
JHDC27 Hex[Cys(ethylene)-hhy39-Pro-Thr-Gln-Phe-Cys]-Glu-(DAPEG2-DOTA)-OH, synthesis method 1, analytical method 1 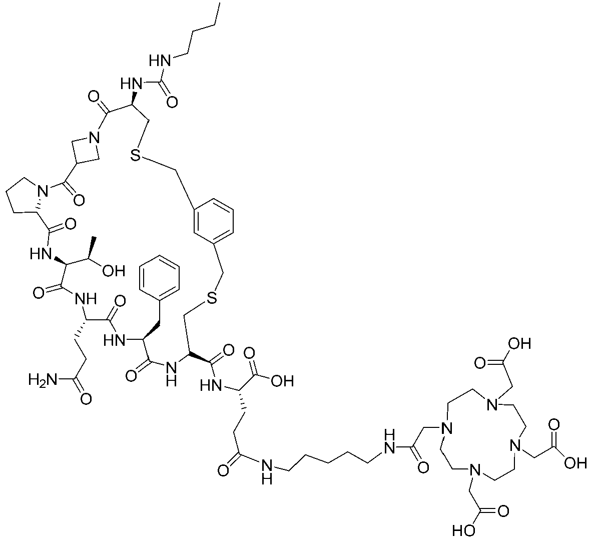
JHDC28 nBu-urea-[Cys(3MeBn)-hhy39-Pro-Thr-Gln-Phe-Cys]-Glu-(PDA-DOTA)-OH, synthesis method 1, analytical method 1 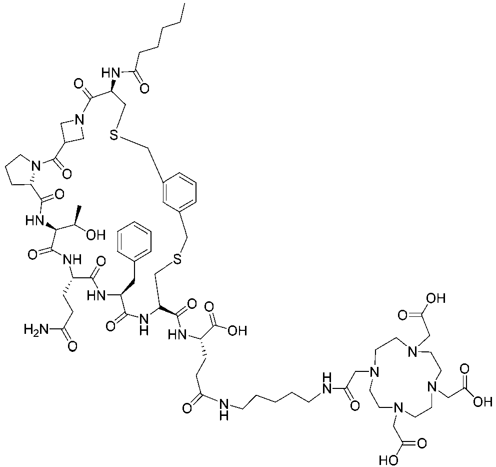
JHDC29 Hex[Cys(3MeBn)-hhy39-Pro-Thr-Gln-Phe-Cys]-Glu-(PDA-DOTA)-OH, synthesis method 1, analytical method 1 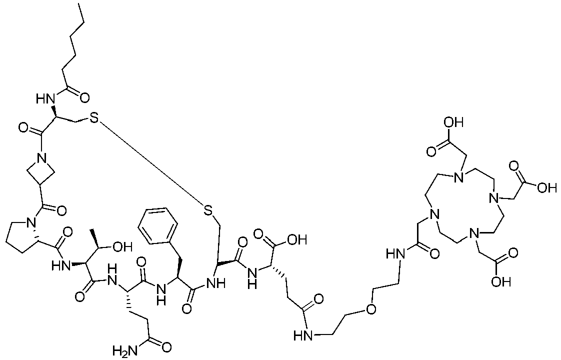
JHDC30 Hex[Cys(SS)-hhy39-Pro-Thr-Gln-Phe-Cys]-Glu-(DAPEG2-DOTA)-OH, synthesis method 1, analytical method 1 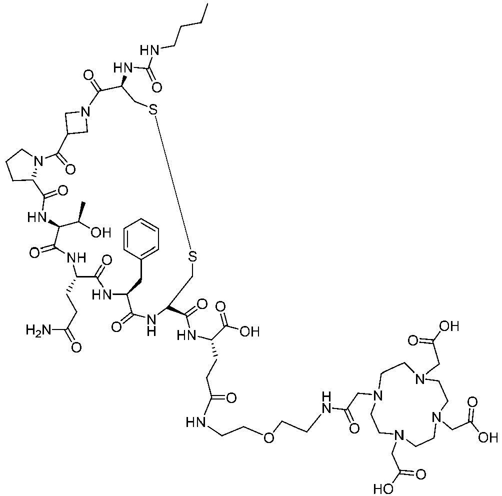
JHDC31 nBu-urea-[Cys(SS)-hhy39-Pro-Thr-Gln-Phe-Cys]-Glu-(DAPEG2-DOTA)-OH, synthesis method 1, analytical method 1 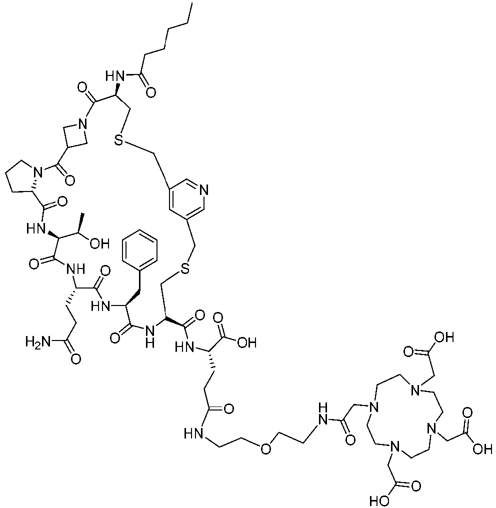
JHDC32 Hex[Cys(3MePy)-hhy39-Pro-Thr-Gln-Phe-Cys]-Glu-(DAPEG2-DOTA)-OH, synthesis method 1, analytical method 1 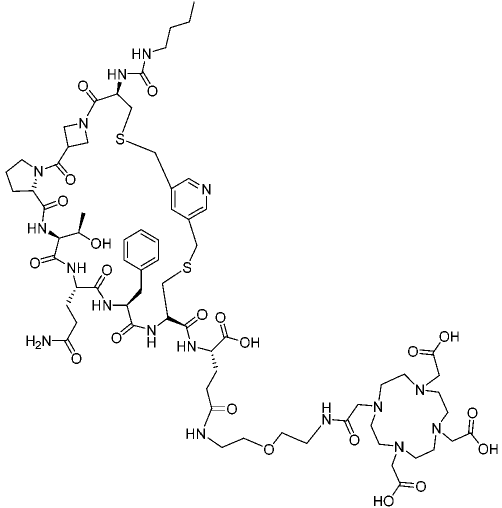
JHDC33 nBu-urea-[Cys(3MePy)-hhy39-Pro-Thr-Gln-Phe-Cys]-Glu-(DAPEG2-DOTA)-OH, synthesis method 1, analytical method 1 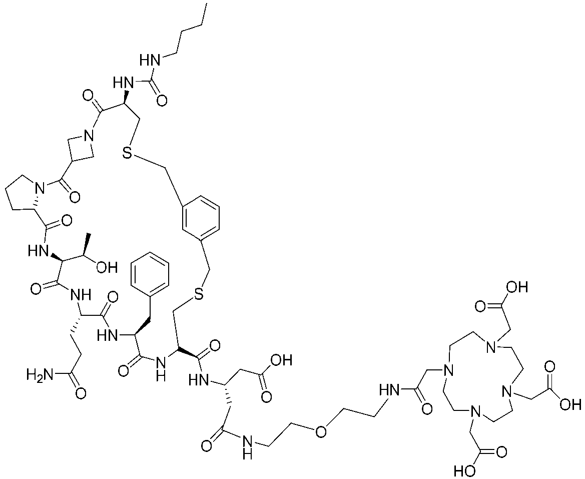
JHDC34 nBu-urea-[Cys(3MeBn)-hhy39-Pro-Thr-Gln-Phe-Cys]-β-Glu-(DAPEG2-DOTA)-OH, synthesis method 1, analytical method 1 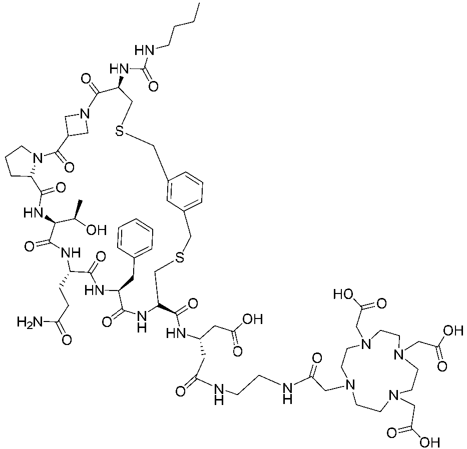
JHDC35 nBu-urea-[Cys(3MeBn)-hhy39-Pro-Thr-Gln-Phe-Cys]-β-Glu-(EDA-DOTA)-OH, synthesis method 1, analytical method 1 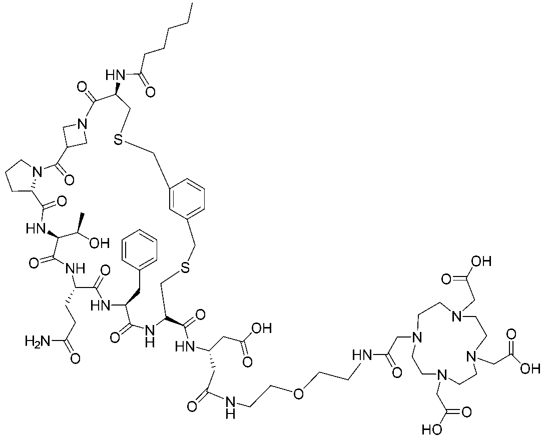
JHDC36 Hex-[Cys(3MeBn)-hhy39-Pro-Thr-Gln-Phe-Cys]-β-Glu-(DAPEG2-DOTA)-OH, synthesis method 1, analytical method 1 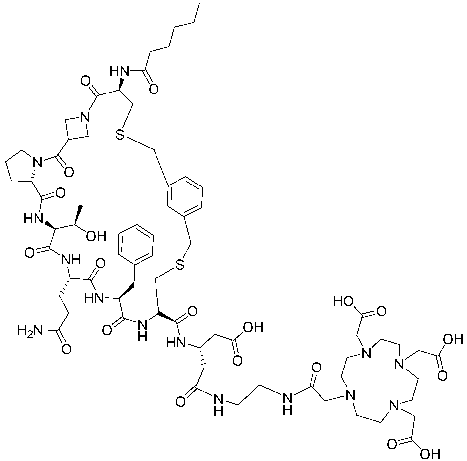
JHDC37 Hex-[Cys(3MeBn)-hhy39-Pro-Thr-Gln-Phe-Cys]-β-Glu-(EDA-DOTA)-OH, synthesis method 1, analytical method 1 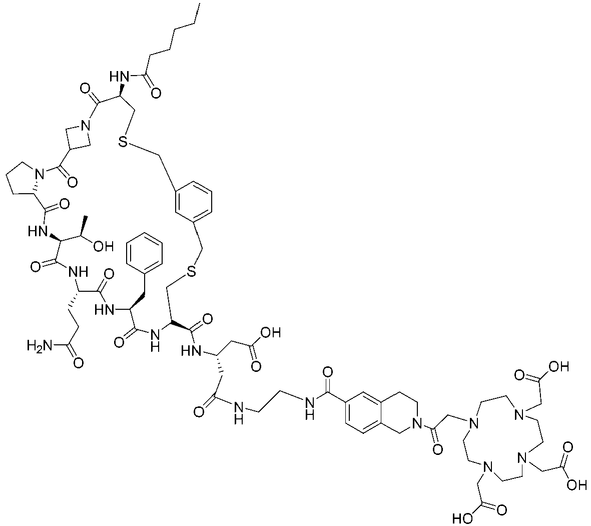
JHDC38 Hex[Cys(3MeBn)-hhy39-Pro-Thr-Gln-Phe-Cys]-β-Glu-(EDA-hhy02-DOTA)-OH, synthesis method 1, analytical method 1 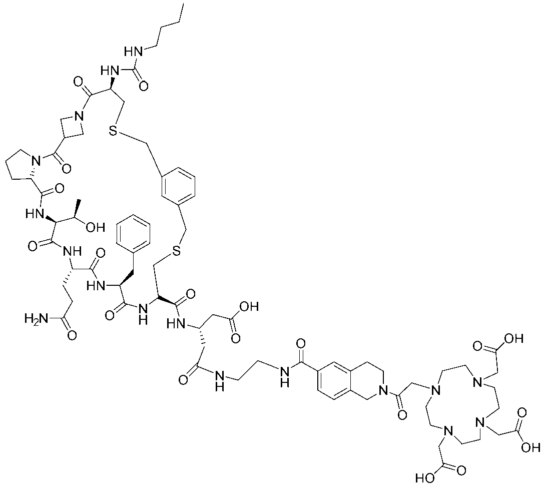
JHDC39 nBu-urea-[Cys(3MeBn)-hhy39-Pro-Thr-Gln-Phe-Cys]-β-Glu-(EDA-hhy02-DOTA)-OH, synthesis method 1, analytical method 1 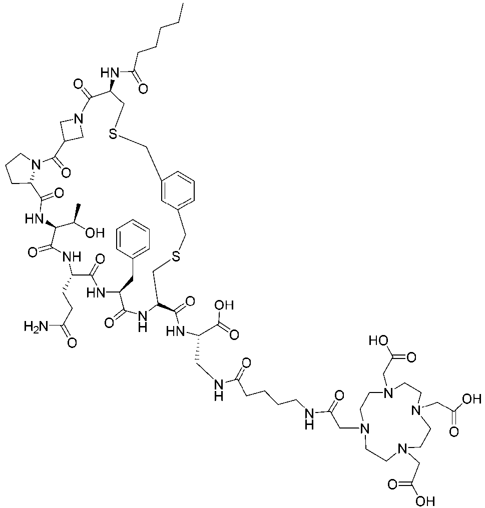
JHDC40 Hex-[Cys(3MeBn)-hhy39-Pro-Thr-Gln-Phe-Cys]-DAPA-(APTA-DOTA)-OH, synthesis method 1, analytical method 1 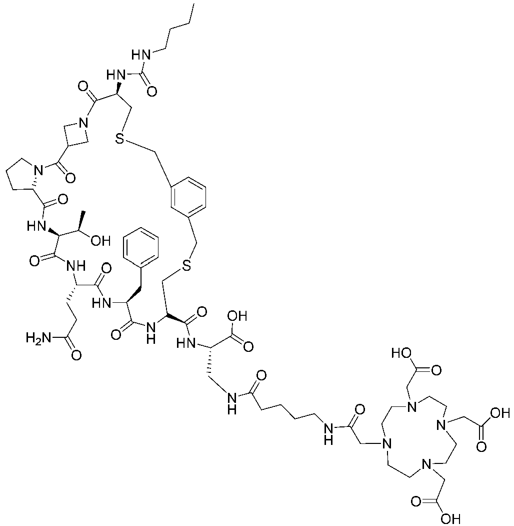
JHDC41 nBu-urea-[Cys(3MeBn)-hhy39-Pro-Thr-Gln-Phe-Cys]-DAPA- (APTA-DOTA) -OH, synthesis method 1, analytical method 1 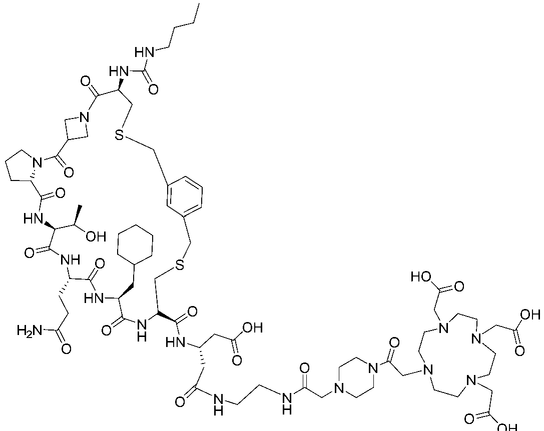
JHDC42 nBu-urea-[Cys(3MeBn)-hhy39-Pro-Thr-Gln-hhy34-Cys]-β-Glu-(EDA-piperazineacetic-DOTA)-OH, synthesis method 1, analytical method 1 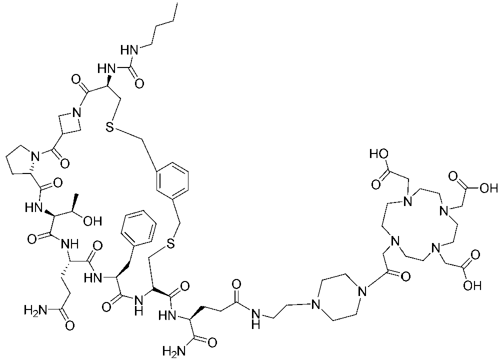
JHDC43 nBu-urea-[Cys(3MeBn)-hhy39-Pro-Thr-Gln-Phe-Cys]-Glu-(aminoethylpiperazine-DOTA)-NH₂, synthesis method 1, analytical method 1 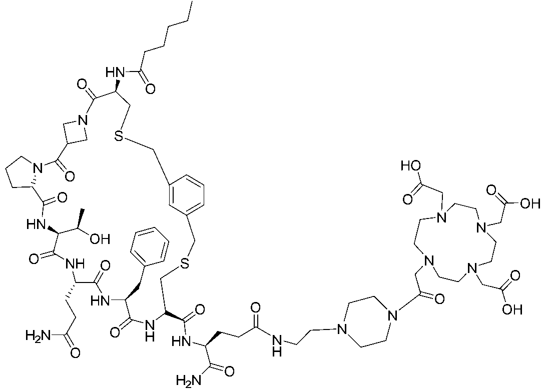
JHDC44 Hex-[Cys(3MeBn)-hhy39-Pro-Thr-Gln-Phe-Cys]-Glu-(aminoethylpiperazine-DOTA)-NH₂, synthesis method 1, analytical method 1 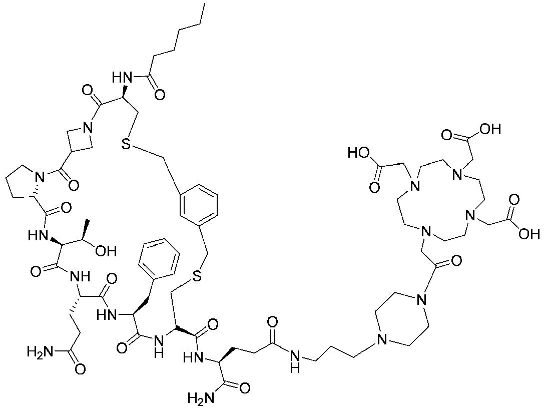
JHDC45 Hex-[Cys(3MeBn)-hhy39-Pro-Thr-Gln-Phe-Cys]-Glu-(aminopropylpiperazine-DOTA)-NH₂, synthesis method 1, analytical method 1 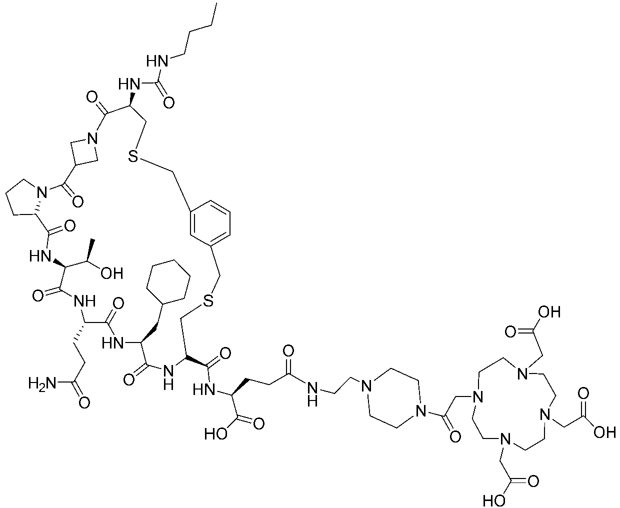
JHDC46 nBu-urea-[Cys(3MePy)-hhy39-Pro-Thr-Gln-hhy34-Cys]-Glu-(aminoethylpiperazine-DOTA)-OH, synthesis method 1, analytical method 1 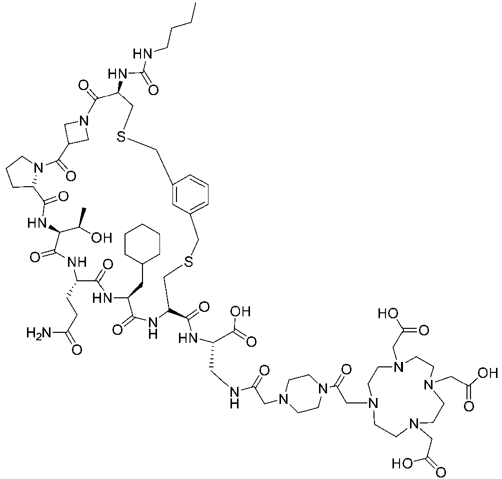
JHDC47 nBu-urea-[Cys(3MeBn)-hhy39-Pro-Thr-Gln-hhy34-Cys]-DAPA-(Ac-piperazine-DOTA)-OH, synthesis method 1, analytical method 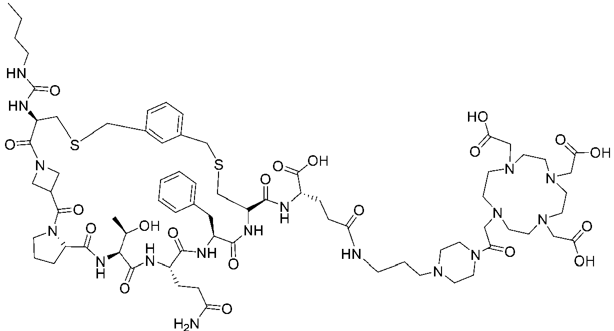
JHDC48 nBu-urea-[Cys(3MeBn)-hhy39-Pro-Thr-Gln-Phe-Cys]-Glu-(aminopropylpiperazine-DOTA)-OH, synthesis method 1, analytical method 1 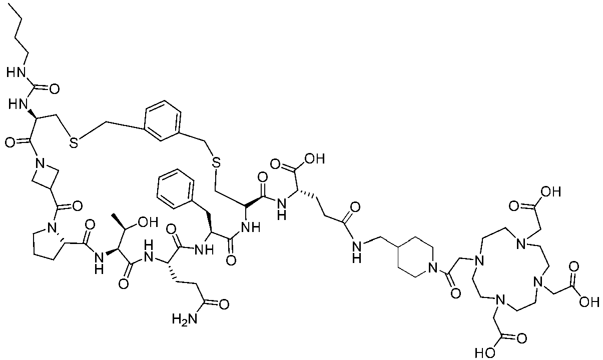
JHDC49 nBu-urea-[Cys(3MeBn)-hhy39-Pro-Thr-Gln-Phe-Cys]-Glu-(aminomethylpiperidine-DOTA)-OH, synthesis method 1, analytical method 1 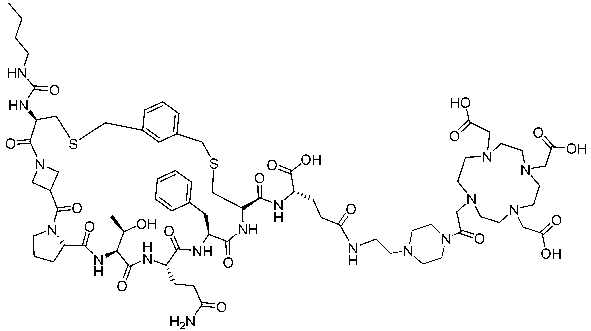
JHDC50 nBu-urea-[Cys(3MeBn)-hhy39-Pro-Thr-Gln-Phe-Cys]-Glu-(aminoethylpiperazine-DOTA)-OH, synthesis method 1, analytical method 1 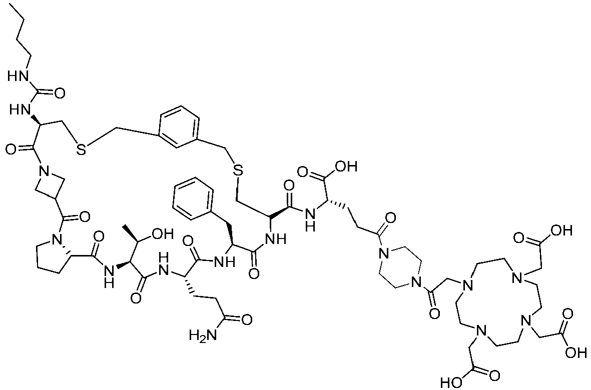
JHDC51 nBu-urea-[Cys(3MeBn)-hhy39-Pro-Thr-Gln-Phe-Cys]-Glu-(piperazine-DOT)-OH, synthesis method 1, analytical method 1 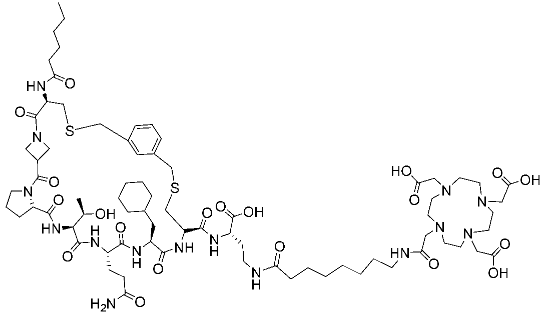
JHDC52 Hex-[Cys(3MeBn)-hhy39-Pro-Thr-Gln-hhy34-Cys]-DABA- (AOA-DOTA) -OH, synthesis method 1, analytical method 1 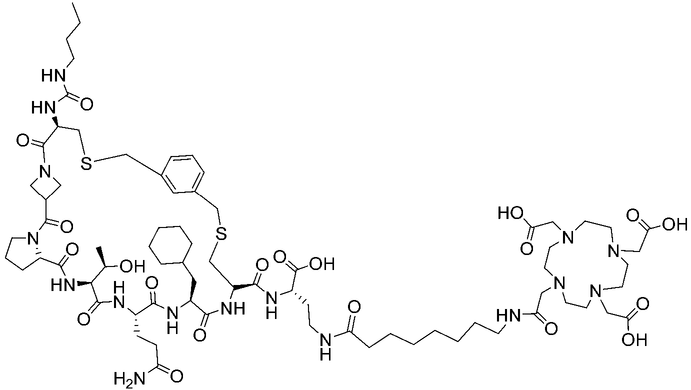
JHDC53 nBu-urea-[Cys(3MeBn)-hhy39-Pro-Thr-Gln-hhy34-Cys]-DABA-(AOA-DOTA)-OH, synthesis method 1, analytical method 1 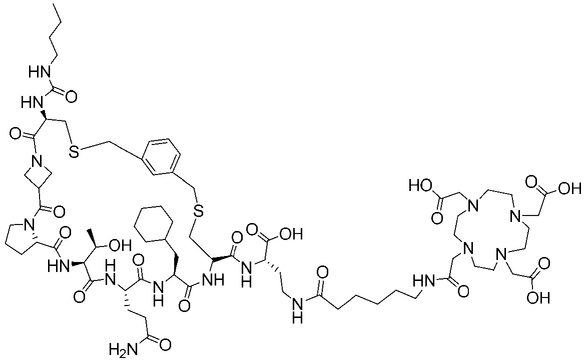
JHDC54 nBu-urea-[Cys(3MeBn)-hhy39-Pro-Thr-Gln-hhy34-Cys]-DABA-(6AHA-DOTA)-OH, synthesis method 1, analytical method 1 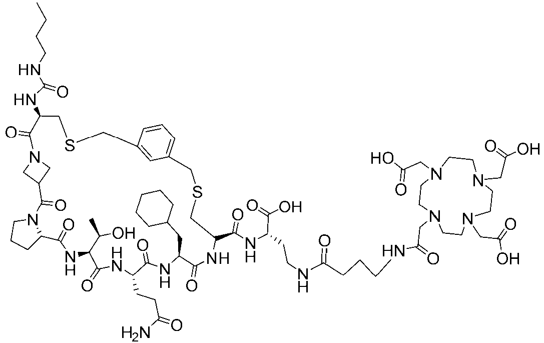
JHDC55 nBu-urea-[Cys(3MeBn)-hhy39-Pro-Thr-Gln-hhy34-Cys]-DABA-(ABA-DOTA)-OH, synthesis method 1, analytical method 1 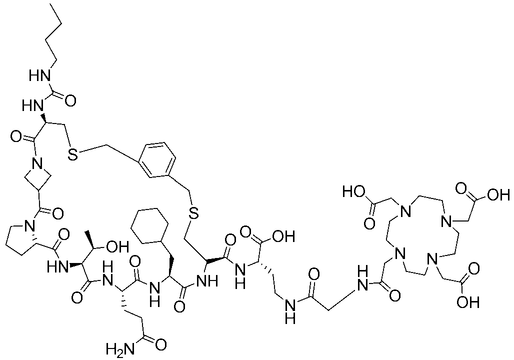
JHDC56 nBu-urea-[Cys(3MeBn)-hhy39-Pro-Thr-Gln-hhy34-Cys]-DABA-(Gly-DOTA)-OH, synthesis method 1, analytical method 1 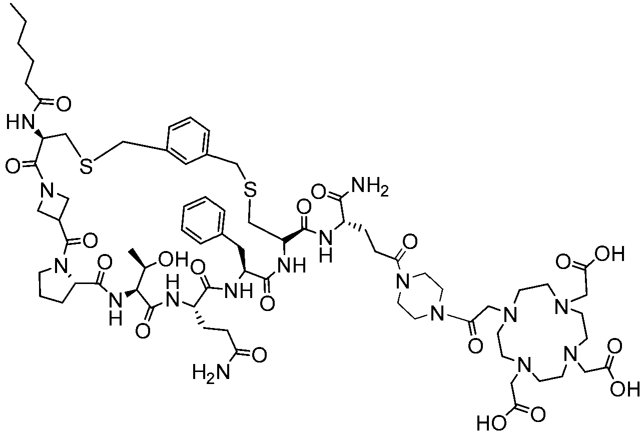
JHDC57 Hex-[Cys(3MeBn)-hhy39-Pro-Thr-Gln-Phe-Cys]-Glu-(piperazine-DOTA)-NH₂, synthesis method 1, analytical method 1 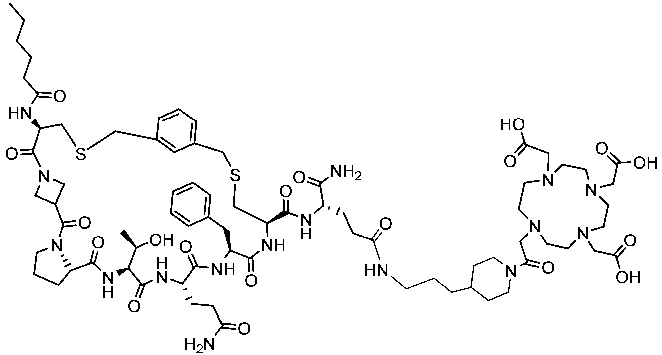
JHDC58 Hex-[Cys(3MeBn)-hhy39-Pro-Thr-Gln-Phe-Cys]-Glu-(aminopropylpiperidine-DOTA)-NH₂, synthesis method 1, analytical method 1 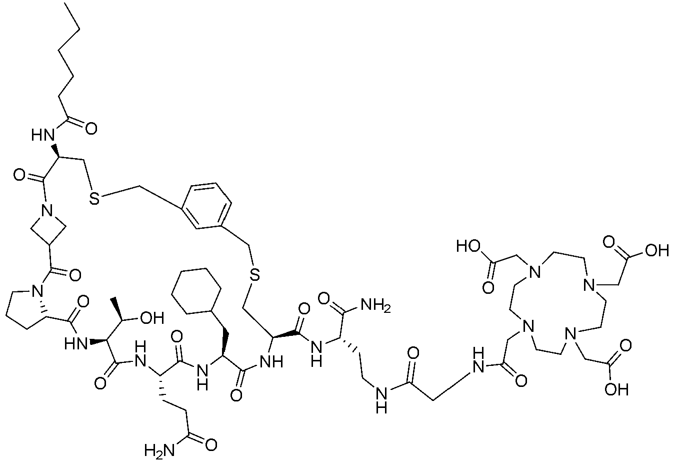
JHDC59 Hex-[Cys(3MeBn)-hhy39-Pro-Thr-Gln-hhy34-Cys]-2,4-diamino-BA-(Gly-DOTA)-NH2, synthesis method 1, analytical method 1 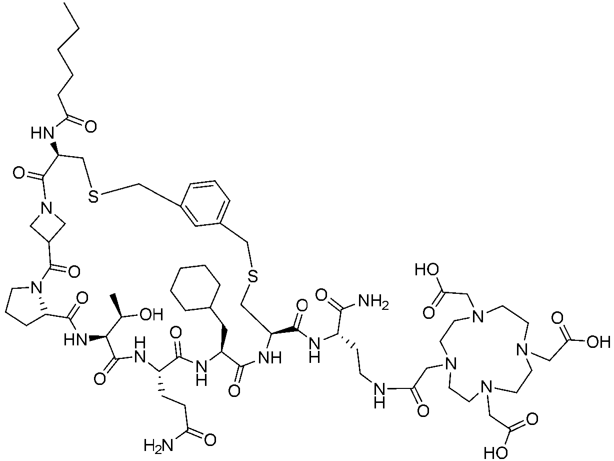
JHDC60 Hex-[Cys(3MeBn)-hhy39-Pro-Thr-Gln-hhy34-Cys]-2,4-diamino-BA-(DOTA)-NH2, synthesis method 1, analytical method 1 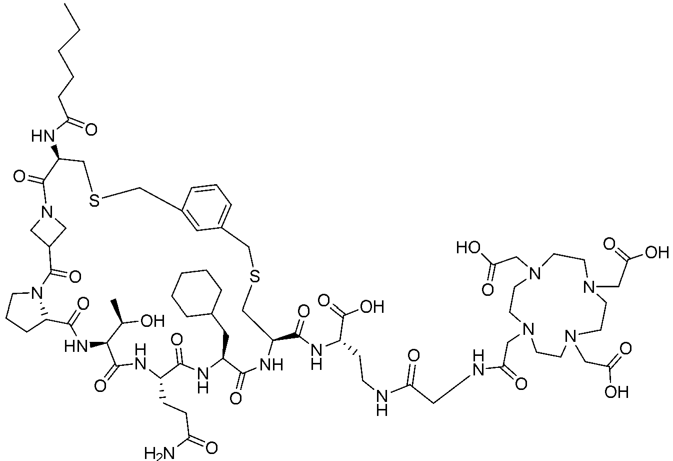
JHDC61 Hex-[Cys(3MeBn)-hhy39-Pro-Thr-Gln-hhy34-Cys]-2,4-diamino-BA-(Gly-DOTA)-OH, synthesis method 1, analytical method 1 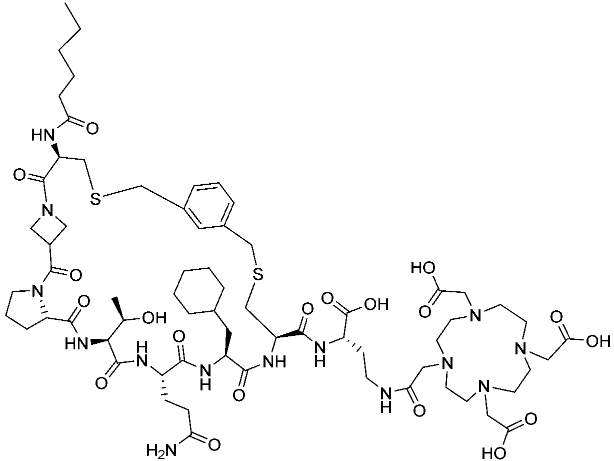
JHDC62 Hex-[Cys(3MeBn)-hhy39-Pro-Thr-Gln-hhy34-Cys]-2,4-diamino-BA-(DOTA)-OH, synthesis method 1, analytical method 1 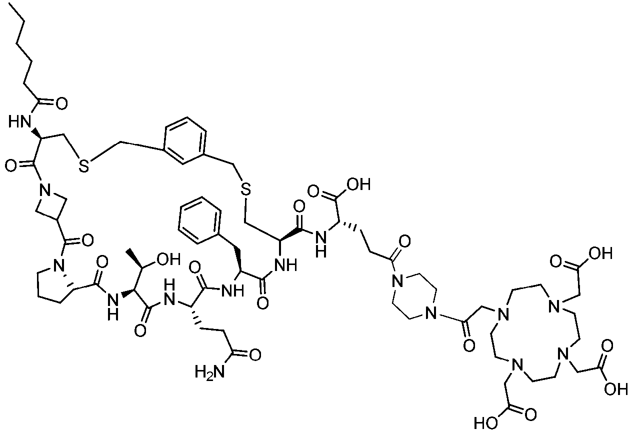
JHDC63 Hex-[Cys(3MeBn)-hhy39-Pro-Thr-Gln-Phe-Cys]-Glu-(piperazine-DOTA)-OH, synthesis method 1, analytical method 1 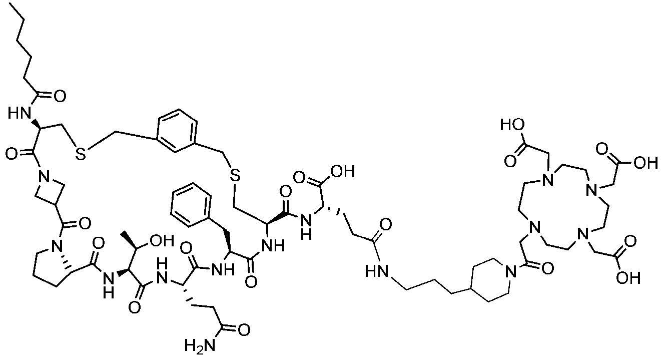
JHDC64 Hex-[Cys(3MeBn)-hhy39-Pro-Thr-Gln-Phe-Cys]-Glu-(aminopropylpiperidine-DOTA)-OH, synthesis method 1, analytical method 1

The HPLC retention times and mass spectra of the above compounds are shown in Table 1.

**Table 1**

| **No.** | **Sequence** | **HPLC (min)** | **MS [M/2 + H]⁺** |
|---|---|---|---|
| JHDC01 | Ac[Cys(3MeBn)-hhy42-Pro-Ala-Thr-Glu-Nal-Cys]-Lys-(DOTA)-OH | 10.11 | 789.12 |
| JHDC02 | Ac[Cys(3MeBn)-hhy39-Pro-Ala-Thr-Glu-Nal-Cys]-Lys-(DOTA)-OH | 12.41 | 782.12 |
| JHDC03 | Hex[Cys(3MeBn)-hhy39-Pro-Gly-Arg-Gly-Nal-Cys]-Lys-(DOTA)-OH | 10.45 | 793.9 |
| JHDC04 | Hex[Cys(3MeBn)-hhy39-Pro-D-Ala-Gln-Gly-Phe-Cys]-Lys-(DOTA)-OH | 9.35 | 761.9 |
| JHDC05 | Hex[Cys(3MeBn)-hhy39-Pro-Thr-Gln-hhy34-Cys]-Glu-(DAPEG2-DOTA)-OH | 15.32 | 795.3 |
| JHDC06 | Hex[Cys(3MeBn)-hhy39-Pro-Thr-Gln-Phe-Cys]-Glu-(DAPEG2-DOTA)-OH | 14.04 | 792.62 |
| JHDC07 | Hex[Cys(3MeBn)-hhy44(NH)-CO-Pro-Thr-Gln-Phe-Cys]-Lys-(DOTA)-OH | 11.57 | 770.5 |
| JHDC08 | Hex[Cys(3MeBn)-hhy40-Pro-Thr-Gln-Phe-Cys]-Lys-(AEAA-DOTA)-OH | 13.24 | 827.4 |
| JHDC09 | Hex[Cys(3MeBn)-hhy39-Pro-Thr-Gln-Gly-hhy34-Cys]-Glu-(DAPEG2-DOTA)-OH | 11.48 | 823.7 |
| JHDC10 | Hex[Cys(3MeBn)-hhy39-Pro-Thr-Gln-Phe-Cys]-DAPEG2-DOTA | 10.81 | 727.8 |
| JHDC 11 | Hex-[2AHA(Triazole)(DOTA-AET)-hhy44(NH)-CO-Pro-Thr-Gln-Phe-CALA]-OH | 9.95 | 740.6 |
| JHDC12 | Hex[2AHA(Triazole)-hhy39-Pro-Thr-Gln-Phe-CSMC]-Lys-(AEAA-DOTA)-OH | 11.7 | 792.5 |
| JHDC13 | Hex-[Cys(3MeBn)-hhy44-NH-CO-Pro-Thr-Gln-hhy34-Cys]-Lys-(6AHA-DOTA)-OH | 15.91 | 829.5 |
| JHDC14 | Hex-[Cys(3MeBn)-hhy44-NH-CO-Pro-Thr-Gln-hhy34-Cys]-Lys-(AEEAA-DOTA)-OH | 15.78 | 846.0 |
| JHDC15 | Hex-[Cys(3MeBn)-hhy44-NH-CO-Pro-Thr-Gln-hhy34-Gly-Cys]-Lys-(AEEAA-DOTA)-OH | 15.22 | 874.1 |
| JHDC16 | Hex[Cys(3MeBn)-hhy39-Pro-Thr-Gln-hhy34-Cys]-Glu-(DAPEG4-DOTA)-OH | 14.00 | 838.9 |
| JHDC17 | Hex[Cys(3MeBn)-hhy39-Pro-Thr-Gln-hhy34-Gly-Cys]-Glu-(DAPEG2-DOTA)-OH | 14.13 | 823.4 |
| JHDC18 | Dec[Cys(3MeBn)-hhy39-Pro-Thr-Gln-Phe-Cys]-Glu(OH)-Arg-Gly-Asp-(EDA-DOTA)-OH | 11.75 | 962.5 |
| JHDC19 | Dec[Cys(3MeBn)-hhy39-Pro-Thr-Gln-hhy34-Cys]-Glu(OH)-Arg-Gly-Asp-(EDA-DOTA)-OH | 12.78 | 965.6 |
| JHDC20 | Oct[Cys(tMeBn-DOTA-AET)-hhy39-Pro-Thr-Gln-Phe-Cys]-Glu-(Arg-Gly-Asp(NH₂))-OH | 13.06 | 971.5 [M/3+H]⁺ |
| JHDC21 | Oct[Cys(tMeBn-DOTA-AET)-hhy39-Pro-Thr-Gln-hhy34-Cys]-Glu-(Arg-Gly-Asp(NH₂))-OH | 14.19 | 650.0 [M/3+H]⁺ |
| JHDC22 | Hex[Cys(3MeBn)-hhy39-Pro-Thr-Gln-Phe-Cys]-Glu-(DAPEG2-hhy02-DOTA)-OH | 13.610 | 872.26 |
| JHDC23 | Hex[Cys(3MeBn)-hhy39-Pro-Thr-Gln-Phe-Cys]-Glu-(EDA-DOTA)-OH | 12.419 | 1540.45 [M+H]⁺ |
| JHDC24 | nBu-urea-[Cys(3MeBn)-hhy39-Pro-Thr-Gln-Phe-Cys]-Glu-(EDA-DOTA)-OH | 10.840 | 1540.79 [M+H]⁺ |
| JHDC25 | Hex[Cys(3MeBn)-hhy39-Pro-Thr-Gln-Phe-Cys]-Glu-(EDA-hhy02-DOTA)-OH | 12.466 | 1699.85 [M+H]⁺ |
| JHDC26 | nBu-urea-[Cys(3MeBn)-hhy39-Pro-Thr-Gln-Phe-Cys]-Glu-(EDA-hhy02-DOTA)-OH | 10.922 | 1700.11 [M+H]⁺ |
| JHDC27 | Hex[Cys(ethylene)-hhy39-Pro-Thr-Gln-Phe-Cys]-Glu-(DAPEG2-DOTA)-OH | 6.82 | 754.34 |
| JHDC28 | nBu-urea-[Cys(3MeBn)-hhy39-Pro-Thr-Gln-Phe-Cys]-Glu-(PDA-DOTA)-OH | 8.59 | 1582.44 [M+H]⁺ |
| JHDC29 | Hex[Cys(3MeBn)-hhy39-Pro-Thr-Gln-Phe-Cys]-Glu-(PDA-DOTA)-OH | 9.93 | 791.64 |
| JHDC30 | Hex[Cys(SS)-hhy39-Pro-Thr-Gln-Phe-Cys]-Glu-(DAPEG2-DOTA)-OH | 14.12 | 1479.26 [M + H]⁺ |
| JHDC31 | nBu-urea-[Cys(SS)-hhy39-Pro-Thr-Gln-Phe-Cys]-Glu-(DAPEG2-DOTA)-OH | 15.62 | 1480.31 [M+ H]⁺ |
| JHDC32 | Hex[Cys(3MePy)-hhy39-Pro-Thr-Gln-Phe-Cys]-Glu-(DAPEG2-DOTA)-OH | 15.95 | 792.97 |
| JHDC33 | nBu-urea-[Cys(3MePy)-hhy39-Pro-Thr-Gln-Phe-Cys]-Glu-(DAPEG2-DOTA)-OH | 14.79 | 793.74 |
| JHDC34 | nBu-urea-[Cys(3MeBn)-hhy39-Pro-Thr-Gln-Phe-Cys]-β-Glu-(DAPEG2-DOTA)-OH | 10.123 | 1584.34 [M+H]⁺ |
| JHDC35 | nBu-urea-[Cys(3MeBn)-hhy39-Pro-Thr-Gln-Phe-Cys]-β-Glu-(EDA-DOTA)-OH | 11.359 | 1540.28 [M+H]⁺ |
| JHDC36 | Hex-[Cys(3MeBn)-hhy39-Pro-Thr-Gln-Phe-Cys]-β-Glu-(DAPEG2-DOTA)-OH | 12.610 | 1583.21 [M+H]⁺ |
| JHDC37 | Hex-[Cys(3MeBn)-hhy39-Pro-Thr-Gln-Phe-Cys]-β-Glu-(EDA-DOTA)-OH | 12.566 | 1539.21 [M+H]⁺ |
| JHDC38 | Hex[Cys(3MeBn)-hhy39-Pro-Thr-Gln-Phe-Cys]-β-Glu-(EDA-hhy02-DOTA)-OH | 9.92 | 850.01 |
| JHDC39 | nBu-urea-[Cys(3MeBn)-hhy39-Pro-Thr-Gln-Phe-Cys]-β-Glu-(EDA-hhy02-DOTA)-OH | 10.85 | 800.88 |
| JHDC40 | Hex-[Cys(3MeBn)-hhy39-Pro-Thr-Gln-Phe-Cys]-DAPA-(APTA-DOTA)-OH | 13.38 | 777.08 |
| JHDC41 | nBu-urea-[Cys(3MeBn)-hhy39-Pro-Thr-Gln-Phe-Cys]-DAPA-(APTA-DOTA)-OH | 8.87 | 777.99 |
| JHDC42 | nBu-urea-[Cys(3MeBn)-hhy39-Pro-Thr-Gln-hhy34-Cys]-β-Glu-(EDA-piperazineacetic-DOTA)-OH | 9.66 | 787.02 |
| JHDC43 | nBu-urea-[Cys(3MeBn)-hhy39-Pro-Thr-Gln-Phe-Cys]-Glu-(aminoethylpiperazine-DOTA)- NH₂ | 8.21 | 755.18 |
| JHDC44 | Hex-[Cys(3MeBn)-hhy39-Pro-Thr-Gln-Phe-Cys]-Glu- (aminoethylpiperazine-DOTA)- NH₂ | 12.12 | 804.10 |
| JHDC45 | Hex-[Cys(3MeBn)-hhy39-Pro-Thr-Gln-Phe-Cys]-Glu-(aminopropylpiperazine-DOTA)- NH₂ | 9.36 | 811.17 |
| JHDC46 | nBu-urea-[Cys(3MePy)-hhy39-Pro-Thr-Gln-hhy34-Cys]-Glu-(aminoethylpiperazine-DOTA)-OH | 11.739 | 1615.94 [M+H]⁺ |
| JHDC47 | nBu-urea-[Cys(3MeBn)-hhy39-Pro-Thr-Gln-hhy34-Cys]-DAPA-(Ac-piperazine-DOTA)-OH | 11.337 | 1587.55 [M+H]⁺ |
| JHDC48 | nBu-urea-[Cys(3MeBn)-hhy39-Pro-Thr-Gln-Phe-Cys]-Glu-(aminopropylpiperazine-DOTA)-OH | 11.16 | 812.15 |
| JHDC49 | nBu-urea-[Cys(3MeBn)-hhy39-Pro-Thr-Gln-Phe-Cys]-Glu-(aminomethylpiperidine-DOTA)-OH | 13.74 | 797.66 |
| JHDC50 | nBu-urea-[Cys(3MeBn)-hhy39-Pro-Thr-Gln-Phe-Cys]-Glu-(aminoethylpiperazine-DOTA)-OH | 11.75 | 804.94 |
| JHDC51 | nBu-urea-[Cys(3MeBn)-hhy39-Pro-Thr-Gln-Phe-Cys]-Glu-(piperazine-DOTA)-OH | 13.39 | 876.32 |
| JHDC52 | Hex-[Cys(3MeBn)-hhy39-Pro-Thr-Gln-hhy34-Cys]-DABA-(AOA-DOTA)-OH | 13.37 | 1615.11 [M + H]⁺ |
| JHDC53 | nBu-urea-[Cys(3MeBn)-hhy39-Pro-Thr-Gln-hhy34-Cys]-DABA-(AOA-DOTA)-OH | 11.14 | 1616.14 [M + H]⁺ |
| JHDC54 | nBu-urea-[Cys(3MeBn)-hhy39-Pro-Thr-Gln-hhy34-Cys]-DABA-(6AHA-DOTA)-OH | 10.45 | 1588.25 [M+H]⁺ |
| JHDC55 | nBu-urea-[Cys(3MeBn)-hhy39-Pro-Thr-Gln-hhy34-Cys]-DABA-(ABA-DOTA)-OH | 10.01 | 1560.11 [M + H]⁺ |
| JHDC56 | nBu-urea-[Cys(3MeBn)-hhy39-Pro-Thr-Gln-hhy34-Cys]-DABA-(Gly-DOTA)-OH | 9.64 | 1532.17 [M + H]⁺ |
| JHDC57 | Hex-[Cys(3MeBn)-hhy39-Pro-Thr-Gln-Phe-Cys]-Glu-(piperazine-DOTA)-NH₂ | 9.86 | 782.68 |
| JHDC58 | Hex-[Cys(3MeBn)-hhy39-Pro-Thr-Gln-Phe-Cys]-Glu-(aminopropylpiperidine-DOTA)-NH₂ | 10.63 | 810.75 |
| JHDC59 | Hex-[Cys(3MeBn)-hhy39-Pro-Thr-Gln-hhy34-Cys]-2,4-diamino-BA-(Gly-DOTA)-NH₂ | 13.75 | 765.68 |
| JHDC60 | Hex-[Cys(3MeBn)-hhy39-Pro-Thr-Gln-hhy34-Cys]-2,4-diamino-BA-(DOTA)-NH2 | 14.11 | 737.19 |
| JHDC61 | Hex-[Cys(3MeBn)-hhy39-Pro-Thr-Gln-hhy34-Cys]-2,4-diamino-BA- (Gly-DOTA)-OH | 11.49 | 766.23 |
| JHDC62 | Hex-[Cys(3MeBn)-hhy39-Pro-Thr-Gln-hhy34-Cys]-2,4-diamino-BA-(DOTA)-OH | 11.66 | 737.75 |
| JHDC63 | Hex-[Cys(3MeBn)-hhy39-Pro-Thr-Gln-Phe-Cys]-Glu-(piperazine-DOTA)-OH | 10.25 | 783.24 |
| JHDC64 | Hex-[Cys(3MeBn)-hhy39-Pro-Thr-Gln-Phe-Cys]-Glu-(aminopropylpiperidine-DOTA)-OH | 11.14 | 811.31 |

**Example 2:** Cold labeling of compound ¹⁷⁵Lu: Taking JHDC23 as an example,

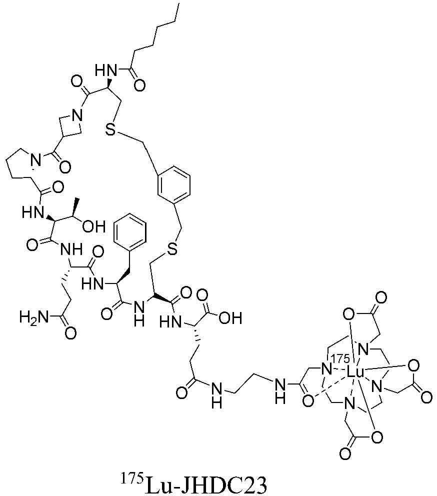

2.66 mg of precursor JHDC23 was precisely weighed and dissolved in 1.9 mL of sodium acetate buffer (pH: 5.0), followed by the addition of 100 µL of lutetium chloride aqueous solution (50 mg/mL). The solution was placed in a reactor at 95°C and reacted for 20 minutes. The reaction mixture was cooled and purified by prep-HPLC (15-45%, acetonitrile/water) to afford a white solid product (1.5 mg). HPLC: 11.21 min, 99.83% purity; MS: [M/2+H]⁺: 895.95.

### Example 3: Test of compound affinity

The Biacore 8K (Cytiva) instrument was used to detect ligand binding of the FAP protein (Sinobiological). FAP protein was captured on the SA chip. Prior to ligand immobilization (flow paths 1 and 2, flow rate of 10 µL/min), FAP protein (10 µg/mL, flow rate of 5 µL/min, injection time of 600 s) was immobilized on flow path 2 using running buffer. The sensor surface was conditioned by injecting 1M NaCl three consecutive times in 50 mM NaOH. After each ligand injection, additional cleaning was performed using isopropanol in 1M NaCl and 50 mM NaOH (flow path 1 and 2, flow rate of 10 µL/min, injection time of 60 s).

The tested compound was dissolved in 100% dimethyl sulfoxide and diluted to 10 mM, then diluted in the assay buffer (PBS, pH 7.4, 1 mM TCEP (tris-(2-hydroxylethyl)phosphine), 0.05% P20, 2% dimethyl sulfoxide) to an appropriate maximum concentration. The analyte was run under the following conditions: analysis temperature of 15 °C, analysis step = all set to LMW kinetics; cycle type = single cycle (contact time of 90 s, dissociation time of 1800 s, flow rate of 30 µL/min, flow channels 1 and 2); channel detection = 2-1. Data was evaluated using the Biacore Insight Evaluation Software, and the data were fitted to a 1:1 binding model.

The Biacore results are shown in Table 2 below: pK_{D} = -LogK_{D}, where K_{D} is the binding affinity of the compound to the FAP protein as measured by Biacore. It was expressed by K_{D} (M) = K_{d}(1/s)/Kₐ(1/Ms), where A: pK_{D} >8, B: 7<pK_{D}<8, C: 6<pK_{D}<7, D: pK_{D}<6.

Additionally, FAPi-46 (CAS: 2374782-04-2) and FAP-2286 (CAS: 2581741-18-4) were used as positive reference compounds.

### Example 4: Determination of compound selectivity:

The specificity of the synthesized compounds for recombinant human dipeptidyl peptidase IV (DPPIV), fibroblast activation protein (FAP), or prolyl oligopeptidase (PREP) was expressed by their half-maximal inhibitory concentration (IC₅₀) against the proteases. The specific steps were as follows:
1) The synthesized compound was dissolved in DMSO to a final concentration of 100 mM.
   a) For FAP assay: the test compound was diluted to a 1 mM solution using a buffer of 50 mM Tris, 140 mM NaCl at pH 7.5;
   b) For DPPIV assay: the test compound was diluted to a 1 mM solution using a 25 mM Tris, 250 mM NaCl buffer at pH 7.5;
   c) For PREP assay: the test compound was diluted to a 1 mM solution using a 140 mM NaCl buffer at pH 8.0.
2) The prepared 1 mM test compound solutions were each serially diluted (1:10) with the corresponding buffers described above into one row of a 96-well plate.
3) The substrate was prepared as a DMSO stock solution (FAP and PREP: 2.5 mM Z-Gly-Pro-AMC (VWR, Cat. No. 1-1145.0050BA) in DMSO; DPPIV: 100 mM Gly-Pro-AMC (VWR, Cat. No. 100042-646) in DMSO), and the stock solution of the substrate was subsequently diluted 20-fold using the corresponding buffer described above.
4) The enzyme was diluted into an appropriate assay buffer. The final concentrations of DPPIV, FAP, and PREP enzymes should be 0.1, 1.2, and 0.6 nM, respectively. 180 µL were added to each well of columns 2-10 as required. Column 1 (A, B, C) was prepared using 200 µL of an appropriate analysis buffer as a control. Column 1 (D, E, F, G, H) was prepared with 20 µL of an appropriate assay buffer and 180 µL of the enzyme as the inhibitor-free control.
5) Where appropriate, 20 µL of the test compound was added from the dilution plate prepared in step 2 to columns 2-10 of the assay plate. Each sample should be tested in triplicate. It was incubated at room temperature for 10 minutes, with the plate being shaken for the first two minutes.
6) 10 µL of the 20x substrate prepared in step 3 was added to each well and incubated at room temperature for 15 minutes, with the plate being shaken for the first two minutes.
7) Fluorescence was read at λex:380 and λem:460.

The above test results indicated that the synthesized compound exhibited good selectivity for FAP,
wherein pIC₅₀ = -Log (IC₅₀), wherein A: pIC₅₀ >8, B: 7<pIC₅₀<8, C: 6<pIC₅₀<7, D: pIC₅₀<6.

**Table 2**

| No. | pKD (FAP) | pIC₅₀ (FAP) | pIC₅₀ (DPPIV) | pIC₅₀ (PREP) |
|---|---|---|---|---|
| JHDC01 | - | | | |
| JHDC02 | - | | | |
| JHDC03 | - | | | |
| JHDC05 | A | | | |
| JHDC06 | A | | | |
| JHDC07 | B | | | |
| JHDC08 | - | | | |
| JHDC09 | B | | | |
| JHDC 10 | B | | | |
| JHDC12 | B | | | |
| JHDC 16 | B | | | |
| JHDC 17 | B | | | |
| JHDC 18 | B | | | |
| JHDC 19 | B | | | |
| JHDC21 | B | | | |
| JHDC23 | B | A | D | D |
| ¹⁷⁵Lu-JHDC23 | B | | | |
| JHDC24 | B | A | D | D |
| JHDC25 | B | | | |
| JHDC26 | B | | | |
| JHDC27 | B | | | |
| JHDC28 | B | | | |
| JHDC29 | B | | | |
| JHDC30 | B | | | |
| JHDC31 | B | | | |
| JHDC32 | B | | | |
| JHDC33 | B | | | |
| JHDC34 | B | | | |
| JHDC35 | B | | | |
| JHDC36 | B | | | |
| JHDC37 | B | A | D | D |
| ¹⁷⁵Lu-JHDC37 | B | | | |
| JHDC40 | B | | | |
| JHDC41 | B | | | |
| JHDC42 | B | | | |
| JHDC43 | B | | | |
| JHDC44 | B | | | |
| JHDC45 | A | | | |
| JHDC46 | B | | | |
| ¹⁷⁵Lu-JHDC46 | B | | | |
| JHDC47 | A | A | D | D |
| JHDC48 | B | A | D | D |
| JHDC50 | B | | | |
| JHDC53 | B | A | D | D |
| JHDC55 | A | | | |
| JHDC56 | A | A | D | D |
| ¹⁷⁵Lu-JHDC56 | B | | | |
| FAP-2286 | B | A | D | D |

| | | | | |
|---|---|---|---|---|
| "-" indicates not tested. | | | | |

### Example 5: Radio labeling of compound with ¹⁷⁷Lu

1. The precursor compound (1 mg) was weighed and dissolved in 0.45 M ascorbic acid buffer at pH 4.5 to prepare a 0.1 mg/mL solution.
2. Labeling was performed according to a nuclide:precursor = 1:7-10 (molar ratio): 2 mCi of ¹⁷⁷LuCl₃ and the amount of precursor calculated based on the real-time specific activity were added to a labeling buffer system (0.5 M pH 4.0 ascorbic acid buffer), such that the reaction system had a volume of 0.15 mL.
3. The reaction was carried out at 95°C for 30 minutes on a constant temperature heater. After completion, the product was analyzed by iTLC and Radio-HPLC.
4. iTLC developing solvent: 1% EDTA, stationary phase: silica gel 254, developed to 1 cm from the top of the stationary phase, which took approximately 20 minutes, and the spotting volume was 0.5 µL.
5. If the results of iTLC and Radio-HPLC reach 95%, the labeled product is considered qualified.
6. The reaction mixture was transferred to a vial, and the reaction tube was rinsed with 0.15 mL of physiological saline, with the rinse also added to the vial.
7. 6.7 µL of DTPA solution (0.25 mM) was added and mixed for later use. 1.0 mg of DTPA was added to 10 mL of physiological saline to prepare a DTPA stock solution (concentration: 0.25 mM), which was mixed and set aside for use.

The final formulation should be a clear solution, prepared immediately before use and restricted to same-day use only.

### Example 6: Cell binding assay of compounds

(1) HT1080 8# cells in the logarithmic growth phase (Genewiz, Suzhou) were prepared into a cell suspension, and the cell density was adjusted to 1×10⁴/mL. Then, 1 mL of the suspension was seeded into a 24-well cell culture plate. The mixture was incubated overnight in a 37°C incubator.
(2) The cell culture medium was aspirated, the cells were washed once with PBS, and 975 µL of additive-free medium was added.
(3) 25 µL of ¹⁷⁷Lu-labeled FAPi-46 (CAS: 2374782-04-2) ligand at a fixed concentration (final concentration in culture medium: 1.35 µCi/mL) and the unlabeled test compound at varying concentrations (final concentrations in culture medium: 1000 ng/mL, 100 ng/mL, 10 ng/mL, 1 ng/mL, 0.1 ng/mL, 0.01 ng/mL, 0.001 ng/mL, 0 ng/mL) were added to each well.
(4) The mixture was incubated on ice for 2 hours.
(5) The cells were washed three times with ice-cold PBS.
(6) The cells were lysed with 0.5 mL of 1M sodium hydroxide, washed twice with 0.5 mL of PBS, and the sodium hydroxide (0.5 mL) and PBS (0.5 mL×2) solutions were collected to determine the uptake count. The results are shown in Table 3.

**Table 3: Cell binding assay results of compounds**

| Compound No. | IC₅₀ (ng/mL) | Compound No. | IC₅₀ (ng/mL) |
|---|---|---|---|
| FAP-2286 | 13.17 | JHDC06 | 6.801 |
| JHDC56 | 3.31 | JHDC64 | 2.75 |

### Example 7: Determination of ClogP

A certain amount of ¹⁷⁷Lu-labeled compound solution was taken and mixed with ultrapure water, and the activity was measured after mixing.

Two EP tubes were taken, and 100 µL of saturated n-octanol aqueous solution and 80 µL of pure water were added to each tube, respectively. Then, 20 µL of the above ¹⁷⁷Lu-labeled compound solution was added to each tube. After shaking and mixing for 2 hours, the tubes were centrifuged at room temperature at 2000 rpm/min for 5 minutes. 20 µL was taken from the upper layer (lipid layer) and lower layer (aqueous layer) of each tube, and gamma counts were measured. The results are shown in Table 4.

**Table 4: Ppartition coefficient (ClogP) of compounds**

| Compound No. | ClogP | Compound No. | ClogP |
|---|---|---|---|
| ¹⁷⁷Lu -FAP-2286 | -2.74 | ¹⁷⁷Lu -JHDC13 | -1.3 |
| ¹⁷⁷Lu -JHDC06 | -2.69 | ¹⁷⁷Lu -JHDC15 | -1.6 |
| ¹⁷⁷Lu -JHDC11 | -3.65 | | |
| ¹⁷⁷Lu -JHDC12 | -3.81 | | |
| ¹⁷⁷Lu -JHDC14 | -1.39 | | |

### Example 8: Binding rate of labeled compound to plasma protein

¹⁷⁷Lu-labeled compound (1 µCi) was taken and mixed with 50 µL PBS to obtain a reaction solution with a concentration of 20 µCi/mL. 50 µL of a 20 µCi/mL reaction solution was added to 200 µL of plasma, mixed, and incubated at room temperature for 10 minutes. The sample was then transferred to a 30K ultrafiltration tube (PALL) and centrifuged at 13,000 rpm for 45 minutes. Subsequently, 50 µL of normal saline was added, and centrifugation was continued for another 15 minutes. Both the retentate and the filtrate were counted separately.

PPB = [(Upper Layer Count - Background Count)] / (Supernatant Count + Upper Layer Count - 2*Background Count)] * 100%.

### Example 9: biodistribution of ¹⁷⁷Lu-labeled compound in tumor-bearing mice

Tissue distribution in FAP-positive tumor-bearing mice: Balb/c Nude mice aged approximately 6-9 weeks were subcutaneously inoculated with 2× 10⁶ HT1080 2# FAP cells (in 50% Matrigel, Corning) in the right shoulder region. When the tumor grew to a size of approximately 150-350 mm³, the ¹⁷⁷Lu radiolabeled compound was administered to the mice via tail vein injection (approximately 3.7 MBq/mouse). After dosing, the animals were euthanized by carbon dioxide inhalation at 24 hours. Following euthanasia, animal blood and organs (liver, kidney, muscle tumor) were collected.

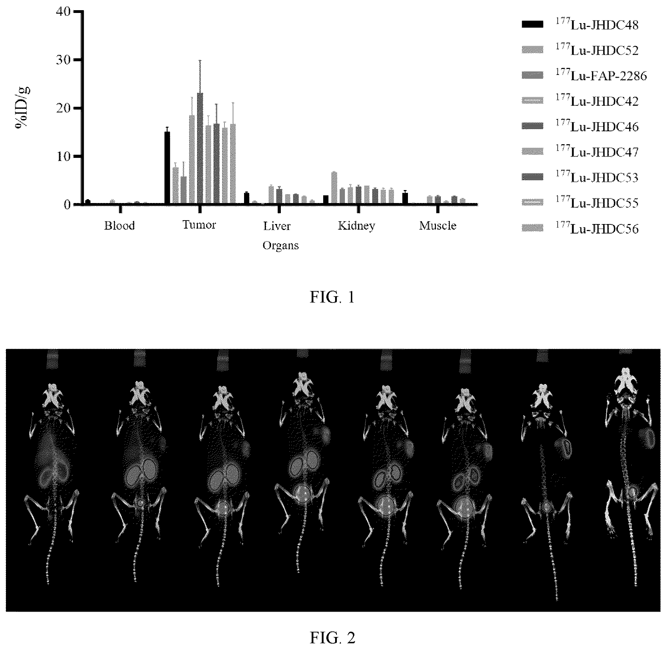

Blood was collected via the inferior vena cava, and 100 µL was immediately aliquoted into a designated centrifuge tube (weighed). After collection, the organs were washed twice with deionized water and dried, then placed into pre-weighed test tubes and weighed again to calculate the sample weight. The samples were measured on the day of collection. All blood and tissue samples were measured for radioactive counts using a gamma counter. Tissue distribution test results are shown in Table 5 and FIG. 1.

**Table 5: Distribution of compounds in tumor-bearing mice (@24 h, %ID/g)**

| | Blood | Tumor | Liver | Kidney | Muscle |
|---|---|---|---|---|---|
| ¹⁷⁷Lu-FAP-2286 | 0.0150±0.0035 | 5.7750±2.1673 | 0.1450±0.0106 | 3.2100±0.1485 | 0.0400±0.0000 |
| ¹⁷⁷Lu-JHDC48 | 0.9650±0.0247 | 15.1300±0.6505 | 2.4650±0.1025 | 1.9000±0.0000 | 2.4400±0.3253 |
| ¹⁷⁷Lu-JHDC52 | 0.0600±0.0000 | 7.7600±0.6293 | 0.7400±0.0495 | 6.7150±0.0601 | 0.3350±0.0177 |
| ¹⁷⁷Lu-JHDC42 | 0.9600±0.1131 | 18.4500±2.7153 | 3.7900±0.1980 | 3.6150±0.3854 | 1.7500±0.0919 |
| ¹⁷⁷Lu-JHDC46 | 0.0500±0.0000 | 23.1900±4.7447 | 3.2350±0.3712 | 3.7650±0.1732 | 1.7150±0.1520 |
| ¹⁷⁷Lu-JHDC47 | 0.4450±0.0318 | 16.4250±1.3753 | 2.1000±0.0141 | 3.9050±0.0035 | 0.6700±0.0990 |
| ¹⁷⁷Lu-JHDC53 | 0.5850±0.0460 | 16.8100±2.8355 | 2.1050±0.0672 | 3.2100±0.1838 | 1.7300±0.0141 |
| ¹⁷⁷Lu-JHDC55 | 0.4600±0.0141 | 15.9650±0.8167 | 1.6900±0.0566 | 3.1400±0.2051 | 1.2500±0.0566 |
| ¹⁷⁷Lu-JHDC56 | 0.0750±0.0035 | 16.7650±3.0795 | 0.8450±0.0672 | 3.0750±0.2369 | 0.3450±0.0035 |

### Example 10: image results of ⁶⁸Ga-labeled compound in tumor-bearing mice

A. JHD compound was dissolved in ascorbic acid buffer to prepare a solution with a concentration of 0.1 mg/mL.
B. The germanium-gallium generator was eluted stepwise with 5 mL of 0.1 M HCl, and the fraction with the highest activity (0.5 mL) was collected. To this, 0.5 mL of a metal-free 0.1 M sodium ascorbate buffer at pH = 4.5 was added. In a 1.5 mL centrifuge tube used as the reaction vessel, a specified amount of precursor [1000 (molecular weight) / 14.94 µL (precursor solution) / mCi (radionuclide)] was added. The mixture was vortexed for 10 s and heated at 95°C with 800 rpm shaking for 15 min.
C. The C18 cartridge was activated with absolute ethanol, rinsed with pure water, and then flushed to dryness.
D. The reaction solution was passed through a C18 column, rinsed with pure water and drained, then eluted with ethanol by collecting 3 drops per tube for a total of approximately 10 tubes.
E. The final formulation should be a clear solution, prepared immediately before use and restricted to same-day use only.

### PCT/CT scan

1) The PET/CT system was powered on according to the standard operating procedures, the scanning software was launched, and daily calibration was performed.
2) Animal anesthesia preparation, wherein tumor-bearing mice were anesthetized with isoflurane.
3) After the tumor-bearing mice lost their righting reflex, the imaging agent was administered via tail vein injection.
4) After administration, dynamic scanning at 1 hour and static scanning was performed at 3 hours were performed for 10 minutes.
5) During the process, animal body weight, injection dose, injection time, and residual dose were recorded according to the record sheet, and the time of measuring the injection dose and the time of measuring the residual dose were separately recorded.
6) The uptake of animal tumors, muscles, and other organs was performed using PMOD software.

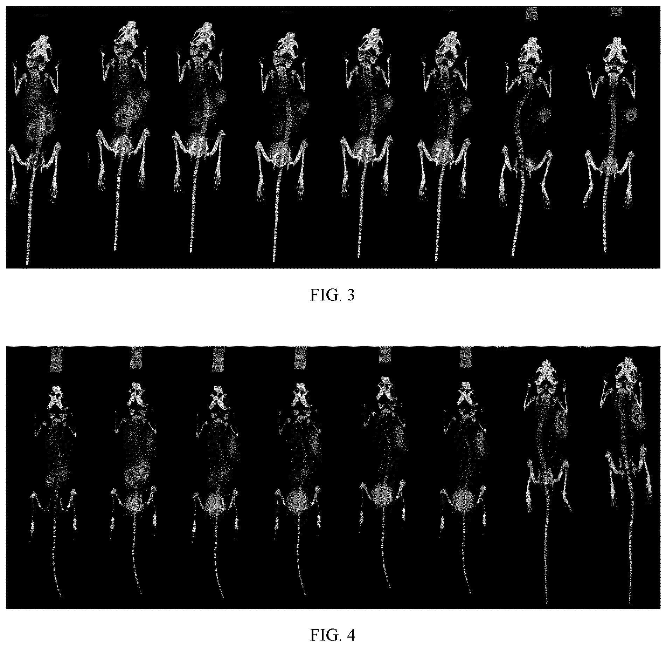

PET-CT results showed that after intravenous injection via the tail vein of tumor-bearing mice, ⁶⁸Ga-labeled compounds were rapidly distributed to various organs and tumors of the animals and were quickly metabolized and excreted through the kidneys. The uptake of the labeled compounds in the tumors varied over time, and compared with the reference compound, several compounds exhibited prolonged retention in the tumors. The results are shown in Tables 5-9 and FIGs. 2-4.

**Table 5: Distribution changes of ⁶⁸Ga-labeled compounds in mouse heart over time**

| Compound No. | 5 minutes | 10 minutes | 15 minutes | 20 minutes | 25 minutes | 30 minutes | 1.5 hours | 3 hours |
|---|---|---|---|---|---|---|---|---|
| ⁶⁸Ga-FAP-2286 | 7.82 | 4.89 | 4.11 | 2.98 | 2.86 | 2.64 | 0.41 | 0.13 |
| ⁶⁸Ga-JHDC16 | 7.25 | 4.60 | 2.92 | 2.74 | 1.95 | 1.61 | 1.00 | 0.50 |
| ⁶⁸Ga-JHDC06 | 4.17 | 4.43 | 2.74 | 2.23 | 1.90 | 1.70 | 1.10 | 0.80 |

**Table 6: Distribution changes of ⁶⁸Ga-labeled compounds in mouse liver over time**

| Compound No. | 5 minutes | 10 minutes | 15 minutes | 20 minutes | 25 minutes | 30 minutes | 1.5 hours | 3 hours |
|---|---|---|---|---|---|---|---|---|
| ⁶⁸Ga-FAP-2286 | 8.07 | 5.34 | 3.92 | 3.08 | 2.52 | 2.20 | 0.84 | 0.33 |
| ⁶⁸Ga-JHDC16 | 4.66 | 3.83 | 2.64 | 2.99 | 2.38 | 2.45 | 2.00 | 1.20 |
| ⁶⁸Ga-JHDC06 | 2.42 | 3.07 | 1.84 | 1.51 | 1.24 | 1.14 | 0.85 | 0.66 |

**Table 7: Distribution changes of ⁶⁸Ga-labeled compounds in mouse kidney over time**

| Compound No. | 5 minutes | 10 minutes | 15 minutes | 20 minutes | 25 minutes | 30 minutes | 1.5 hours | 3 hours |
|---|---|---|---|---|---|---|---|---|
| ⁶⁸Ga-FAP-2286 | 12.67 | 28.58 | 28.94 | 22.80 | 17.03 | 14.06 | 2.40 | 1.80 |
| ⁶⁸Ga-JHDC16 | 14.69 | 14.23 | 7.15 | 5.72 | 4.88 | 4.11 | 1.40 | 1.50 |
| ⁶⁸Ga-JHDC06 | 5.62 | 11.98 | 5.33 | 3.83 | 3.10 | 2.94 | 1.60 | 1.50 |

**Table 8: Distribution changes of ⁶⁸Ga-labeled compounds in mouse muscle over time**

| Compound No. | 5 minutes | 10 minutes | 15 minutes | 20 minutes | 25 minutes | 30 minutes | 1.5 hours | 3 hours |
|---|---|---|---|---|---|---|---|---|
| ⁶⁸Ga-FAP-2286 | 0.78 | 1.58 | 1.86 | 1.42 | 0.78 | 0.61 | 0.22 | 0.04 |
| ⁶⁸Ga-JHDC16 | 0.62 | 1.59 | 1.09 | 0.58 | 1.21 | 1.23 | 0.79 | 0.14 |
| ⁶⁸Ga-JHDC06 | 0.51 | 1.46 | 1.33 | 1.17 | 1.10 | 1.16 | 0.85 | 0.66 |

**Table 9: Distribution changes of ⁶⁸Ga-labeled compounds in mouse tumor over time**

| Compound No. | 5 minutes | 10 minutes | 15 minutes | 20 minutes | 25 minutes | 30 minutes | 1.5 hours | 3 hours |
|---|---|---|---|---|---|---|---|---|
| ⁶⁸Ga-FAP-2286 | 1.34 | 3.39 | 4.51 | 5.36 | 5.99 | 6.36 | 9.20 | 8.60 |
| ⁶⁸Ga-JHDC16 | 2.48 | 6.60 | 7.67 | 8.05 | 8.08 | 8.50 | 7.90 | 7.30 |
| ⁶⁸Ga-JHDC06 | 0.74 | 2.68 | 3.37 | 3.72 | 3.93 | 4.14 | 5.90 | 7.00 |

### Example 11: therapeutic results of ¹⁷⁷Lu-labeled compound in tumor-bearing mice

6- to 8-week-old (Balb/c Nude) mice were subcutaneously inoculated on the right scapula with 2×10⁶ cells of HT1080 2# (50% Matrigel, Corning). When the tumors grew to the size as required by the assay, the animals were randomly assigned to 13 experimental groups based on tumor volume, with 5 animals per group. The body weight and tumor size were measured. On the day of grouping, administration was initiated for the control group (normal saline) and groups 1-12. General health and appearance were observed daily after the experiment began. Body weight and tumor size were measured prior to each sample collection time point. Any abnormal observations found during the entire study period were required to be recorded in the raw data.

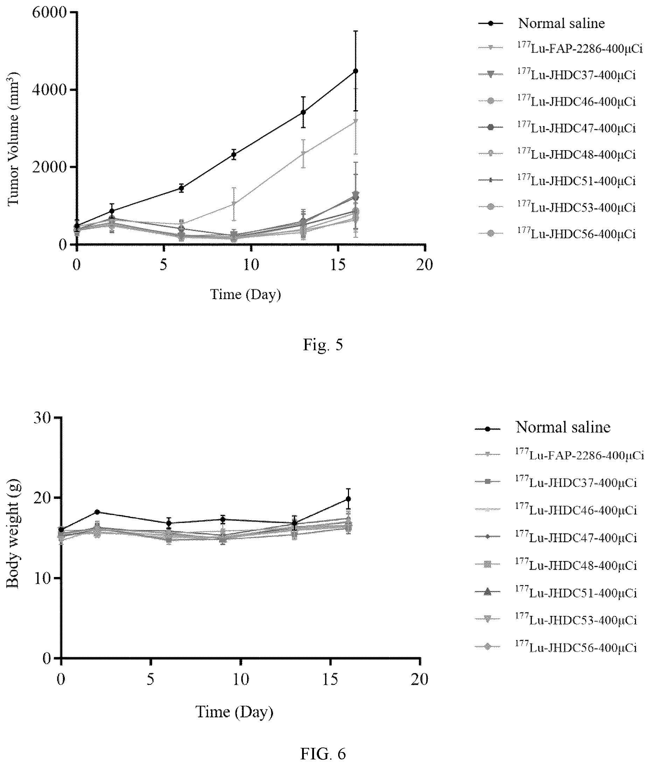

The experimental results were shown in Tables 10 and 11, and FIGs. 5 and 6.

**Table 10: Changes in animal tumors over time after injection of ¹⁷⁷Lu-labeled compound**

| Administered compound No. | Tumor volume (mm³) | | | | | |
|---|---|---|---|---|---|---|
| | Day 0 | Day 2 | Day 6 | Day 9 | Day 13 | Day 16 |
| Normal saline | 487.53±124.06 | 867.15±149.59 | 1,456.69±87.56 | 2,325.07±110.86 | 3,422.98±323.59 | 4,487.42±842.22 |
| ¹⁷⁷Lu-FAP-2286 | 466.80±118.37 | 630.23±96.79 | 525.29±100.39 | 1,041.55±348.07 | 2,345.02±298.66 | 3,180.37±697.35 |
| ¹⁷⁷Lu-JHDC37 | 405.37±75.52 | 560.56±63.81 | 222.90±33.45 | 258.63±109.81 | 553.91±289.85 | 1,273.95±697.35 |
| ¹⁷⁷Lu-JHDC46 | 400.31±78.63 | 483.08±95.00 | 203.26±49.63 | 168.29±33.83 | 313.94±119.95 | 686.05±297.66 |
| ¹⁷⁷Lu-JHDC47 | 393.07±85.34 | 681.73±164.08 | 412.83±163.68 | 231.93±66.97 | 603.69±200.80 | 1,217.7±1484.39 |
| ¹⁷⁷Lu-JHDC48 | 388.04±95.13 | 510.10±138.13 | 181.69±63.38 | 190.03±78.60 | 368.56±196.92 | 631.39±362.81 |
| ¹⁷⁷Lu-JHDC51 | 376.25±100.20 | 493.55±140.08 | 196.06±80.88 | 198.76±85.86 | 510.78±224.50 | 862.07±368.51 |
| ¹⁷⁷Lu-JHDC53 | 365.39±106.36 | 523.47±148.96 | 251.92±78.48 | 152.08±60.80 | 500.37±234.98 | 879.33±403.99 |
| ¹⁷⁷Lu-JHDC56 | 361.81±112.47 | 522.71±177.91 | 192.24±79.30 | 143.80±58.74 | 393.23±168.05 | 814.66±344.00 |

**Table 11: Changes in animal body weight over time after injection of ¹⁷⁷Lu-labeled compound**

| Administered compound No. | Animal body weight (g) | | | | | |
|---|---|---|---|---|---|---|
| | Day 0 | Day 2 | Day 6 | Day 9 | Day 13 | Day 16 |
| Normal saline | 16.03±0.19 | 18.23±0.20 | 16.83±0.55 | 17.30±0.41 | 16.87±0.72 | 19.87±1.03 |
| ¹⁷⁷Lu-FAP-2286 | 14.67±0.37 | 16.10±0.85 | 15.60±0.49 | 15.87±0.68 | 16.23±0.92 | 17.07±1.02 |
| ¹⁷⁷Lu-JHDC37 | 15.57±0.29 | 16.23±0.23 | 14.77±0.24 | 14.80±0.28 | 15.40±0.46 | 16.23±0.57 |
| ¹⁷⁷Lu-JHDC46 | 15.40±0.36 | 16.17±0.69 | 14.67±0.38 | 15.10±0.41 | 16.00±0.69 | 16.43±0.52 |
| ¹⁷⁷Lu-JHDC47 | 15.20±0.38 | 16.00±0.54 | 15.87±0.33 | 15.33±0.41 | 16.73±0.40 | 17.43±0.48 |
| ¹⁷⁷Lu-JHDC48 | 15.40±0.56 | 15.60±0.45 | 15.40±0.08 | 15.00±0.41 | 15.97±0.50 | 16.40±0.46 |
| ¹⁷⁷Lu-JHDC51 | 15.43±0.41 | 16.30±0.38 | 15.57±0.28 | 14.90±0.59 | 16.30±0.45 | 16.97±0.35 |
| ¹⁷⁷Lu-JHDC53 | 15.93±0.40 | 16.00±0.25 | 15.23±0.35 | 15.03±0.22 | 16.40±0.12 | 16.57±0.12 |
| ¹⁷⁷Lu-JHDC56 | 15.23±0.20 | 15.75±0.10 | 15.00±0.34 | 15.17±0.50 | 16.20±0.33 | 16.57±0.44 |

Experimental results showed that after the synthesized ¹⁷⁷Lu-labeled compound was administered to tumor-bearing mice via tail vein injection, it exhibited time-dependent inhibitory effects on tumor growth. Compared with the reference compound at equivalent doses, the synthesized compound demonstrated superior efficacy. Moreover, at equivalent doses, the body weight of the animals did not change significantly.

Although specific embodiments of the present disclosure have been described above, it should be understood by those skilled in the art that these are merely illustrative examples, and various changes or modifications may be made to these embodiments without departing from the principles and essence of the present disclosure. Therefore, the scope of the present disclosure is defined by the appended claims.

## Claims

1. A compound X or a pharmaceutically acceptable salt thereof, 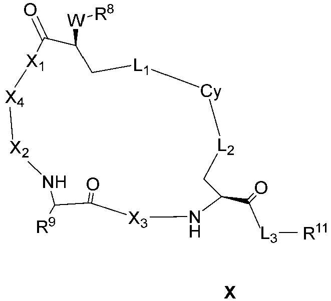
wherein -X₁- is 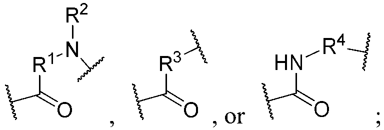
R¹ is C₁-C₄ alkylene, C₃-C₆ cycloalkylene, C₁-C₄ alkylene-C₃-C₆ cycloalkylene, or C₁-C₄ alkylene-C₃-C₆ heterocycloalkylene, wherein the heteroatom of the 3- to 6-membered heterocycloalkylene is selected from one or more of N, O, and S, the number of heteroatoms is 1, 2, or 3, and at least one N is included;
R² is hydrogen or C₁-C₄ alkyl;
R³ is C₁-C₄ alkylene, C₁-C₄ alkylene-3- to 6-membered heterocycloalkylene, 3- to 6-membered heterocycloalkylene, or 6- to 12-membered heteroarylene; the heteroatom of the 3-to 6-membered heterocycloalkylene is selected from one or more of N, O, and S, the number of heteroatoms is 1, 2, or 3, and at least one N is included; the heteroatom of the 6- to 12-membered heteroarylene is selected from one or more of N, O, and S, the number of heteroatoms is 1, 2, or 3, and at least one N is included;
R⁴ is 3- to 6-membered heterocycloalkylene; the heteroatom of the 3- to 6-membered heterocycloalkylene is selected from one or more of N, O, and S, the number of heteroatoms is 1, 2, or 3, and at least one N is included;
-X₂- is -X₂₋₁-Y₁-; -X₂₋₁- is a chemical bond, non-natural amino acid residue, or natural amino acid residue; Y₁ is 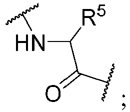
R⁵ is each independently C₁-C₄ alkylene or C₁-C₄ alkylene substituted by 1, 2, or 3 R⁵⁻¹;
R⁵⁻¹ is each independently halogen, hydroxyl, guanidino, carboxyl, or amido;
-X₃- is -X₃₋₁-Y₂-; -X₃₋₁- is a chemical bond, non-natural amino acid residue, or natural amino acid residue; Y₂ is 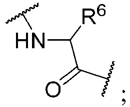
R⁶ is C₁-C₄ alkyl, C₁-C₄ alkyl substituted by 1 or 2 R⁶⁻¹, or C₃-C₆ cycloalkyl; each R⁶⁻¹ is independently halogen, C₃-C₆ cycloalkyl, C₆-C₁₀ aryl, or C₆-C₁₀ aryl substituted by one or more R⁶⁻¹⁻¹;
R⁶⁻¹⁻¹ is each independently halogen;
-X₄- is 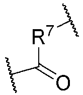 ; R⁷ is 3- to 6-membered heterocycloalkylene; the heteroatom of the 3- to 6-membered heterocycloalkylene is selected from one or more of N, O, and S, the number of heteroatoms is 1, 2, or 3, and at least one N is included;
W is -NH-CO- or -NH-CO-NH-;
R⁸ is linear C₁₋₁₀ alkyl;
R⁹ is H or C₁-C₄ alkyl substituted by 1 or 2 R⁹⁻¹; R⁹⁻¹ is carboxyl or amido;
L₁ and L₂ are each independently a chemical bond, C₁-C₄ alkylene, or -S-(CH₂)ₖ-; k is 0, 1, 2, 3, or 4;
Cy is a chemical bond, 3- to 6-membered heterocycloalkylene, 5- to 6-membered heteroarylene, C₆-C₁₀ arylene, or C₆-C₁₀ arylene-L₄-R¹²; the heteroatom of the 3- to 6-membered heterocycloalkylene is selected from one or more of N, O, and S, the number of heteroatoms is 1, 2, or 3, and at least one N is included; in the 5- to 6-membered heteroarylene, the heteroatom is selected from one or more of N, O, and S, the number of heteroatoms is 1, 2, or 3, and at least one N is included;
-L₃- is -L₃₋₁-L₃₋₂-L₃₋₃-;
-L₃₋₁ is a chemical bond, 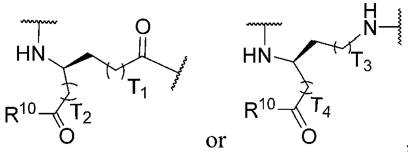 , wherein T₁ and T₂ are each independently 0, 1, 2, 3, or 4, and T₃ and T₄ are each independently 0, 1, 2, 3, or 4;
-L₃₋₂- is -X₅-Y₃-; -X₅- is a chemical bond, a natural amino acid residue, or a polypeptide formed by 2-6 natural amino acids; -Y₃- is a chemical bond, 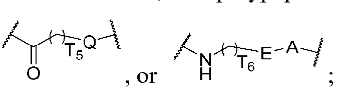 T₅ and T₆ are each independently 0, 1, 2, 3, or 4; E and Q are each independently C₁₋₆ alkylene or -(OCH₂CH₂)_{j'}-, and j' is 1, 2, 3, 4, 5, or 6; A is a chemical bond or -NH-;
-L₃₋₃- is a chemical bond, -NH-, -carbonyl-6- to 12-membered heteroarylene-, -carbonyl-C₁₋₆ alkylene-3- to 6-membered heterocycloalkylene-, -NH-C₁₋₆ alkylene-3- to 6-membered heterocycloalkylene-, or 3- to 6-membered heterocycloalkylene; the heteroatom of the 3- to 6-membered heterocycloalkylene is selected from one or more of N, O, and S, the number of heteroatoms is 1, 2, or 3, and at least one N is included; the heteroatom of the 6- to 12-membered heteroarylene is selected from one or more of N, O, and S, the number of heteroatoms is 1, 2, or 3, and at least one N is included;
R¹⁰ is hydroxyl or amino;
-L₄- is 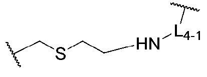 ; L₄₋₁ is a chemical bond, 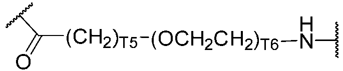 or 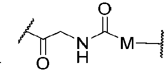 , T₅ and T₆ are each independently a natural number from 0 to 10;
M is 6- to 12-membered heteroarylene; the heteroatom of the 6- to 12-membered heteroarylene is selected from one or more of N, O, and S, the number of heteroatoms is 1, 2, or 3, and at least one N is included;
R¹¹ and R¹² are each independently H or a chelating group; and when R¹¹ is H, R¹² is a chelating group, or when R¹² is H R¹¹ is a chelating group;
the chelating group is 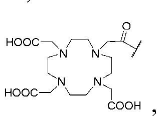
NOTA, HBED-CC, NODAGA, NOTAGA, DOTAGA, TRAP, NOPO, PCTA, DFO, DTPA, CHX-DTPA, AAZTA, or DEDPA;
the compound X satisfies one or more of the following conditions:
(1) -X₁- is 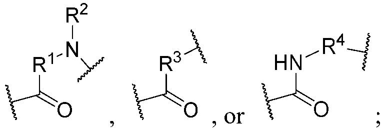
R¹ is C₁-C₄ alkylene, C₃-C₆ cycloalkylene, C₁-C₄ alkylene-C₃-C₆ cycloalkylene, or C₁-C₄ alkylene-C₃-C₆ heterocycloalkylene;
R² is hydrogen or C₁-C₄ alkyl;
R³ is C₁-C₄ alkylene, C₁-C₄ alkylene-3- to 6-membered heterocycloalkylene, 4-membered or 6-membered heterocycloalkylene, or 6- to 12-membered heteroarylene; the heteroatom of the 3- to 6-membered heterocycloalkylene is selected from one or more of N, O, and S, the number of heteroatoms is 1, 2, or 3, and at least one N is included; the heteroatom of the 4-membered or 6-membered heterocycloalkylene is selected from one or more of N, O, and S, the number of heteroatoms is 1, 2, or 3, and at least one N is included; the heteroatom of the 6-to 12-membered heteroarylene is selected from one or more of N, O, and S, the number of heteroatoms is 1, 2, or 3, and at least one N is included;
R⁴ is 3- to 6-membered heterocycloalkylene; the heteroatom of the 3- to 6-membered heterocycloalkylene is selected from one or more of N, O, and S, the number of heteroatoms is 1, 2, or 3, and at least one N is included;
(2) -X₂₋₁- is a natural amino acid or a non-natural amino acid;
(3) -X₃₋₁- is a natural amino acid or a non-natural amino acid, or R⁶ is C₁-C₄ alkyl, C₁-C₄ alkyl substituted by 1 or 2 R⁶⁻¹, or C₃-C₆ cycloalkyl; each R⁶⁻¹ is independently halogen, C₃-C₆ cycloalkyl, or C₆-C₁₀ aryl substituted by one or more R⁶⁻¹⁻¹;
(4) -X₄- is 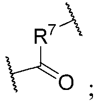 R⁷ is 4-membered heterocycloalkylene; the heteroatom of the 4-membered heterocycloalkylene is selected from one or more of N, O, and S, the number of heteroatoms is 1, 2, or 3, and at least one N is included;
(5) L₄₋₁ is

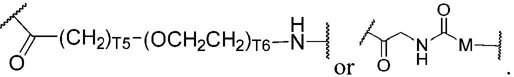

2. The compound X or the pharmaceutically acceptable salt thereof according to claim 1, wherein the compound X satisfies one or more of the following conditions:
(1) in R¹, the C₁-C₄ alkylene from the C₁-C₄ alkylene, the C₁-C₄ alkylene-C₃-C₆ cycloalkylene, and the C₁-C₄ alkylene-C₃-C₆ heterocycloalkylene is each independently methylene, ethylene, n-propylene, or isopropylene, for example methylene;
(2) in R¹, the C₃-C₆ cycloalkylene from the C₁-C₄ alkylene-C₃-C₆ cycloalkylene is cyclobutylene or cyclopropylene, for example, the cyclobutylene is 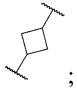
(3) in R¹, the C₃-C₆ heterocycloalkylene from the C₁-C₄ alkylene-C₃-C₆ heterocycloalkylene is C₄-C₆ heterocycloalkylene, where in the C₃-C₆ heterocycloalkylene, the heteroatom is preferably N, and the number of heteroatoms is preferably 1, for example, the C₃-C₆ heterocycloalkylene is piperidinylene, and the piperidinylene is for example 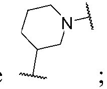
(4) in R², the C₁-C₄ alkyl is methyl, ethyl, n-propyl, or isopropyl;
(5) in R³, the C₁-C₄ alkylene from the C₁-C₄ alkylene and the C₁-C₄ alkylene-3- to 6-membered heterocycloalkylene is each independently methylene, ethylene, n-propylene, or isopropylene;
(6) in R³, the 3- to 6-membered heterocycloalkylene from the C₁-C₄ alkylene-3- to 6-membered heterocycloalkylene and the 3- to 6-membered heterocycloalkylene is each independently 4- to 6-membered heterocycloalkylene, where in the 3- to 6-membered heterocycloalkylene, the heteroatom is preferably N, and the number of heteroatoms is preferably 1, for example, the 3- to 6-membered heterocycloalkylene is azetidinylene, and the azetidinylene is for example 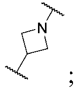
(7) in R³, the 6- to 12-membered heteroarylene is 6- to 10-membered heteroarylene, where in the 6- to 12-membered heteroarylene, the heteroatom is preferably N, and the number of heteroatoms is preferably 1;
(8) in R⁴, the 3- to 6-membered heterocycloalkylene is 4- to 6-membered heterocycloalkylene, where in the 3- to 6-membered heterocycloalkylene, the heteroatom is preferably N, and the number of heteroatoms is preferably 1, for example, the 3- to 6-membered heterocycloalkylene is piperidinylene, and the piperidinylene is for example 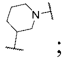
(9) in -X₂₋₁-, the non-natural amino acid residue is D-alaninyl or 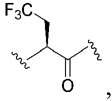 wherein the D-alanyl is for example 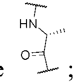
(10) in -X₂₋₁-, the natural amino acid residue is glycinyl or L-alaninyl, wherein the glycinyl is for example 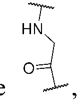 and the L-alaninyl is for example 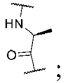
(11) in R⁵, the C₁-C₄ alkylene from the C₁-C₄ alkylene and the C₁-C₄ alkylene substituted by 1, 2, or 3 R⁵⁻¹ is each independently methylene, ethylene, n-propylene, or isopropylene, for example ethylene or n-propylene;
(12) in -X₃₋₁-, the non-natural amino acid residue is 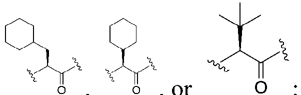
(13) in -X₃₋₁-, the natural amino acid residue is glycyl, 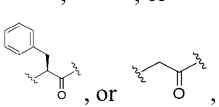 for example, the natural amino acid residue is 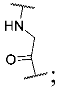
(14) in R⁶, the C₁-C₄ alkylene from the C₁-C₄ alkylene and the C₁-C₄ alkylene substituted by 1 or 2 R⁶⁻¹ is each independently methylene, ethylene, n-propylene, or isopropylene, for example methylene;
(15) in R⁶, the C₃-C₆ cycloalkyl is cyclobutyl, cyclopentyl, or cyclohexyl;
(16) in R⁶⁻¹, the C₃-C₆ cycloalkyl is cyclobutyl, cyclopentyl, or cyclohexyl, for example cyclohexyl, and the cyclohexyl is for example 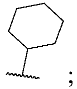
(17) in R⁶⁻¹, the C₆-C₁₀ aryl from the C₆-C₁₀ aryl and the C₆-C₁₀ aryl substituted by one or more R⁶⁻¹⁻¹ is each independently phenyl or naphthyl, for example phenyl;
(18) in R⁶⁻¹⁻¹, the halogen is fluorine, chlorine, or bromine;
(19) in R⁷, the 3- to 6-membered heterocycloalkylene is 4- to 6-membered heterocycloalkylene, where in the 3- to 6-membered heterocycloalkylene, the heteroatom is preferably N, and the number of heteroatoms is preferably 1, for example, the 3- to 6-membered heterocycloalkylene is pyrrolidinylene, and the pyrrolidinylene is for example 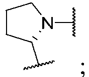
(20) in R⁸, the C₁₋₁₀ alkyl is methyl, ethyl, n-propyl, n-butyl, n-pentyl, n-hexyl, n-heptyl, n-octyl, or n-nonyl, for example methyl, n-pentyl, n-nonyl, n-heptyl, or n-butyl;
(21) in R⁹, the C₁-C₄ alkyl is methyl, ethyl, n-propyl, or isopropyl, for example ethyl, and the ethyl is for example 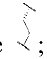
(22) in L₁ and L₂, the C₁-C₄ alkylene is methylene, ethylene, n-propylene, or isopropylene, for example n-propylene;
(23) in Cy, the 3- to 6-membered heterocycloalkylene is 5- to 6-membered heterocycloalkylene, where in the 3- to 6-membered heterocycloalkylene, the heteroatom is preferably N, and the number of heteroatoms is preferably 3;
(24) in Cy, the heteroatom in the 5- to 6-membered heteroarylene is N, and the number of heteroatoms is preferably 1 or 3, for example, the 5- to 6-membered heteroarylene is pyridyl or 1H-1,2,3-triazole, and the pyridyl is for example 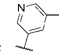 , and the 1H-1,2,3-triazole is for example 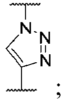
(25) in Cy, the C₆-C₁₀ aryl from the C₆-C₁₀ arylene or C₆-C₁₀ arylene-L₄-R¹² is each independently phenyl or naphthyl, for example phenyl, and the phenyl is for example 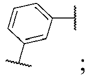
(26) in L₃₋₂, the natural amino acid residue from the natural amino acid residue or the polypeptide formed by 2-6 natural amino acids is selected from one or more of glutamic acid, arginine, glycine, and aspartic acid; preferably, the polypeptide is formed by 3 natural amino acids; more preferably, the polypeptide is 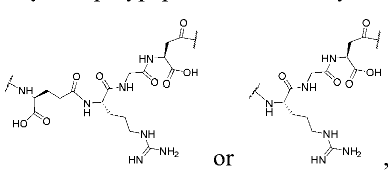 for example 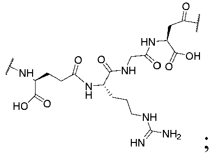
(27) in Y₃, the C₁₋₆ alkylene is linear C₁₋₆ alkylene; preferably, the C₁₋₆ alkylene is methyl, ethyl, n-propyl, n-butyl, n-pentyl, or n-hexyl, for example methyl, ethyl, or n-propyl;
(28) in L₃₋₃, the 6- to 12-membered heteroarylene is 12-membered heteroarylene, where in the 6- to 12-membered heteroarylene, the heteroatom is preferably N, and the number of heteroatoms is preferably 1, for example, the 6- to 12-membered heteroarylene is 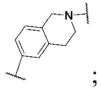
(29) in L₃₋₃, the 3- to 6-membered heterocycloalkylene from the carbonyl-C₁₋₆ alkylene-3-to 6-membered heterocycloalkylene, NH-C₁₋₆alkylene-3- to 6-membered heterocycloalkylene, and 3- to 6-membered heterocycloalkylene is each independently 4- to 6-membered heterocycloalkylene, the heteroatom is preferably N, and the number of heteroatoms is preferably 1 or 2, for example, the 3- to 6-membered heterocycloalkylene is piperidinylene, and the piperidinylene is for example 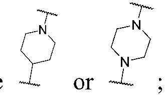
(30) in L₃₋₃, the C₁₋₆ alkylene from the carbonyl-C₁₋₆ alkylene-3- to 6-membered heterocycloalkylene and NH-C₁₋₆ alkylene-3- to 6-membered heterocycloalkylene is each independently methyl, ethyl, n-propyl, n-butyl, n-pentyl, or n-hexyl, for example methyl, ethyl, or n-propyl;
(31) in L₃₋₁₋, 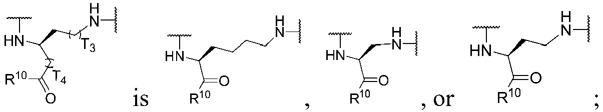
(32) in -L₃₋₁₋,

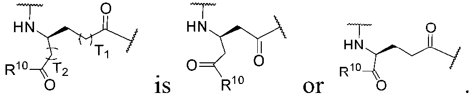

3. The compound X or the pharmaceutically acceptable salt thereof according to claim 1, wherein the compound X satisfies one or more of the following conditions:
(1) -X₁- is 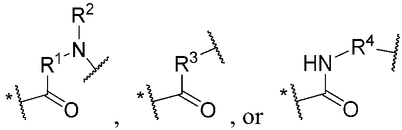 , wherein 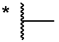 is connected to X₄;
(2) -X₂- is -X₂₋₁-Y₁-, wherein X₂₋₁ is connected to X₄;
(3) -X₃- is -X₃₋₁-Y₂-, wherein X₃₋₁ is connected to -NH- or carbonyl;
(4) -X₄- is 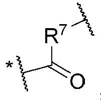 , wherein 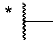 is connected to X₂;
(5) W is -NH-CO-* or -NH-CO-NH-*, wherein -* is connected to R⁸;
(6) L₁ and L₂ are each independently a chemical bond, C₁-C₄ alkylene, or -S-(CH₂)ₖ-*, wherein -* is connected to Cy;
(7) -L₃- is -L₃₋₁-L₃₋₂-L₃₋₃-*, wherein -* is connected to R¹¹;
(8) -L₃₋₁- is a chemical bond, 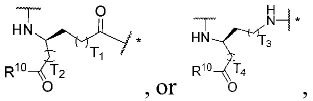 , wherein 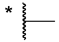 is connected to -L₃₋₂-;
(9) -L₃₋₂- is *-X₅-Y₃-, wherein *- is connected to -L₃₋₁-;
(10) -L₃₋₃- is a chemical bond, -NH-, -carbonyl-6- to 12-membered heteroarylene-*, - carbonyl-C₁₋₆ alkylene-3- to 6-membered heterocycloalkylene-*, -NH-C₁₋₆ alkylene-3- to 6-membered heterocycloalkylene-*, or 3- to 6-membered heterocycloalkylene, wherein -* is connected to R¹¹;
(11) -L₄- is 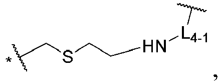 wherein 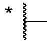 is connected to Cy.

4. The compound X or the pharmaceutically acceptable salt thereof according to claim 1, wherein the compound X satisfies one or more of the following conditions:
(1) -X₁- is 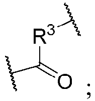
(2) R¹ is C₃-C₆ cycloalkylene or C₁-C₄ alkylene-C₃-C₆ heterocycloalkylene, wherein the heteroatom of the 3- to 6-membered heterocycloalkylene is selected from one or more of N, O, and S, the number of heteroatoms is 1, 2, or 3, and at least one N is included;
(3) R² is hydrogen;
(4) R³ is 3- to 6-membered heterocycloalkylene; the heteroatom of the 3- to 6-membered heterocycloalkylene is selected from one or more of N, O, and S, the number of heteroatoms is 1, 2, or 3, and at least one N is included;
(5) -X₂₋₁- is a chemical bond, a non-natural amino acid residue, or a natural amino acid residue, wherein the unnatural amino acid residue is 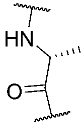 , and the natural amino acid residue is 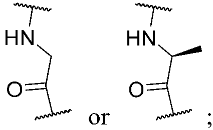 for example, -X₂₋₁- is a chemical bond;
(6) R⁵ is each independently C₁-C₄ alkylene substituted by 1, 2, or 3 R⁵⁻¹;
(7) R⁵⁻¹ is each independently halogen, hydroxyl, guanidino, or amido, for example, hydroxyl, guanidino, or amido, or for example hydroxyl;
(8) -X₃₋₁- is a chemical bond or a natural amino acid residue, wherein the natural amino acid residue is for example 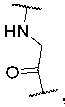 and for example, -X₃₋₁- is a chemical bond;
(9) R⁶ is C₁-C₄ alkyl substituted by 1 or 2 R⁶⁻¹;
(10) R⁶⁻¹ is each independently halogen, C₃-C₆ cycloalkyl, or C₆-C₁₀ aryl, for example C₃-C₆ cycloalkyl or C₆-C₁₀ aryl;
(11) R⁹ is C₁-C₄ alkyl substituted by 1 or 2 R⁹⁻¹; R⁹⁻¹ is amido;
(12) k is 0 or 1, for example 1;
(13) Cy is a chemical bond, 5- to 6-membered heteroarylene, C₆-C₁₀ arylene, or C₆-C₁₀ arylene-L₄-R¹², where in the 5- to 6-membered heteroarylene, the heteroatom is selected from one or more of N, O, and S, the number of heteroatoms is 1, 2, or 3, and at least one N is included; for example, Cy is C₆-C₁₀ arylene;
(14) T₁ and T₂ are each independently 0 or 1;
(15) T₃ and T₄ are each independently 0, 1, or 3, for example 0 or 1;
(16) -L₃₋₂- is -X₅-Y₃-; -X₅- is a chemical bond or a polypeptide formed by 2-6 natural amino acids, and -Y₃- is a chemical bond, 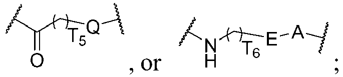 for example, -X₅-is a chemical bond and -Y₃- is 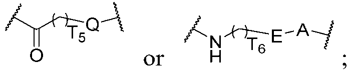 alternatively, -X₅- is a polypeptide formed by 2-6 natural amino acids and -Y₃- is a chemical bond or 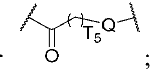 as another example, -X₅- is a chemical bond and -Y₃- is a chemical bond, 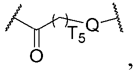 or 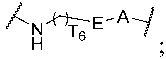 A is a chemical bond or -NH-;
(17) E and Q are each independently C₁₋₆ alkylene;
(18) T₅ and T₆ are each independently 0, 1, 2, or 4, for example 0, 1 or 2;
(19) j' is 1, 2, or 3;
(20) R¹⁰ is hydroxyl;
(21) -L₄- is 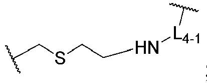 ; L₄₋₁ is a chemical bond;
(22) the chelating group is 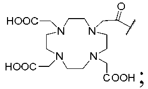
(23) R¹¹ is a chelating group; the chelating group is 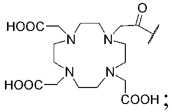
(24) the compound X satisfies one or more of the following conditions:
(1) -X₁- is 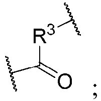 R³ is 4-membered or 6-membered heterocycloalkylene; the heteroatom of the 4- to 6-membered heterocycloalkylene is selected from one or more of N, O, and S, the number of heteroatoms is 1, 2, or 3, and at least one N is included;
(2) R⁶ is C₁-C₄ alkyl substituted by 1 or 2 R⁶⁻¹; each R⁶⁻¹ is independently C₃-C₆ cycloalkyl.

5. The compound X or the pharmaceutically acceptable salt thereof according to claim 1, wherein the compound X satisfies one or more of the following conditions:
(1) -X₂₋₁- is -X₂₋₁-Y₁-; -X₂₋₁- is a chemical bond and Y₁ is 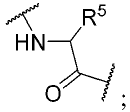
(2) -X₃- is -X₃₋₁-Y₂-; -X₃₋₁- is a chemical bond and Y₂ is 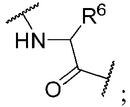
preferably, -L₃₋₁ is 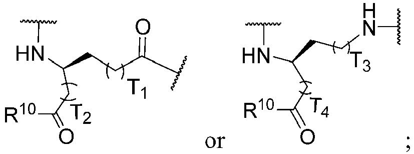
for example, -L₃₋₁ is 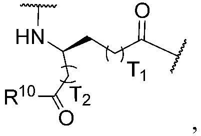 wherein T₁ and T₂ are each independently 0, 1, 2, 3, or 4;
-L₃₋₂- is -X₅-Y₃-; -X₅- is a chemical bond and -Y₃- is 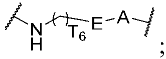 T₆ is 0, 1, 2, 3, or 4, and E is C₁₋₆ alkylene or -(OCH₂CH₂)_{j'}-, wherein j' is 1, 2, 3, 4, 5, or 6, for example j' is 1; A is a chemical bond or -NH-;
-L₃₋₃- is a chemical bond;
for example, -L₃₋₁ is 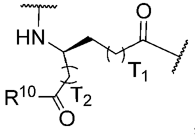
, wherein T₁ and T₂ are each independently 0, 1, 2, 3, or 4;
-L₃₋₂- is -X₅-Y₃-; -X₅- is a chemical bond and -Y₃- is a chemical bond;
-L₃₋₃- is -NH-C₁₋₆ alkylene-3- to 6-membered heterocycloalkylene- or 3- to 6-membered heterocycloalkylene, wherein the heteroatom of the 3- to 6-membered heterocycloalkylene is selected from one or more of N, O, and S, the number of heteroatoms is 1, 2, or 3, and at least one N is included;
for example, -L₃₋₁ is 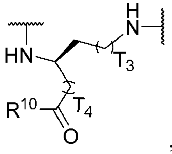 , wherein T₃ and T₄ are each independently 0, 1, 2, 3, or 4;
-L₃₋₂- is -X₅-Y₃-; -X₅- is a chemical bond and -Y₃- is 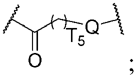 T₅ is 0, 1, 2, 3, or 4, and Q is C₁₋₆ alkylene or -(OCH₂CH₂)_{j'}-, wherein j' is 1, 2, 3, 4, 5, or 6;
-L₃₋₃- is -NH-;
for example, -L₃₋₁ is 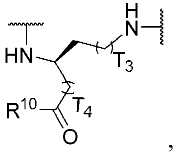 wherein T₃ and T₄ are each independently 0, 1, 2, 3, or 4;
-L₃₋₂- is -X₅-Y₃-; -X₅- is a chemical bond and -Y₃- is a chemical bond;
-L₃₋₃- is -carbonyl-6- to 12-membered heteroarylene- or -carbonyl-C₁₋₆ alkylene-3- to 6-membered heterocycloalkylene-, wherein the heteroatom of the 3- to 6-membered heterocycloalkylene is selected from one or more of N, O, and S, the number of heteroatoms is 1, 2, or 3, and at least one N is included; the heteroatom of the 6- to 12-membered heteroarylene is selected from one or more of N, O, and S, the number of heteroatoms is 1, 2, or 3, and at least one N is included;
for example, -L₃₋₁ is 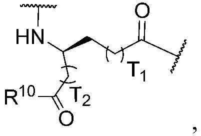 wherein T₁ and T₂ are each independently 0, 1, 2, 3, or 4;
-L₃₋₂- is -X₅-Y₃-; -X₅- is a natural amino acid residue or a polypeptide formed by 2-6 natural amino acids, and -Y₃- is a chemical bond;
-L₃₋₃- is a chemical bond;
(3) R⁹ is C₁-C₄ alkyl substituted by 1 R⁹⁻¹; R⁹⁻¹ is amido;
(4) L₁ and L₂ are -S-(CH₂)ₖ-; k is 1;
(5) Cy is a chemical bond, 3- to 6-membered heterocycloalkylene, 5- to 6-membered heteroarylene, or C₆-C₁₀ arylene; the heteroatom of the 3- to 6-membered heterocycloalkylene is selected from one or more of N, O, and S, the number of heteroatoms is 1, 2, or 3, and at least one N is included; in the 5- to 6-membered heteroarylene, the heteroatom is selected from one or more of N, O, and S, the number of heteroatoms is 1, 2, or 3, and at least one N is included;
R¹¹ is a chelating group;
preferably, Cy is C₆-C₁₀ arylene; R¹¹ is a chelating group; for example, in Cy, the C₆-C₁₀ arylene is 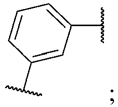
(6) -L₃₋₂- is -X₅-Y₃-; -X₅- is a chemical bond and -Y₃- is a chemical bond, 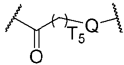 or 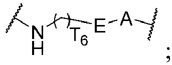 T₅ and T₆ are each independently 0, 1, 2, or 4, and E and Q are each independently C₁₋₆ alkylene; A is a chemical bond or -NH-;
(7) -L₃₋₃- is a chemical bond, -NH-, -carbonyl-C₁₋₆ alkylene-3- to 6-membered heterocycloalkylene-, -NH-C₁₋₆ alkylene-3- to 6-membered heterocycloalkylene-; for example -L₃₋₃- is -NH-, -carbonyl-C₁₋₆ alkylene-3- to 6-membered heterocycloalkylene-, -NH-C₁₋₆ alkylene-3- to 6-membered heterocycloalkylene-; the heteroatom of the 3- to 6-membered heterocycloalkylene is selected from one or more of N, O, and S, the number of heteroatoms is 1, 2, or 3, and at least one N is included.

6. The compound X or a pharmaceutically acceptable salt thereof according to claim 1, wherein the compound X is compound X-1, compound X-2, compound X-3, compound X-4, or compound X-35, 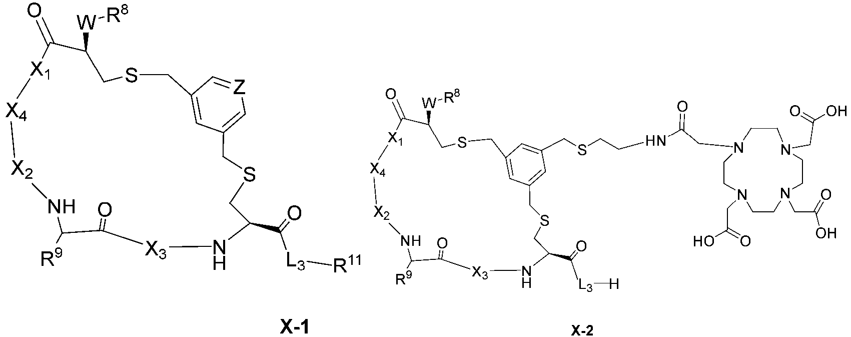 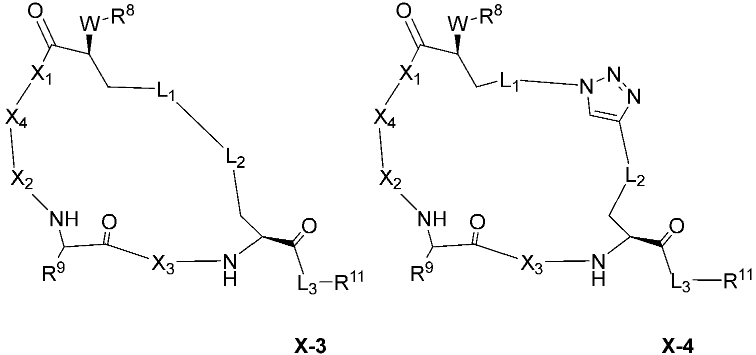 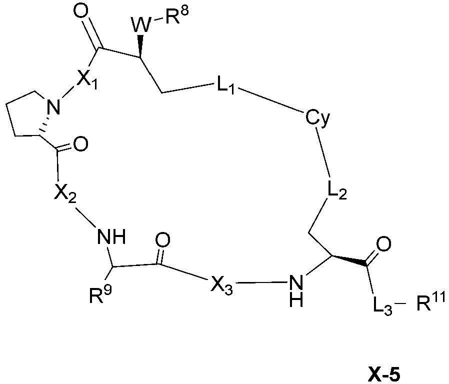
Z is N or CH;
X₁, X₂, X₃, X₄, R⁹, R⁸, L₁, W, L₂, L₃, and R¹¹ are as defined in claim 1.

7. The compound X or a pharmaceutically acceptable salt thereof according to claim 1, wherein the compound X is compound X-6, compound X-7, compound X-8, or compound X-9, 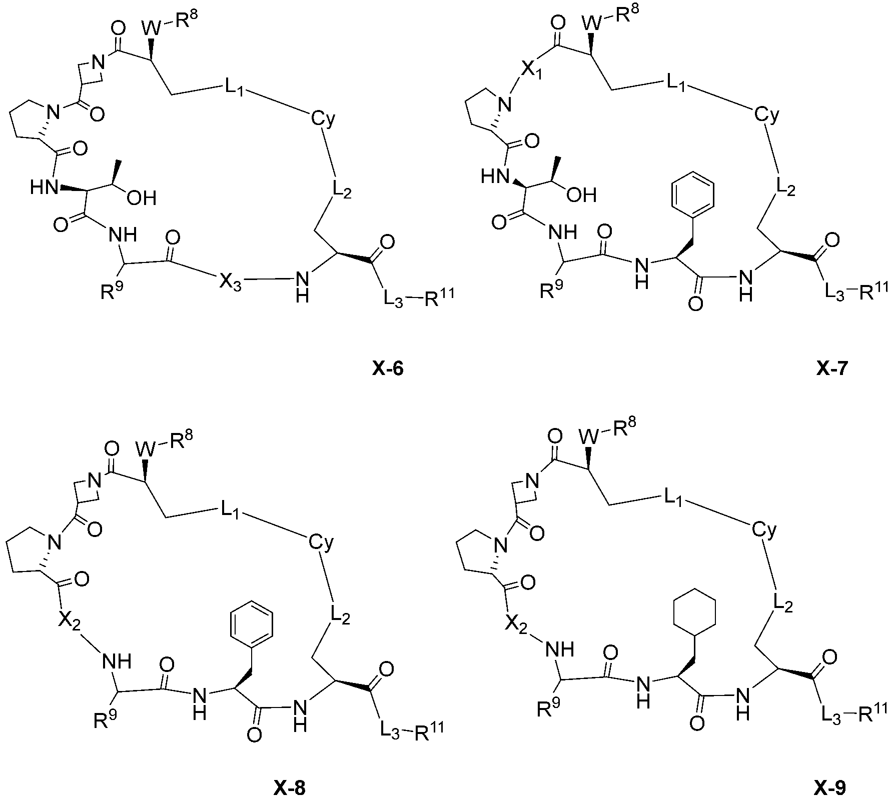 wherein X₁, X₂, X₃, R⁹, R⁸, W, L₁, L₂, Cy, L₃, and R¹¹ are as defined in claim 1.

8. The compound X or the pharmaceutically acceptable salt thereof according to claim 1, wherein the compound X follows one of the following schemes:
scheme 1: in the compound X,
-X₁- is 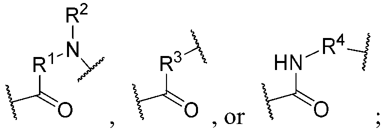
R¹ is C₃-C₆ cycloalkylene or C₁-C₄ alkylene-C₃-C₆ heterocycloalkylene, wherein the heteroatom of the 3- to 6-membered heterocycloalkylene is selected from one or more of N, O, and S, the number of heteroatoms is 1, 2, or 3, and at least one N is included;
R² is hydrogen;
R³ is 3- to 6-membered heterocycloalkylene; the heteroatom of the 3- to 6-membered heterocycloalkylene is selected from one or more of N, O, and S, the number of heteroatoms is 1, 2, or 3, and at least one N is included;
R⁴ is 3- to 6-membered heterocycloalkylene; the heteroatom of the 3- to 6-membered heterocycloalkylene is selected from one or more of N, O, and S, the number of heteroatoms is 1, 2, or 3, and at least one N is included;
-X₂- is -X₂₋₁-Y₁-; -X₂₋₁- is a chemical bond, non-natural amino acid residue, or natural amino acid residue,
the non-natural amino acid residue is 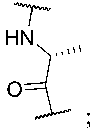 the natural amino acid residue is 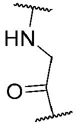 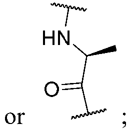 Y₁ is 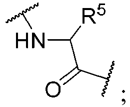
R⁵ is each independently C₁-C₄ alkylene substituted by 1, 2, or 3 R⁵⁻¹;
R⁵⁻¹ is each independently hydroxyl, guanidino, or amido;
-X₃- is -X₃₋₁-Y₂-; -X₃₋₁- is a chemical bond or a natural amino acid residue,
the natural amino acid residue is 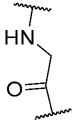 ; Y₂ is 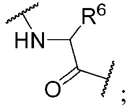
R⁶ is C₁-C₄ alkyl substituted by 1 or 2 R⁶⁻¹; each R⁶⁻¹ is independently halogen, C₃-C₆ cycloalkyl, or C₆-C₁₀ aryl;
-X₄- is 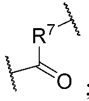 ; R⁷ is 3- to 6-membered heterocycloalkylene; the heteroatom of the 3- to 6-membered heterocycloalkylene is selected from one or more of N, O, and S, the number of heteroatoms is 1, 2, or 3, and at least one N is included;
W is -NH-CO- or -NH-CO-NH-;
R⁸ is linear C₁₋₁₀ alkyl;
R⁹ is H or C₁-C₄ alkyl substituted by 1 or 2 R⁹⁻¹; R⁹⁻¹ is carboxyl (-COOH) or amido (-CONH₂);
L₁ and L₂ are each independently a chemical bond, C₁-C₄ alkylene, or -S-(CH₂)ₖ-; k is 0 or 1;
Cy is a chemical bond, 5- to 6-membered heteroarylene, C₆-C₁₀ arylene, or C₆-C₁₀ arylene-L₄-R¹²; in the 5- to 6-membered heteroarylene, the heteroatom is selected from one or more of N, O, and S, the number of heteroatoms is 1, 2, or 3, and at least one N is included;
-L₃- is -L₃₋₁-L₃₋₂-L₃₋₃-;
-L₃₋₁ is a chemical bond, 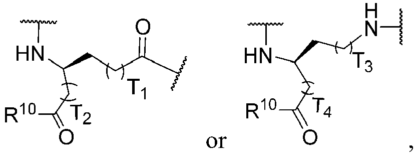 T₁ and T₂ are each independently 0 or 1, and T₃ and T₄ are each independently 0, 1, or 3;
-L₃₋₂- is -X₅-Y₃-; -X₅- is a chemical bond or a polypeptide formed by 2-6 natural amino acids; -Y₃- is a chemical bond, 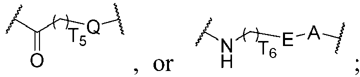 T₅ and T₆ are each independently 0, 1, 2, or 4; E and Q are each independently C₁₋₆ alkylene or -(OCH₂CH₂)_{j'}-, and j' is 1, 2, or 3; A is a chemical bond or -NH-;
-L₃₋₃- is a chemical bond, -NH-, -carbonyl-6- to 12-membered heteroarylene-, -carbonyl-C₁₋₆ alkylene-3- to 6-membered heterocycloalkylene-, -NH-C₁₋₆ alkylene-3- to 6-membered heterocycloalkylene-, or 3- to 6-membered heterocycloalkylene; the heteroatom of the 3- to 6-membered heterocycloalkylene is selected from one or more of N, O, and S, the number of heteroatoms is 1, 2, or 3, and at least one N is included; the heteroatom of the 6- to 12-membered heteroarylene is selected from one or more of N, O, and S, the number of heteroatoms is 1, 2, or 3, and at least one N is included;
R¹⁰ is hydroxyl or amino;
L₄ is 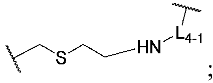 L₄₋₁ is a chemical bond;
R¹² and R¹¹ are each independently H or a chelating group; and when R¹¹ is H, R¹² is a chelating group, or when R¹² is H, R¹¹ is a chelating group;
the chelating group is 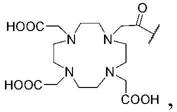 NOTA, HBED-CC, NODAGA, NOTAGA, DOTAGA, TRAP, NOPO, PCTA, DFO, DTPA, CHX-DTPA, AAZTA, or DEDPA;
the compound X satisfies one or more of the following conditions:
(1) -X₁- is 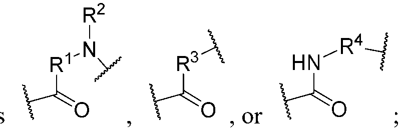
R¹ is C₃-C₆ cycloalkylene or C₁-C₄ alkylene-C₃-C₆ heterocycloalkylene;
R² is hydrogen;
R³ is 4-membered or 6-membered heterocycloalkylene; the heteroatom of the 4-membered or 6-membered heterocycloalkylene is selected from one or more of N, O, and S, the number of heteroatoms is 1, 2, or 3, and at least one N is included;
R⁴ is 3- to 6-membered heterocycloalkylene; the heteroatom of the 3- to 6-membered heterocycloalkylene is selected from one or more of N, O, and S, the number of heteroatoms is 1, 2, or 3, and at least one N is included;
(2) -X₂₋₁- is a natural amino acid or a non-natural amino acid;
(3) -X₃₋₁- is a natural amino acid; or R⁶ is C₁-C₄ alkyl substituted by 1 or 2 R⁶⁻¹; each R⁶⁻¹ is independently C₃-C₆ cycloalkyl or halogen;
(4) -X₄- is 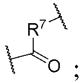 R⁷ is 4-membered heterocycloalkylene; the heteroatom of the 4-membered heterocycloalkylene is selected from one or more of N, O, and S, the number of heteroatoms is 1, 2, or 3, and at least one N is included;
scheme 2:
in the compound X,
-X₁- is 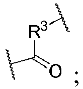
R³ is 3- to 6-membered heterocycloalkylene; the heteroatom of the 3- to 6-membered heterocycloalkylene is selected from one or more of N, O, and S, the number of heteroatoms is 1, 2, or 3, and at least one N is included;
-X₂- is -X₂₋₁-Y₁-; -X₂₋₁- is a chemical bond and Y₁ is 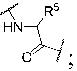
R⁵ is each independently C₁-C₄ alkylene substituted by 1, 2, or 3 R⁵⁻¹;
R⁵⁻¹ is each independently hydroxyl;
-X₃- is -X₃₋₁-Y₂-; -X₃₋₁- is a chemical bond and Y₂ is 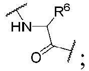
R⁶ is C₁-C₄ alkyl substituted by 1 or 2 R⁶⁻¹; each R⁶⁻¹ is independently C₃-C₆ cycloalkyl or C₆-C₁₀ aryl;
-X₄- is 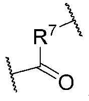 ; R⁷ is 3- to 6-membered heterocycloalkylene; the heteroatom of the 3- to 6-membered heterocycloalkylene is selected from one or more of N, O, and S, the number of heteroatoms is 1, 2, or 3, and at least one N is included;
W is -NH-CO- or -NH-CO-NH-;
R⁸ is linear C₁₋₁₀ alkyl;
R⁹ is H or C₁-C₄ alkyl substituted by 1 or 2 R⁹⁻¹; R⁹⁻¹ is amido (-CONH₂);
L₁ and L₂ are each independently -S-(CH₂)ₖ-; k is 1;
Cy is C₆-C₁₀ arylene;
-L₃- is -L₃₋₁-L₃₋₂-L₃₋₃-;
-L₃₋₁ is a chemical bond, 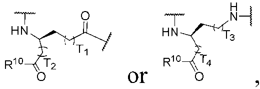 T₁ and T₂ are each independently 0 or 1, and T₃ and T₄ are each independently 0 or 1;
-L₃₋₂- is -X₅-Y₃-; -X₅- is a chemical bond and -Y₃- is a chemical bond, 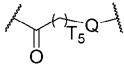 or 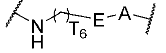 ; T₅ and T₆ are each independently 0, 1, or 2, and E and Q are each independently C₁₋₆ alkylene; A is a chemical bond or -NH-;
-L₃₋₃- is a chemical bond, -NH-, -carbonyl-C₁₋₆ alkylene-3- to 6-membered heterocycloalkylene-, -NH-C₁₋₆ alkylene-3- to 6-membered heterocycloalkylene-, or 3- to 6-membered heterocycloalkylene, wherein the heteroatom of the 3- to 6-membered heterocycloalkylene is selected from one or more of N, O, and S, the number of heteroatoms is 1, 2, or 3, and at least one N is included;
R¹⁰ is hydroxyl;
R¹¹ is a chelating group; the chelating group is 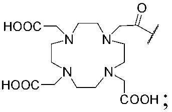
the compound X satisfies one or more of the following conditions:
(1) -X₁- is 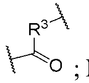 R³ is 4-membered or 6-membered heterocycloalkylene; the heteroatom of the 4- to 6-membered heterocycloalkylene is selected from one or more of N, O, and S, the number of heteroatoms is 1, 2, or 3, and at least one N is included;
(2) R⁶ is C₁-C₄ alkyl substituted by 1 or 2 R⁶⁻¹; each R⁶⁻¹ is independently C₃-C₆ cycloalkyl;
scheme 3:
in the compound X,
-X₁- is 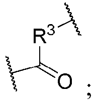
R³ is 3- to 6-membered heterocycloalkylene; the heteroatom of the 3- to 6-membered heterocycloalkylene is selected from one or more of N, O, and S, the number of heteroatoms is 1, 2, or 3, and at least one N is included;
-X₂- is -X₂₋₁-Y₁-; -X₂₋₁- is a chemical bond and Y₁ is 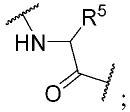
R⁵ is each independently C₁-C₄ alkylene substituted by 1, 2, or 3 R⁵⁻¹;
R⁵⁻¹ is each independently hydroxyl;
-X₃- is -X₃₋₁-Y₂-; -X₃₋₁- is a chemical bond and Y₂ is 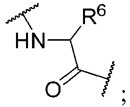
R⁶ is C₁-C₄ alkyl substituted by 1 or 2 R⁶⁻¹; each R⁶⁻¹ is independently C₃-C₆ cycloalkyl or C₆-C₁₀ aryl;
-X₄- is 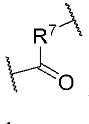 ; R⁷ is 3- to 6-membered heterocycloalkylene; the heteroatom of the 3- to 6-membered heterocycloalkylene is selected from one or more of N, O, and S, the number of heteroatoms is 1, 2, or 3, and at least one N is included;
W is -NH-CO- or -NH-CO-NH-;
R⁸ is linear C₁₋₁₀ alkyl;
R⁹ is C₁-C₄ alkyl substituted by 1 R⁹⁻¹; R⁹⁻¹ is amido;
L₁ and L₂ are each independently -S-(CH₂)ₖ-; k is 1;
Cy is C₆-C₁₀ arylene;
-L₃- is -L₃₋₁-L₃₋₂-L₃₋₃-;
-L₃₋₁ is 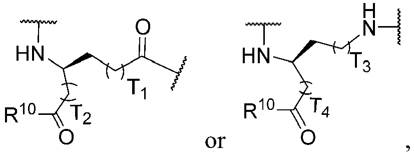 T₁ and T₂ are each independently 0 or 1, and T₃ and T₄ are each independently 0 or 1;
-L₃₋₂- is -X₅-Y₃-; -X₅- is a chemical bond, and -Y₃- is a chemical bond, 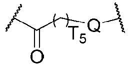 or 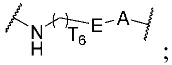 T₅ and T₆ are each independently 0, 1, or 2, and E and Q are each independently C₁₋₆ alkylene or -(OCH₂CH₂)_{j'}-, wherein j' is 1; A is a chemical bond or -NH-;
-L₃₋₃- is a chemical bond, -NH-, -carbonyl-C₁₋₆ alkylene-3- to 6-membered heterocycloalkylene- or -NH-C₁₋₆ alkylene-3- to 6-membered heterocycloalkylene-, wherein the heteroatom of the 3- to 6-membered heterocycloalkylene is selected from one or more of N, O, and S, the number of heteroatoms is 1, 2, or 3, and at least one N is included;
R¹⁰ is hydroxyl or amino;
R¹¹ is a chelating group;
the chelating group is 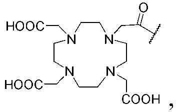 NOTA, HBED-CC, NODAGA, NOTAGA, DOTAGA, TRAP, NOPO, PCTA, DFO, DTPA, CHX-DTPA, AAZTA, or DEDPA;
scheme 4:
in the compound X,
-X₁- is 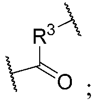
R³ is 3- to 6-membered heterocycloalkylene; the heteroatom of the 3- to 6-membered heterocycloalkylene is selected from one or more of N, O, and S, the number of heteroatoms is 1, 2, or 3, and at least one N is included;
-X₂- is -X₂₋₁-Y₁-; -X₂₋₁- is a chemical bond and Y₁ is 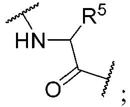
R⁵ is each independently C₁-C₄ alkylene substituted by 1, 2, or 3 R⁵⁻¹;
R⁵⁻¹ is each independently hydroxyl;
-X₃- is -X₃₋₁-Y₂-; -X₃₋₁- is a chemical bond and Y₂ is 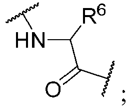
R⁶ is C₁-C₄ alkyl substituted by 1 or 2 R⁶⁻¹; each R⁶⁻¹ is independently C₃-C₆ cycloalkyl or C₆-C₁₀ aryl;
-X₄- is 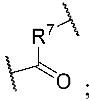 R⁷ is 3- to 6-membered heterocycloalkylene; the heteroatom of the 3- to 6-membered heterocycloalkylene is selected from one or more of N, O, and S, the number of heteroatoms is 1, 2, or 3, and at least one N is included;
W is -NH-CO- or -NH-CO-NH-;
R⁸ is linear C₁₋₁₀ alkyl;
R⁹ is C₁-C₄ alkyl substituted by 1 or 2 R⁹⁻¹; R⁹⁻¹ is amido (-CONH₂);
L₁ and L₂ are each independently -S-(CH₂)ₖ-; k is 1;
Cy is C₆-C₁₀ arylene;
-L₃- is -L₃₋₁-L₃₋₂-L₃₋₃-;
-L₃₋₁ is 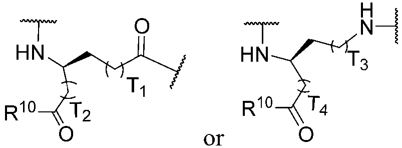 , T₁ and T₂ are each independently 0 or 1, and T₃ and T₄ are each independently 0 or 1;
-L₃₋₂- is -X₅-Y₃-; -X₅- is a chemical bond and -Y₃- is a chemical bond, 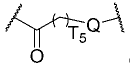 or 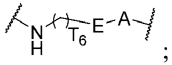 T₅ and T₆ are each independently 0, 1, 2, or 4, and E and Q are each independently C₁₋₆ alkylene; A is a chemical bond or -NH-;
-L₃₋₃- is -NH-, -carbonyl-C₁₋₆ alkylene-3- to 6-membered heterocycloalkylene- or -NH-C₁₋₆ alkylene-3- to 6-membered heterocycloalkylene-, wherein the heteroatom of the 3- to 6-membered heterocycloalkylene is selected from one or more of N, O, and S, the number of heteroatoms is 1, 2, or 3, and at least one N is included;
R¹⁰ is hydroxyl;
R¹¹ is a chelating group;
the chelating group is 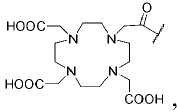 NOTA, HBED-CC, NODAGA, NOTAGA, DOTAGA, TRAP, NOPO, PCTA, DFO, DTPA, CHX-DTPA, AAZTA, or DEDPA.

9. The compound X or the pharmaceutically acceptable salt thereof according to claim 1, wherein the compound X satisfies one or more of the following conditions:
(1) -X₁- is 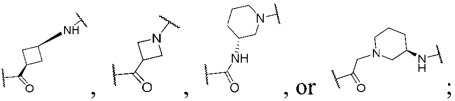 preferably, -X₁- is 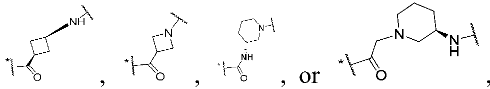 , wherein 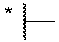 is connected to X₄;
(2) -X₄- is 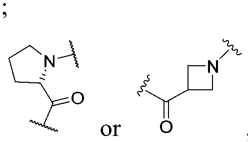 , for example 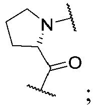 preferably, -X₄- is 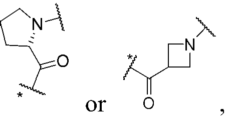 wherein 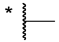 is connected to X₂;
(3) -X₂- is 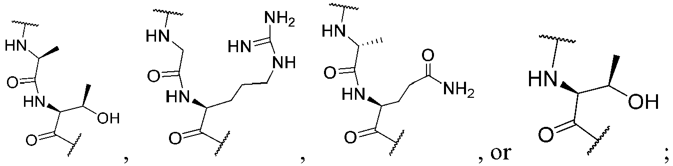 preferably, -X₂- is 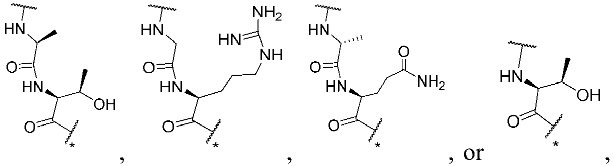 wherein 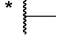 is connected to -NH-;
(4) -X₃- is 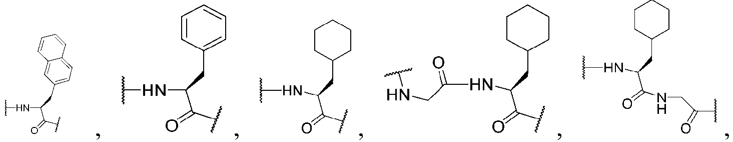 or 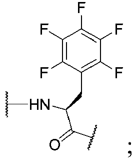 preferably, -X₃-is 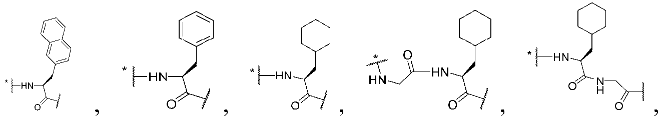 or 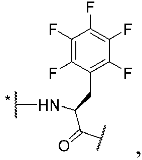 wherein 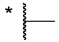 is connected to carbonyl;
(5) -L₄- is 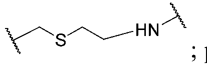 preferably, -L₄- is 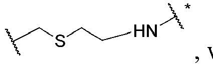 wherein 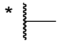 is connected to R¹¹;
(6) -L₃₋₁- is a chemical bond, 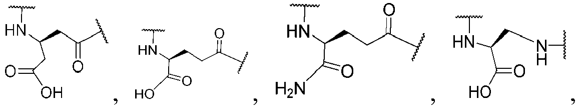 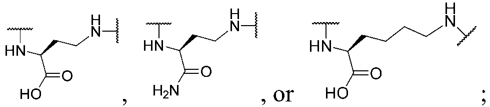 preferably, -L₃₋₁- is a chemical bond, 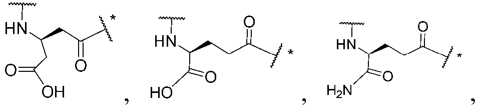 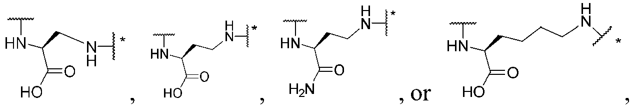 wherein 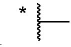 is connected to -L₃₋₂-;
(7) -L₃₋₂- is a chemical bond, 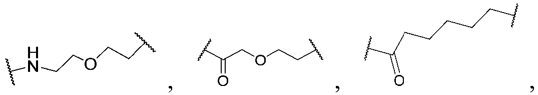 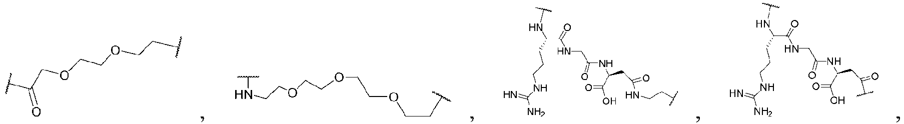 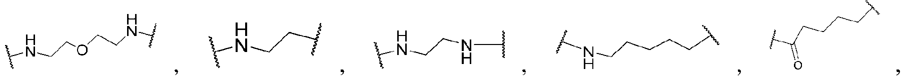 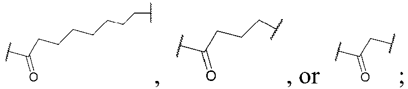 preferably, -L₃₋₂- is a chemical bond, 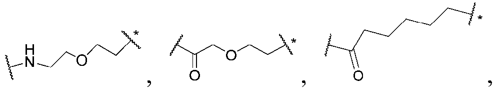 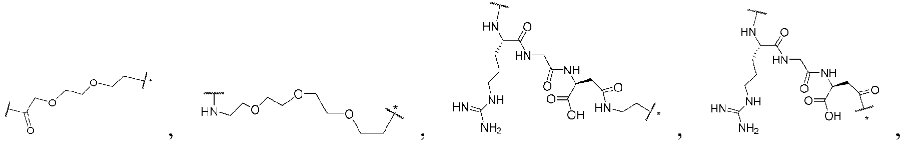 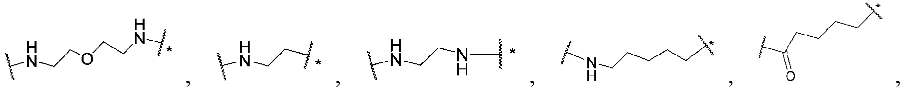 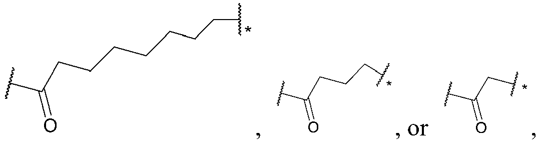 wherein 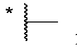 is connected to -L₃₋₃-;
(8), -L₃₋₃- is a chemical bond, -NH-, 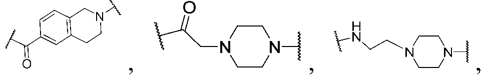 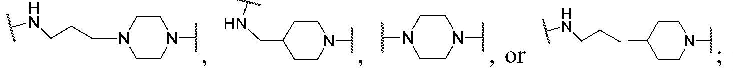 preferably, -L₃₋₃- is a chemical bond, 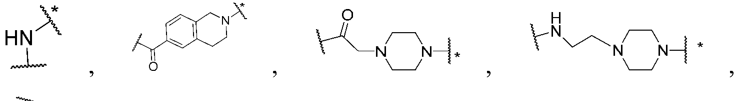 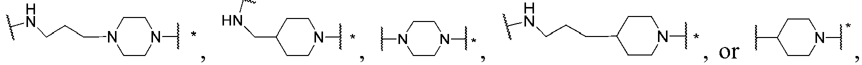 wherein 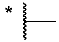 is connected to R¹¹;
(9) R⁸ is methyl, n-pentyl, n-nonyl, n-heptyl, or n-butyl;
(10) R⁹ is H, 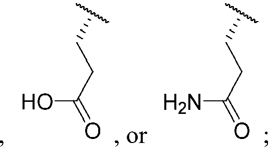
(11) L₁ and L₂ are each independently -S-methylene-, n-propylene, a chemical bond, or - S-;
(12) Cy is 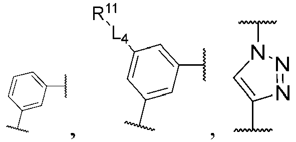 , a chemical bond, or 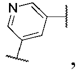 wherein 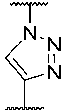 is for example 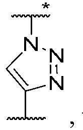 wherein 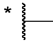 is connected to L₁.

10. The compound X or the pharmaceutically acceptable salt thereof according to claim 1, wherein the compound X has a structure as shown in any one of the following:

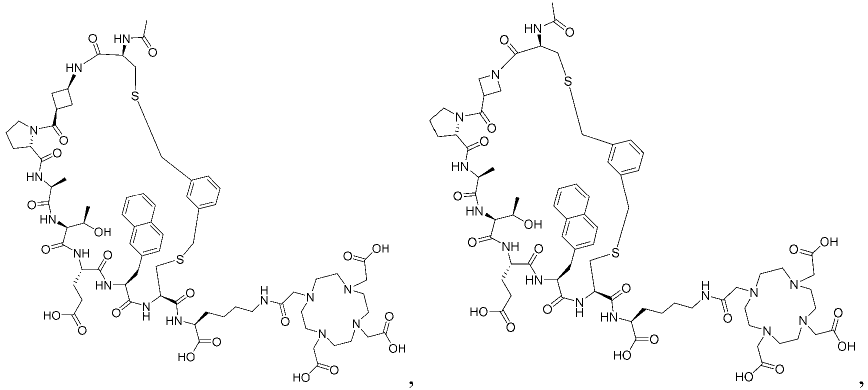

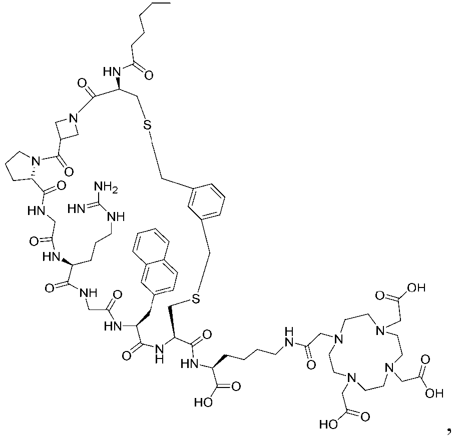

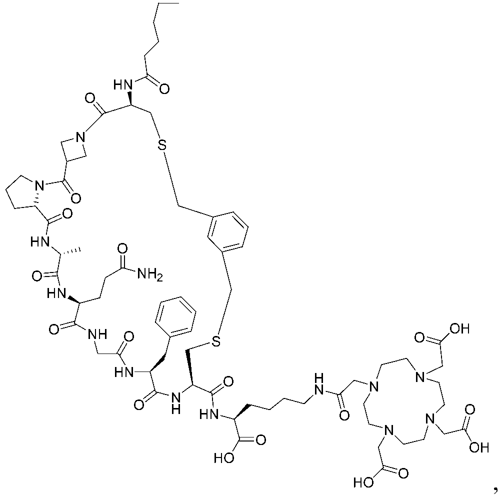

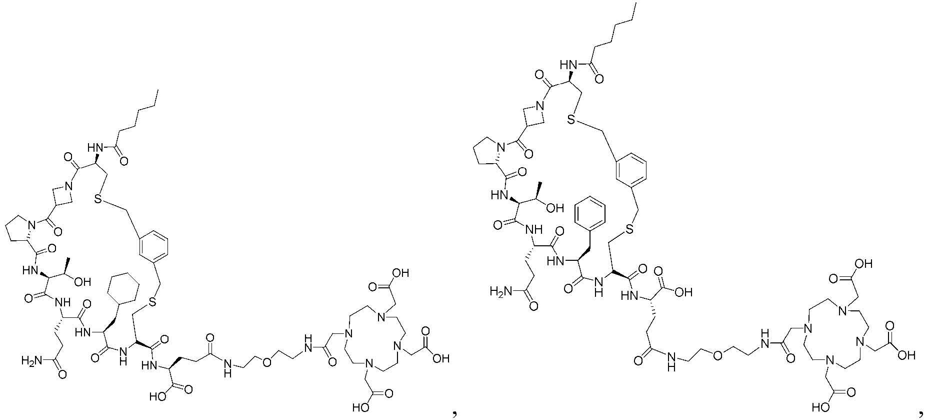

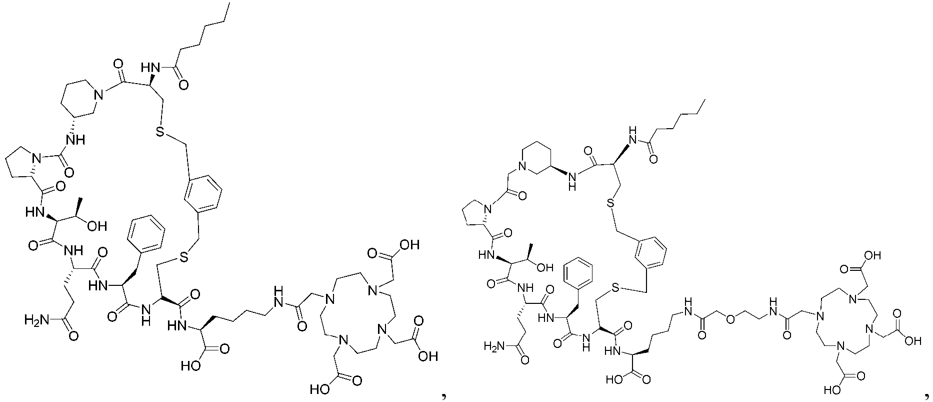

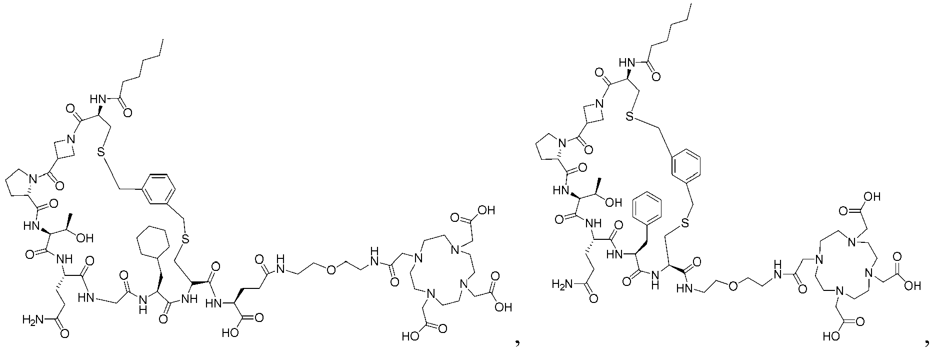

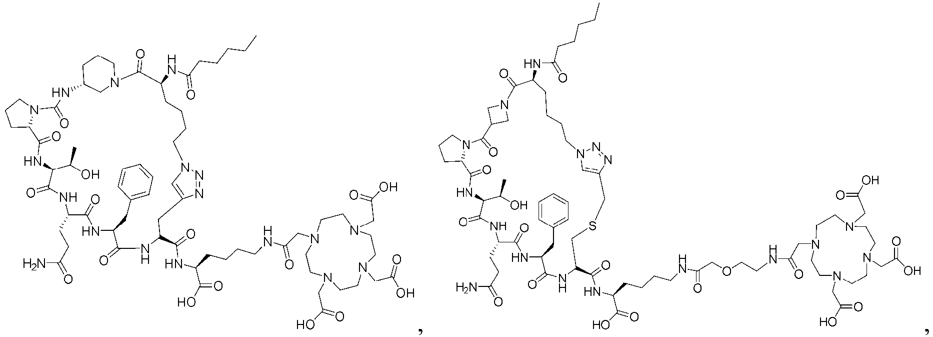

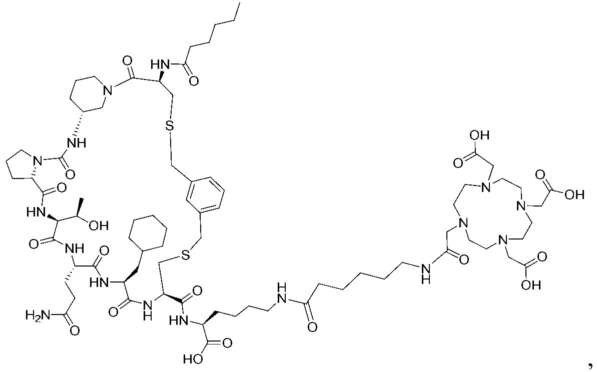

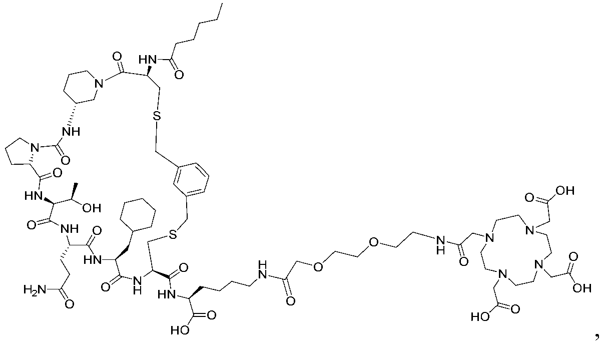

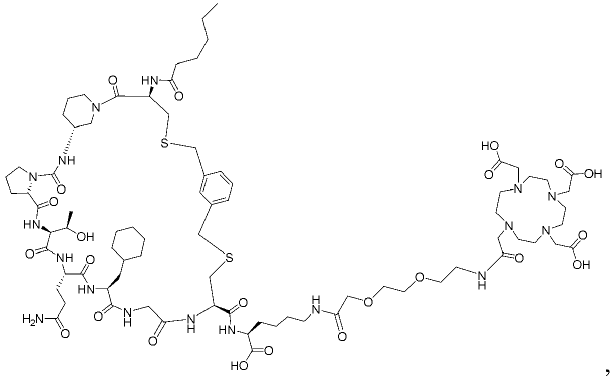

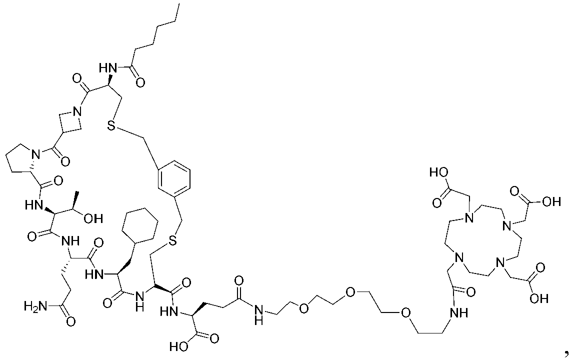

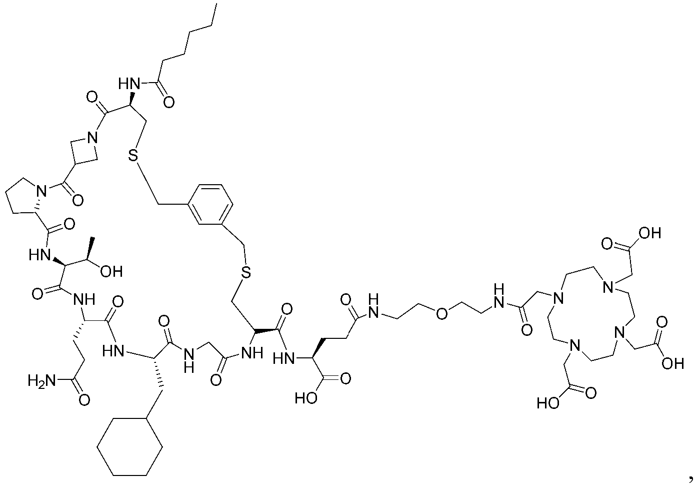

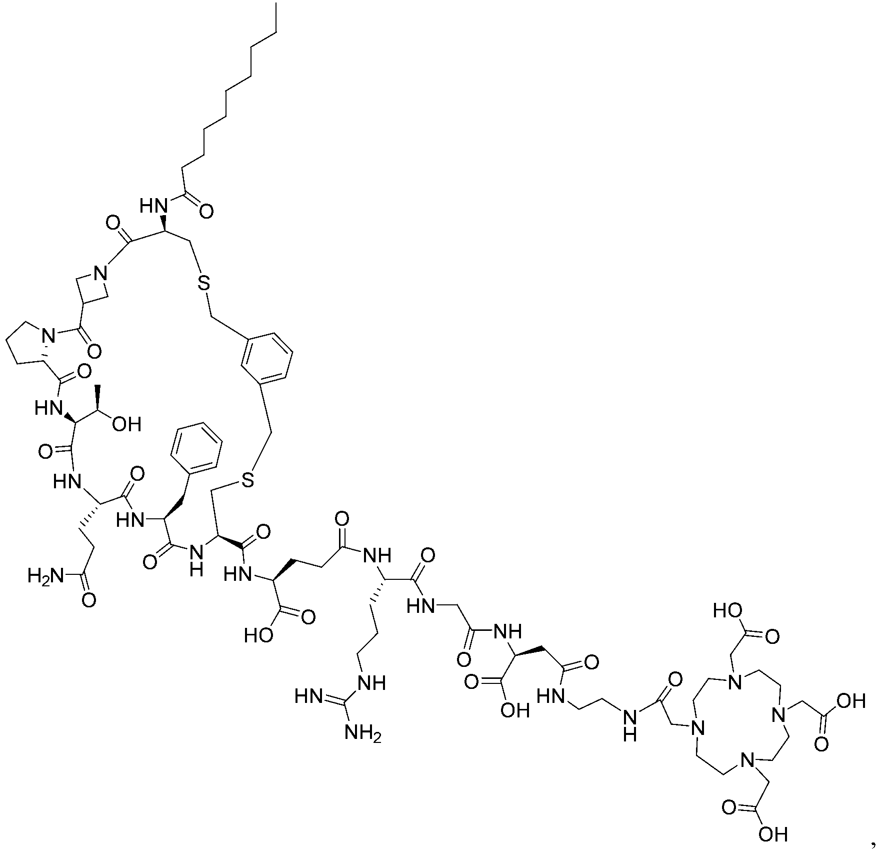

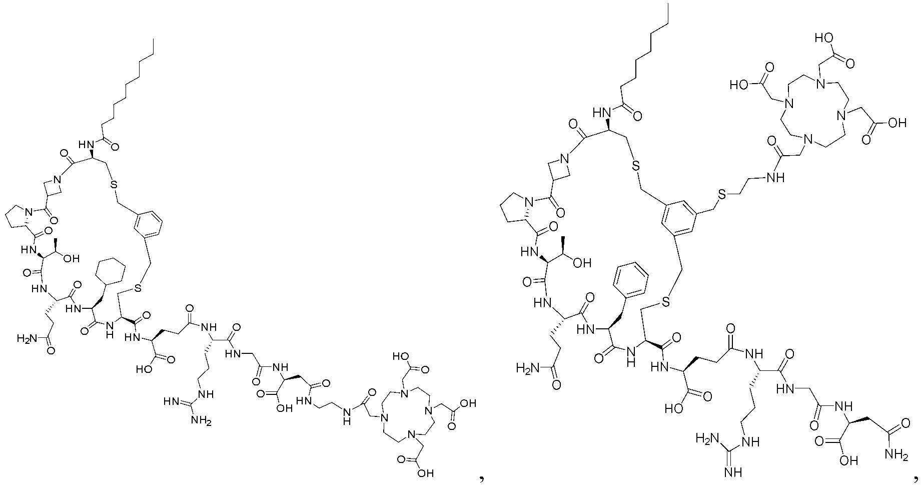

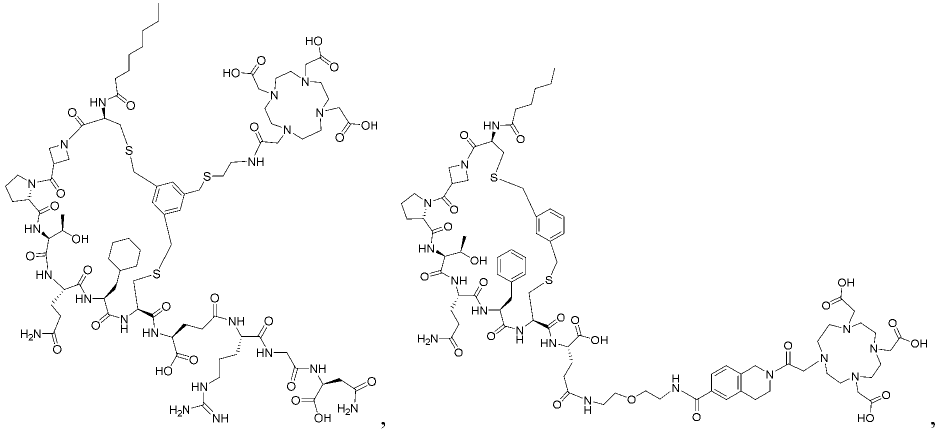

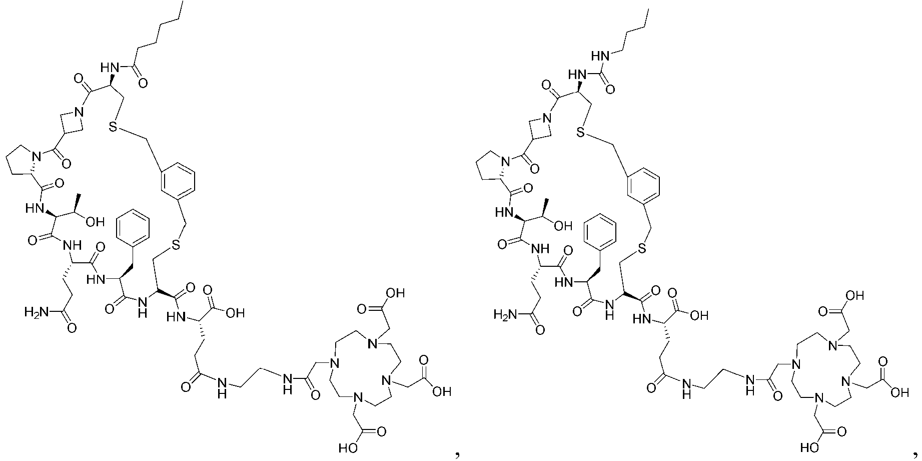

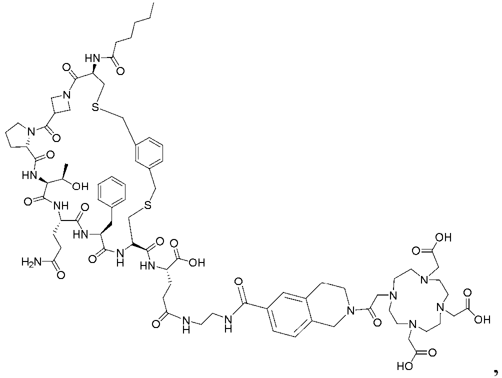

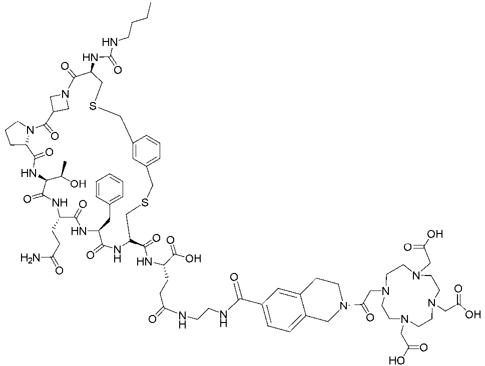

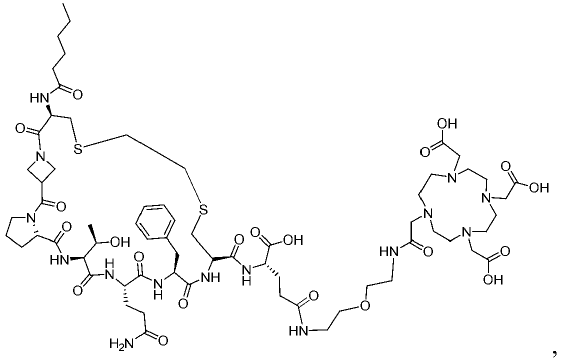

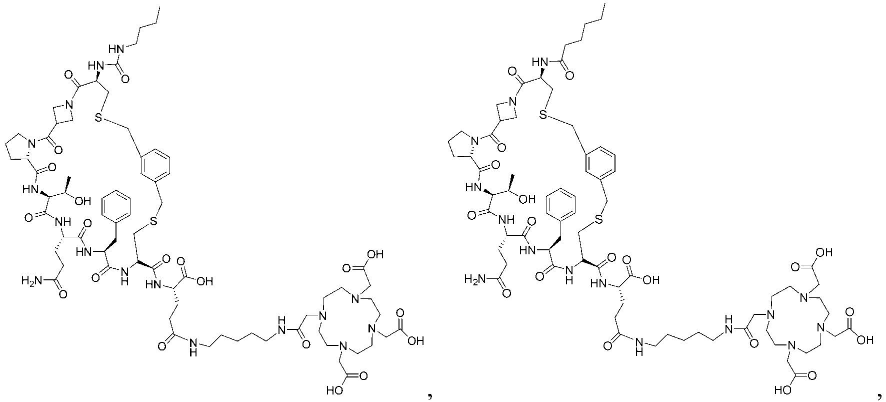

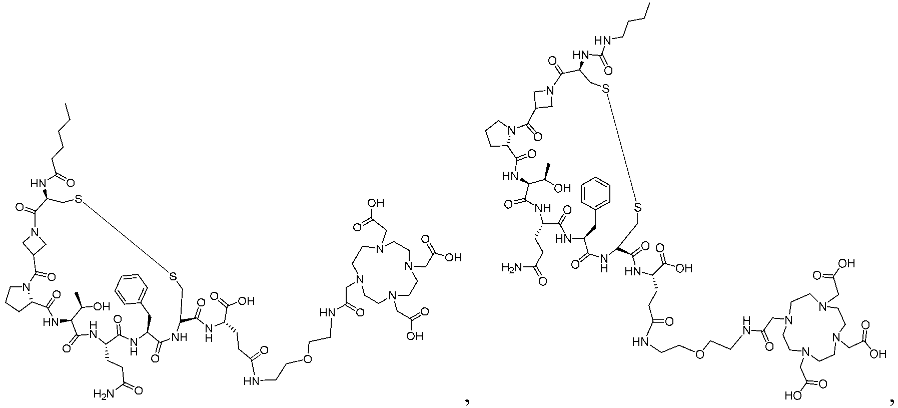

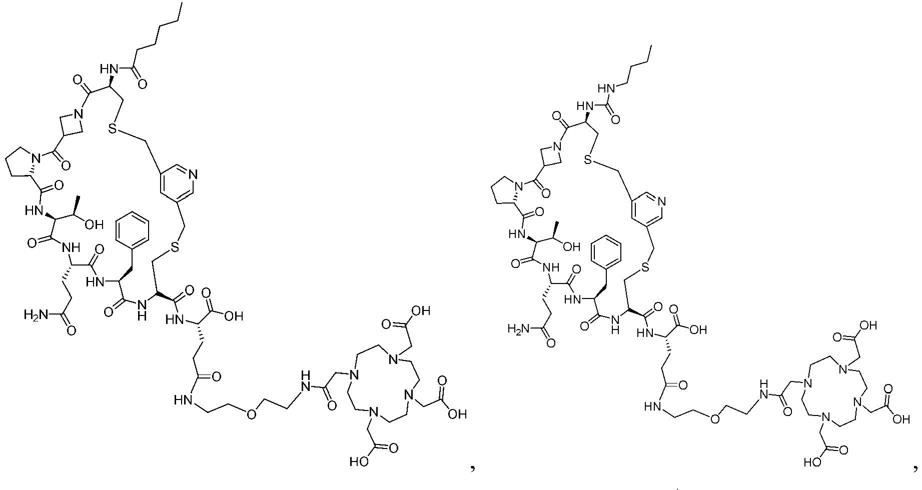

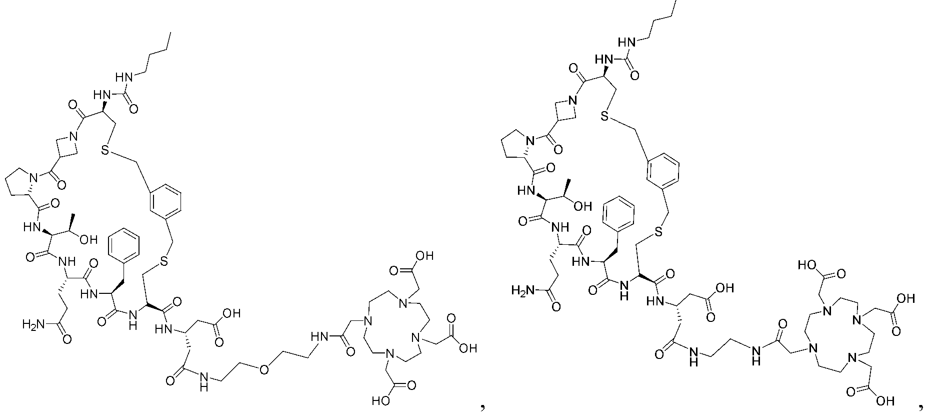

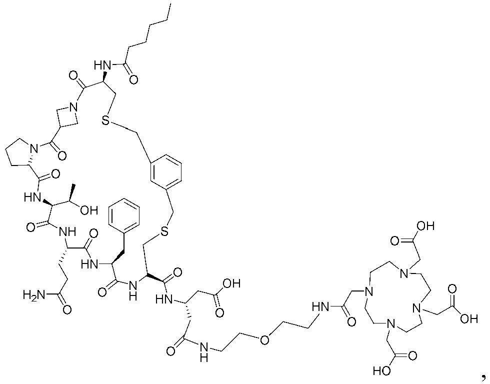

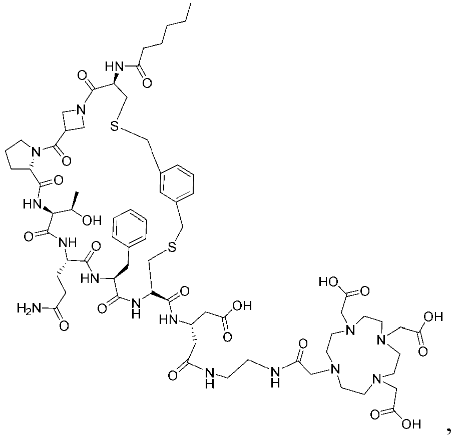

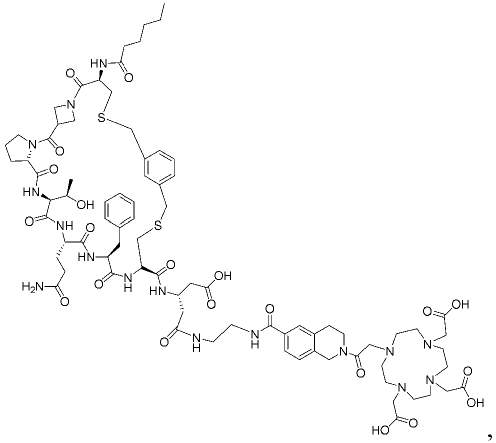

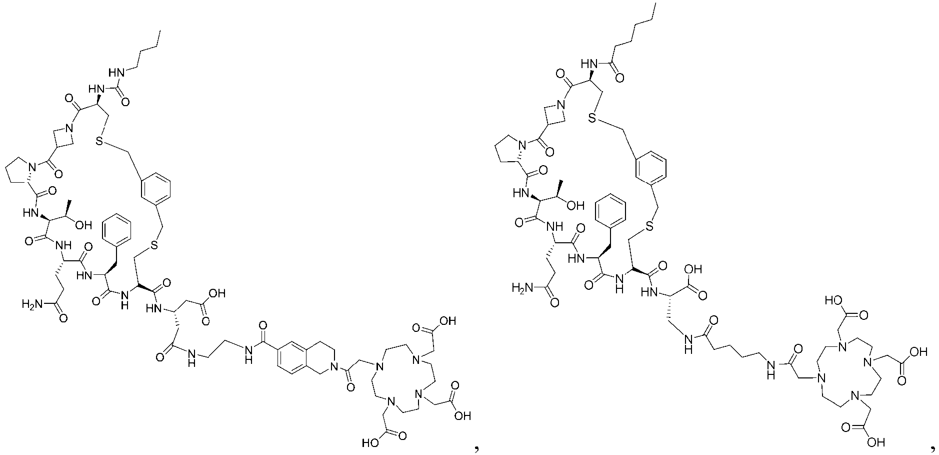

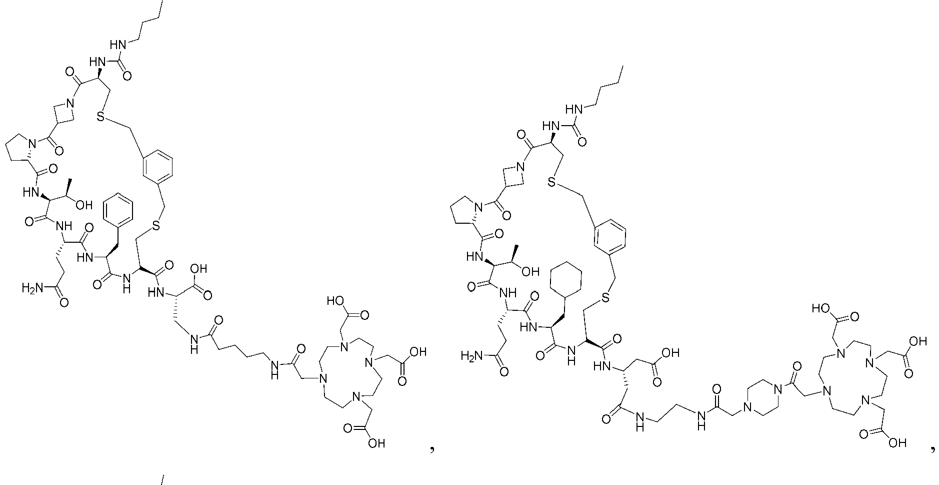

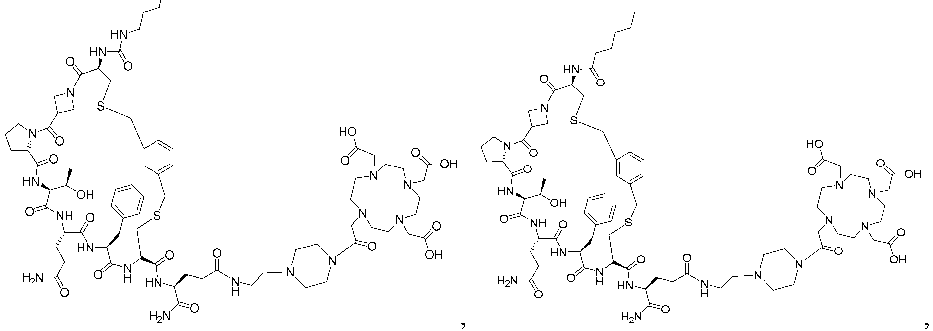

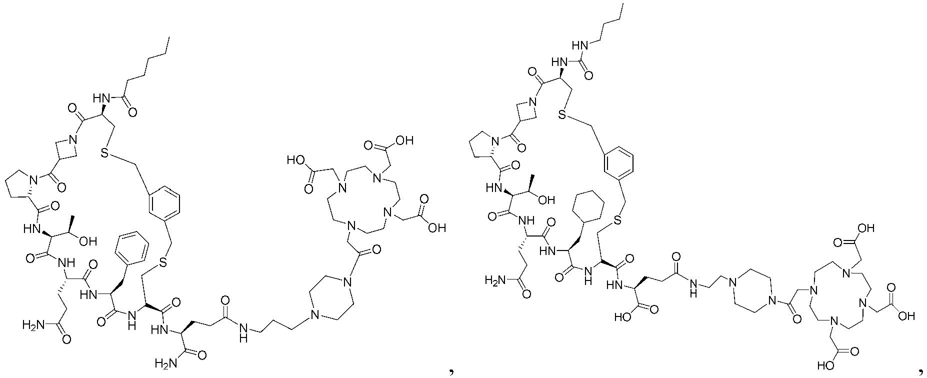

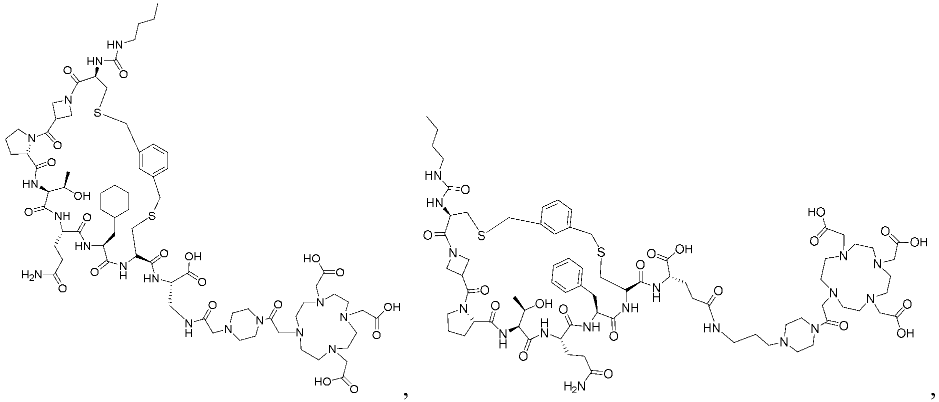

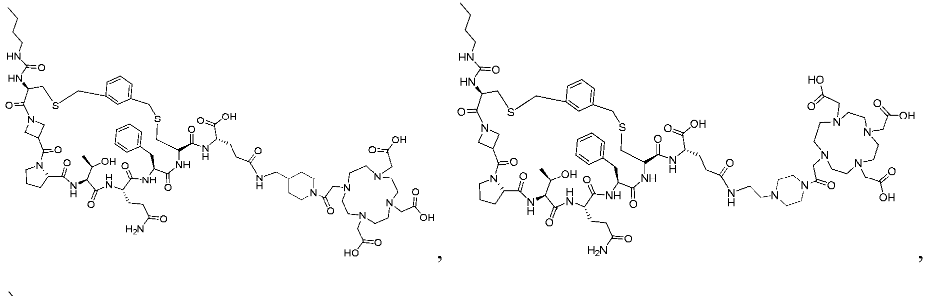

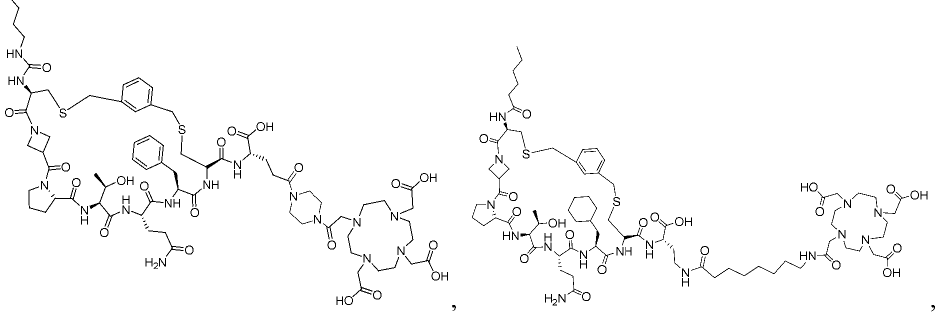

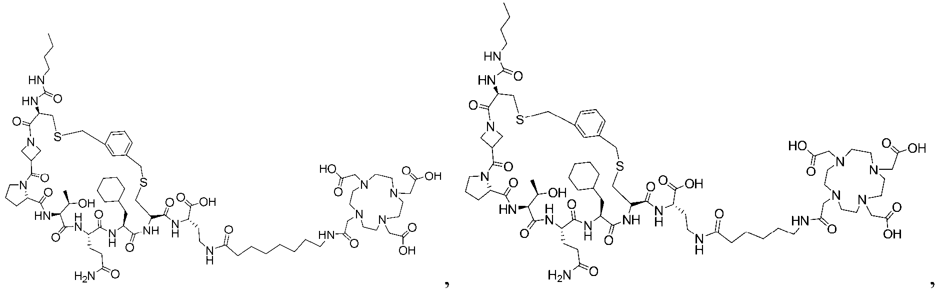

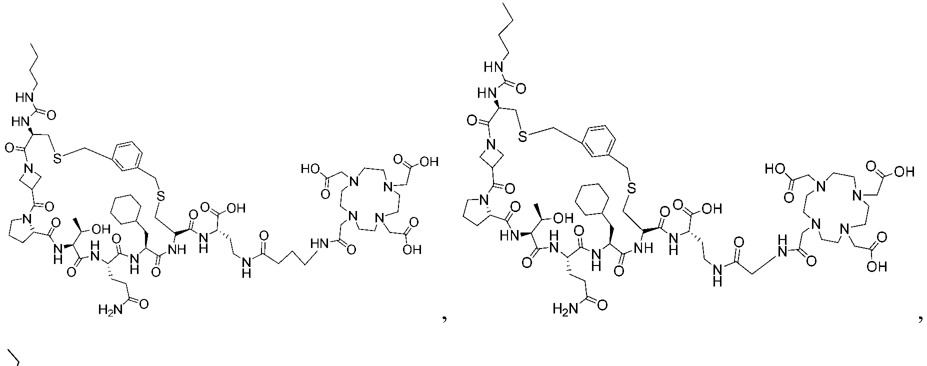

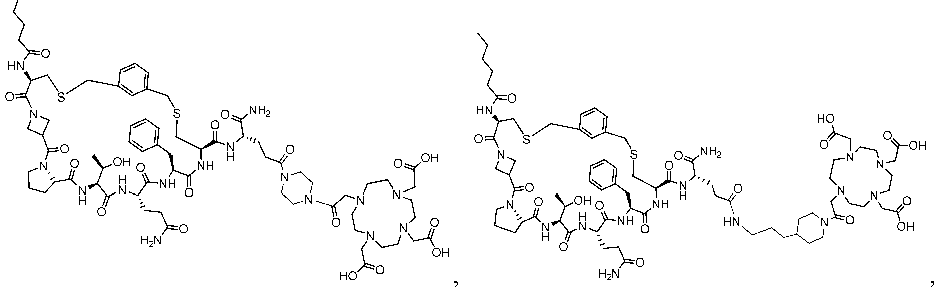

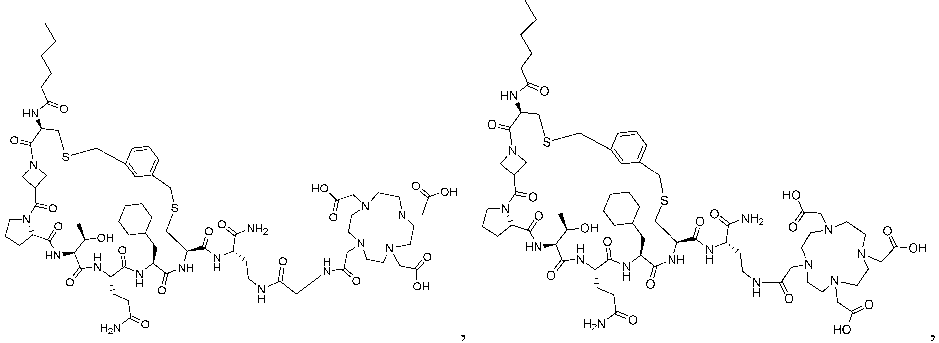

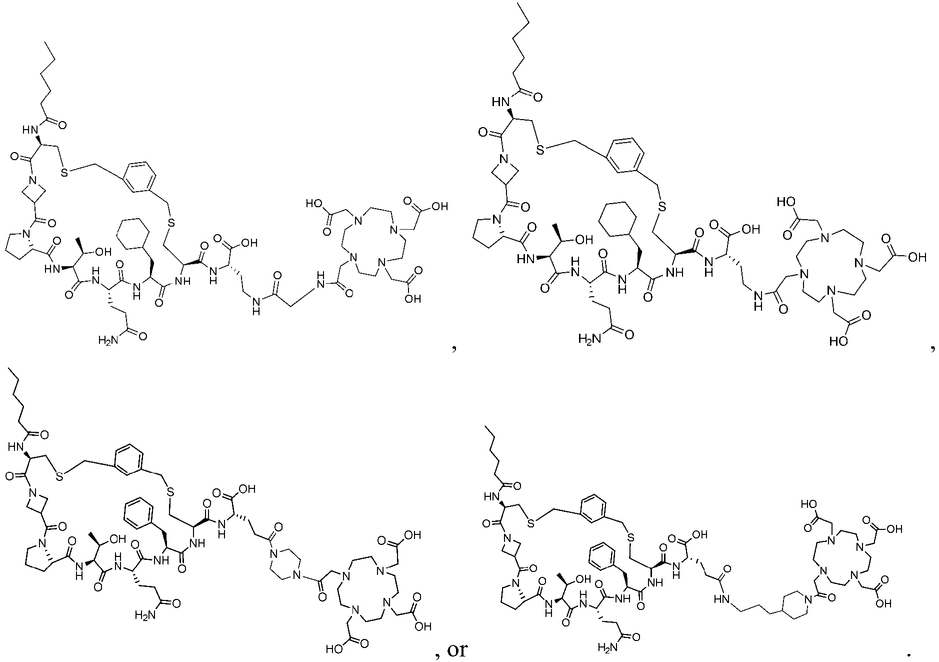

11. A cyclic polypeptide compound A, wherein the cyclic polypeptide compound A is a compound formed by chelating an ion of a therapeutic radionuclide with the compound X according to any one of claims 1 to 10.

12. The cyclic polypeptide compound A according to claim 11, wherein the cyclic polypeptide compound A satisfies one or more of the following conditions:
(1) the therapeutic radionuclide is ¹⁷⁷Lu, ⁹⁰Y, ⁸⁹Sr, ¹⁸⁸Re, ²²⁵Ac, ²¹³Bi, or ²¹²Pb;
(2) a valence state of the ion of the therapeutic radionuclide is monovalent, divalent, trivalent, or tetravalent;
(3) the ion of the therapeutic radionuclide is ¹⁷⁷Lu³⁺, ²²⁵Ac³⁺, ⁹⁰Y³⁺, ²¹²Pb²⁺, or ²¹³Bi³⁺.

13. The cyclic peptide compound A according to claim 11, wherein the cyclic peptide compound A is a compound formed by chelating ¹⁷⁷Lu³⁺ with the compound X.

14. A cyclic polypeptide compound B, wherein the cyclic polypeptide compound B is a compound formed by chelating an ion of a diagnostic radionuclide with a compound X, and the compound X has a structure as described in any one of claims 1 to 10.

15. The cyclic polypeptide compound B according to claim 14, wherein the cyclic polypeptide compound B satisfies one or more of the following conditions:
(1) the diagnostic radionuclide is ¹⁸F, ⁶⁸Ga, ¹¹¹In, or ⁶⁴Cu;
(2) a valence state of the ion of the diagnostic radionuclide is monovalent, divalent, trivalent, or tetravalent;
(3) the ion of the diagnostic radioactive metal is ⁶⁸Ga³⁺ or ⁶⁴Cu²⁺.

16. The cyclic peptide compound B according to claim 14, wherein the cyclic peptide compound B is a compound formed by chelating ⁶⁸Ga³⁺ with a compound X, and the compound X has a structure as described in any one of claims 1 to 10.

17. A pharmaceutical composition, comprising a substance Y and a pharmaceutically acceptable excipient, wherein the substance Y is the cyclic polypeptide compound A according to any one of claims 11 to 13 or the cyclic polypeptide compound B according to any one of claims 14 to 16.

18. A use of the cyclic peptide compound A according to any one of claims 11 to 13 in the preparation of a medicament for treating tumors, wherein the tumor may be a solid tumor with positive FAP expression, for example breast cancer, ovarian cancer, lung cancer, colorectal cancer, gastric cancer, pancreatic cancer, prostate cancer, liver cancer, or cutaneous melanoma.

19. A use of the cyclic peptide compound B according to any one of claims 14 to 16 in the preparation of a medicament for diagnosing tumors, wherein the tumor may be a solid tumor with positive FAP expression, for example breast cancer, ovarian cancer, lung cancer, colorectal cancer, gastric cancer, pancreatic cancer, prostate cancer, liver cancer, or cutaneous melanoma.
